# EUROPÄISCHE PATENTANMELDUNG

(11) **EP 4 023 276 A1**
(43) Veröffentlichungstag der Anmeldung: **06.07.2022**
(21) Anmeldenummer: 20217882.8
(22) Anmeldetag: 30.12.2020
(51) Int. Cl.: A61M 15/08, A61M 15/00

(54) **NASENAPPLIKATOR**

(71) Anmelder: HATCHMORE Labs GmbH, 82031 Grünwald (DE)
(72) Erfinder: Sinner, Klaus, 80801 München (DE); Souren, Francois Henri Mathieu Marie, 81541 München (DE)
(74) Vertreter: Bobbert & Partner Patentanwälte PartmbB

(57) **Zusammenfassung**

Die vorliegende Erfindung betrifft einen Nasenapplikator (100) zum nasalen Verabreichen wenigstens einer medizinischen Substanz (S), insbesondere einem Schmerzmittel. Hierbei umfasst der Nasenapplikator ein Gehäuse (2), das seinerseits ein Substanzreservoir (R) aufweist oder mit einem solchen verbunden ist. Ferner umfasst das Gehäuse eine Abrufvorrichtung (1) zum Betätigen durch den Benutzer mit dem Ziel, eine nächste Applikationsdosis (Dₙ) der Substanz (S) abzurufen oder ist mit einer solchen verbunden. Weiter umfasst das Gehäuse einen Abgabedosierer sowie eine Sperrvorrichtung (5) zum zeitweisen Sperren des Abgabedosierers (3) oder ist mit solchen Vorrichtungen verbunden. Weiter sind vom Gehäuse eine Dosiserfassungsvorrichtung (7) zum Erfassen der Höhe der mittels des Abgabedosierers (3) jeweils abgegebenen Applikationsdosis (D₀, D₁, D₂, ..., Dₙ₋₁, Dₙ, Dₙ₊₁, ...), eine elektronische Steuervorrichtung (9) und ein Datenspeicher (M) umfasst oder das Gehäuse ist mit diesen verbunden. Mittels einer Erfassungsvorrichtung (11) kann in Zusammenwirken mit der Steuervorrichtung (9) und dem Datenspeicher (M) mittels Auslesens, Auswertens und/oder Vorbestimmens sowohl ein geeigneter Zeitpunkt als auch eine geeignete Dosis für eine folgende Applikation einer Substanz (S) ermittelt werden. Die Erfindung betrifft ferner ein System.

## Beschreibung

Die vorliegende Erfindung betrifft eine Medikamentenabgabevorrichtung, insbesondere einen Nasenapplikator, gemäß Anspruch 1. Sie betrifft ferner ein System gemäß Anspruch 17.

Aus der Praxis sind Nasenapplikatoren bekannt, mittels welcher sich ein Patient ein Medikament oder einen Wirkstoff nasal applizieren kann.

Es ist Aufgabe der vorliegenden Erfindung, einen weiteren Nasenapplikator vorzuschlagen.

Die erfindungsgemäße Aufgabe wird durch den Nasenapplikator mit den Merkmalen des Anspruchs 1 gelöst, ferner durch das System mit den Merkmalen des Anspruchs 17.

Erfindungsgemäß wird eine Medikamentenabgabevorrichtung (für die nasale, inhalative, intravenöse, orale, bukkale, sublinguale, usw. Abgabe), insbesondere ein Nasenapplikator zum Verabreichen bzw. nasalen Verabreichen wenigstens einer Substanz, z. B. einer medizinischen oder nicht-medizinischen Substanz, z. B. ein medizinischer oder nicht-medizinischer Wirkstoff, vorgeschlagen. Die medizinische Substanz kann insbesondere ein Schmerzmittel sein. Hierzu weist die Medikamentenabgabevorrichtung bzw. der Nasenapplikator ein Gehäuse auf.

Die Medikamentenabgabevorrichtung weist ein Substanzreservoir zum Vorhalten einer Menge der Substanz auf oder ist mit einem solchen verbunden.

Die Medikamentenabgabevorrichtung ist mit einer Abrufvorrichtung zum Betätigen durch den Benutzer/Patienten verbunden oder weist eine solche auf. Die Abrufvorrichtung ist programmiert oder anderweitig vorbereitet, damit der Benutzer der Medikamentenabgabevorrichtung mittels Betätigens der Abrufvorrichtung eine nächste Applikationsdosis der Substanz abrufen kann. Zweck oder Ziel der Abrufvorrichtung ist somit, eine Applikationsdosis auslösen zu können.

Die Medikamentenabgabevorrichtung weist weiter einen Abgabedosierer auf oder ist mit einem solchen verbunden. Der Abgabedosierer ist vorgesehen zum Abgeben von Applikationsdosen auf eine Betätigung der Abrufvorrichtung hin. Die Abgabe erfolgt, wenn sie erfolgt, jeweils zu einem sogenannten Abgabezeitpunkt.

Die Medikamentenabgabevorrichtung ist ferner mit einer Sperrvorrichtung verbunden oder weist eine solche auf. Die Sperrvorrichtung ist konfiguriert zum zeitweisen Sperren des Abgabedosierers während einer, insbesondere vordefinierten, Sperrdauer. Die Sperrdauer beginnt beispielsweise nach Abgabe einer, mancher oder jeder Applikationsdosis. Es kann somit vorgesehen sein, zu oder nach dem jeweiligen Abgabezeitpunkt der abgegebenen Applikationsdosis erneut eine Sperrdauer beginnen zu lassen. Die Sperrdauer ist definiert durch ihren jeweiligen Sperrdauerbeginn und ihr jeweiliges Sperrdauerende. Von der Sperrvorrichtung angesetzte Sperrdauern können unterschiedliche Dauern aufweisen oder jeweils die gleiche Dauer haben.

Außerdem weist die Medikamentenabgabevorrichtung eine Dosiserfassungsvorrichtung auf oder ist mit einer solchen verbunden. Die Dosiserfassungsvorrichtung ist programmiert zum Erfassen der Höhe (z. B. als Menge, in Gewichtseinheit, in Volumeneinheit, in Stoffeinheit wie Mol, usw.) der Applikationsdosis, die mittels des Abgabedosierers zum jeweiligen Abgabezeitpunkt jeweils abgegeben wird. Sie kann auch programmiert sein zum Erfassen der Höhe einer hierin als Ausgangsdosis bezeichneten Applikationsdosis.

Ferner ist die Medikamentenabgabevorrichtung mit einer elektronischen Steuervorrichtung verbunden oder weist eine solche auf.

Die Medikamentenabgabevorrichtung kann einen Datenspeicher zum Speichern aufweisen oder hiermit verbunden sein. Er kann zumindest die Abgabezeitpunkte einer oder mehrerer bereits abgegebener Applikationsdosen, welche also z. B. vor dem nächsten Abgabezeitpunkt oder in der Vergangenheit liegen und/oder der Höhen dieser Applikationsdosen speichern und/oder aufweisen.

Die Medikamentenabgabevorrichtung kann eine Erfassungsvorrichtung aufweisen, oder hiermit verbunden sein, welche programmiert ist, die Betätigung der Abrufvorrichtung durch den Benutzer zu erfassen. Das Betätigungsverhalten kann zumindest eine zu einem Betätigungszeitpunkt erfolgte oder erfolgende Betätigung der Abrufvorrichtung durch den Benutzer erfassen und ist vorzugweise entsprechend ausgestaltet, programmiert und/oder vorgesehen. Das Betätigungsverhalten hat zum Ziel, eine nächste oder künftige Applikationsdosis auszulösen, abgeben oder applizieren zu lassen und/oder um die Umstände hierfür zu überprüfen, beispielsweise, ob ein vorausgehender Abgabezeitpunkt bereits eine ausreichend lange Zeitdauer zurückliegt.

Die elektronische Steuervorrichtung ist programmiert, um im Datenspeicher gespeicherte Daten auszulesen. Sie ist ferner programmiert, um das Betätigungsverhalten des Benutzers, das mittels der Erfassungsvorrichtung erfasst wurde, auszuwerten.

Weiter ist sie programmiert, um zu einem Vorbestimmungszeitpunkt (oder Bestimmungszeitpunkt), die Höhe der nächsten Applikationsdosis, z. B. aus einer Vielzahl von möglichen oder festlegbaren Applikationsdosen, vorzubestimmen oder zu bestimmen, etwa auszuwählen, zu errechnen oder festzulegen, welche als nächstes, z. B. zu einem nächsten Abgabezeitpunkt, an den Benutzer abgebbar oder für eine Abgabe an diese abgebbar ist, sollte dieser außerhalb einer Sperrdauer, falls eine solche gesetzt ist, die Abrufvorrichtung betätigen.

Ein solcher Vorbestimmungszeitpunkt liegt zu oder nach dem Betätigungszeitpunkt. Der Schritt des Vorbestimmens erfolgt unter Berücksichtigung von aus dem Datenspeicher ausgelesenen Daten. Die ausgelesenen Daten umfassen hierbei vorzugsweise zumindest einerseits den Abgabezeitpunkt einer oder mehrerer der bereits abgegebenen Applikationsdosen, oder die zwischen dem oder den mehreren Abgabezeitpunkten der bereits abgegebenen Applikationsdosen und dem zu oder nach dem nächsten Betätigungszeitpunkt liegenden Abgabezeitpunkt verstrichenen Zeitdauer. Alternativ oder ergänzend umfassen die ausgelesenen Daten vorzugweise andererseits die Höhe einer oder mehrerer der insbesondere mittels der Medikamentenabgabevorrichtung bereits abgegebenen Applikationsdosen.

Das erfindungsgemäße System umfasst einen oder mehrere erfindungsgemäße Nasenapplikatoren sowie ein oder mehrere Peripheriegeräte, wobei einer oder mehrere der Nasenapplikatoren, insbesondere mittels seiner Steuervorrichtung, mit einem oder mehreren der Peripheriegeräte in Signal- oder Kommunikationsverbindung stehen oder hierzu vorbereitet oder programmiert sind.

Wenn hierin von einem Benutzer die Rede ist, so umfasst dies den Patienten, der sich selbst mediziert, den Pfleger, die Ärztin, Angehörige von Patienten, und mehr, der z. B. auf Geheiß des Patienten die Medikamentenabgabevorrichtung wie hierin beschrieben bei der Medikation des Patienten verwendet.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert", "vorgesehen" oder "konfiguriert" die Rede ist, so ist auch offenbart, diese Begriffe gegeneinander auszutauschen.

Wenn hierin von "programmiert", "vorgesehen" oder "konfiguriert" zum Ausführungen eines Schritts oder einer Handlung die Rede ist, so ist auch offenbart, dass dieser Schritt oder diese Handlung optional automatisch vorgenommen wird, z. B. von der Steuervorrichtung.

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Erfindungsgemäße Ausführungsformen können eines oder mehrere der oben und/oder im Folgenden genannten Merkmale in jeder technisch möglichen Kombination aufweisen.

Wann immer hierin eine Eignung, ein Zweck, ein Schritt oder ein Verfahrensschritt genannt ist, umfasst die vorliegende Erfindung auch eine entsprechende Programmierung oder Konfiguration einer zum Erzielen oder Ausführen geeigneten Vorrichtung oder eines Abschnitts hiervon.

Auch wenn die vorliegende Erfindung nicht auf einen Nasenapplikator beschränkt ist, sondern Medikamentenabgabevorrichtungen als solche betrifft, wird im Folgenden schwerpunktmäßig auf Nasenapplikatoren eingegangen. Zum Nasenapplikator gemachte Ausführen, Erläuterungen und Vorteile treffen ungeschmälert auch auf Medikamentenabgabevorrichtungen zu, und umgekehrt.

In manchen Ausführungsformen umfasst der Datenspeicher weiter pharmakologische, pharmakodynamische, pharmakometrische und/oder pharmakokinetische Daten, insbesondere die Substanz betreffend, die appliziert wird oder werden soll. Diese Daten können Modelle, insbesondere PK-Modelle, oder PK-Kurven, sein oder umfassen. Diese Daten können Formeln sein und/oder basierend auf ihnen erzeugt werden, z. B. on-line oder in *real time* (Echtzeit), oder erzeugt worden sein. Sie können patientenindividuell erfasst bzw. zusammengestellt worden sein. Alternativ oder ergänzend können auch andere, der Substanz zugeordnete, Daten vom Datenspeicher umfasst oder dort hinterlegt sein.

Zu den Daten können beispielsweise die Dosishalbwertzeit und/oder die Eliminations- oder terminale Halbwertszeit der Substanz zählen.

Die Dosishalbwertszeit ist die benötigte Zeit, bis die Konzentration (z. B. im Plasma, im Blut, am Wirkort (ZNS, zentrales Nervensystem), im Fettgewebe, in der Leber, etc.) auf den halben Wert (50%) der verabreichten Dosis gesenkt ist.

Die Eliminations- oder terminale Halbwertszeit ist die benötigte Zeit, bis die bei Pseudo-Equilibrium vorherrschende Konzentration um auf den halben Wert (50%) absinkt.

Die elektronische Steuervorrichtung ist optional programmiert, um zusätzlich auch solche Daten aus dem Datenspeicher auszulesen.

Ferner erfolgt hierbei das Vorbestimmen der nächsten oder weiteren Applikationsdosis der Substanz unter zusätzlicher Berücksichtigung der zusätzlich ausgelesenen Daten. Dabei kann der Vorbestimmungszeitpunkt, wie optional auch der Betätigungszeitpunkt, dem Abgabezeitpunkt der nächsten Applikationsdosis entsprechen, er kann aber auch vor dem Abgabezeitpunkt liegen.

Das Sperrdauerende der Sperrdauer kann dem Zeitpunkt der maximalen Konzentration (dieser Zeitpunkt ist hierin auch bezeichnet als: tₘₐₓ) zwischen Abgabe zweier aufeinander folgenden Applikationsdosen der Substanz entsprechen oder hiervon abgeleitet sein.

Die Sperrdauer kann in bestimmten Ausführungsformen vom Konzentrationsmaximum abhängen und entsprechend vom Arzt oder anderen berechtigten Personen oder automatisch von der Medikamentenabgabevorrichtung, alternativ von einer definierten Konzentration, alternativ von der Zeit bis diese definierte Konzentration unterschritten wird.

In einigen Ausführungsformen ist die elektronische Steuervorrichtung weiter programmiert zum Veranlassen eines Abgebens der nächsten oder der weiteren Applikationsdosis in der vorbestimmten Höhe zum entsprechenden Abgabezeitpunkt mittels des Abgabedosierers. Die elektronische Steuervorrichtung ist ferner programmiert, um nachfolgend die Höhe und/oder eine daraus resultierende Konzentration der Applikationsdosis, beispielsweise als Mengenangabe oder Volumenangabe usw. der Substanz, der abgegebenen nächsten Applikationsdosis im Datenspeicher zu speichern oder speichern zu lassen. Weiter ist die Steuervorrichtung vorzugsweise programmiert, um beispielsweise zeitgleich oder nachfolgend zur Abgabe der Applikationsdosis die Sperrvorrichtung zum zeitweisen Sperren des Abgabedosierers für eine bestimmte Sperrdauer zu veranlassen.

Die Sperrdauer beginnt beispielsweise ab oder nach dem Abgabezeitpunkt dieser nächsten Applikationsdosis und ist definiert durch einen Sperrdauerbeginn und/oder ein Sperrdauerende, was auch auf beliebige andere hierin genannte Sperrdauern zutreffen kann.

Der Abgabezeitpunkt der abgegebenen, nächsten Applikationsdosis kann im Datenspeicher gespeichert werden, die Steuervorrichtung kann hierzu programmiert sein.

In manchen Ausführungsformen ist die elektronische Steuervorrichtung weiter programmiert, um die Abfolge der weiteren vorstehend genannten Schritte auch im Zusammenhang mit einer oder mehreren der auf die nächste Applikationsdosis folgenden Abgaben von weiteren Applikationsdosen, die zeitlich nach der nächsten Applikationsdosis liegen, auszuführen oder zu veranlassen.

In einigen Ausführungsformen ist die elektronische Steuervorrichtung programmiert, aus dem Datenspeicher Daten auszulesen, welche z. B. PK-Kurven und/oder PK-Modelle umfassen, und welche einen zeitlichen Verlauf der Konzentration der Substanz im Körper, insbesondere im Blut, des Patienten abbilden. Basierend auf diesen Kurven oder Modellen kann die elektronische Steuervorrichtung ein nächstes Maximum der Konzentration errechnen. Das nächste Maximum kann das Maximum sein, das die Konzentration zwischen unmittelbar aufeinanderfolgenden Abgabezeitpunkten einnimmt. Es kann das Ergebnis kumulierter Applikationsdosen sein.

In bestimmten Ausführungsformen wird dieses Maximum der Konzentration errechnet und zur Festlegung der Sperrdauer herangezogen. Beim Festlegen der Sperrdauer kann der Zeitpunkt, zu welchem dieses Maximum erzielt wird, berücksichtigt werden.

In manchen Ausführungsformen ist die elektronische Steuervorrichtung weiter programmiert, die nächste Applikationsdosis derart zu bestimmen, dass das nächste Maximum der Konzentration einen vorgegebenen Maximalwert oder ein vorgegebenes Maximum nicht übersteigen wird. Die Bestimmung der Applikationsdosis erfolgt in diesen Ausführungsformen basierend auf im Datenspeicher gespeicherten Daten, insbesondere PK-Kurven oder Modelle, und einem im Datenspeicher vorgehaltenen, therapeutischen Maximum. Das therapeutische Maximum kann das Maximum einer einzelnen Applikationsdosis sein oder das Maximum einer Summe von mehreren Applikationen.

In einigen Ausführungsformen umfasst das Auswerten des erfassten Betätigungsverhaltens des Benutzers das Feststellen des Betätigungszeitpunkts, zu welchem der Benutzer die nächste oder die weitere Applikationsdosis abrufen möchte. Alternativ umfasst das Auswerten des Benutzungsverhaltens die Zeitspanne, die zwischen diesem Betätigungszeitpunkt und dem Sperrdauerende der zuletzt abgelaufenen Sperrdauer liegt.

Hierbei umfasst das Vorbestimmen das Berücksichtigen des festgestellten nächsten Betätigungszeitpunkts oder das Berücksichtigen der festgesellten Zeitspanne.

In manchen Ausführungsformen weist die Medikamentenabgabevorrichtung oder der Nasenapplikator weiter eine Feedbackvorrichtung auf. Sie dient dem Benutzer, ein Feedback z. B. zu seinem Wohlbefinden, seinem Schmerzzustand oder dergleichen abzugeben. Die Feedbackvorrichtung, welche ein Schalter, eine Touchfläche, usw. sein kann, kann z. B. vorgesehen sein, um vom Benutzer dann betätigt zu werden, wenn, oder solange er schmerzfrei ist oder auf andere Weise eine Aussage über die Wirkung der Substanz in seinem Körper machen kann. So kann verabredet sein, dass der Benutzer die Feedbackvorrichtung erstmals dann betätigt, z. B. drückt, wenn der Schmerz nach der dem Benutzer zuletzt applizierten Applikationsdosis erstmals als erträglich empfunden wird. Weiter kann verabredet sein, dass der Benutzer die Feedbackvorrichtung ab dem Zeitpunkt nicht mehr betätigt, zu dem er erstmals wieder (aufgrund nachlassender Konzentration der Substanz im Körper) Schmerzen empfindet.

Die Feedbackvorrichtung kann mit der Abrufvorrichtung identisch sein, z. B. mit einer eigenen Schalterauswertung, etwa indem sie länger, kürzer, öfter oder auf andere Weise anders zu betätigen ist als die Abrufvorrichtung, alternativ kann sie eine eigene Vorrichtung zum Betätigen durch den Benutzer sein.

Hierbei ist die Erfassungsvorrichtung programmiert zum Erfassen einer erfolgten Betätigung der Feedbackvorrichtung durch den Benutzer zu wenigstens einem, vorzugsweise ersten, Feedbackzeitpunkt.

Ferner ist die Steuervorrichtung hierbei programmiert zum Ermitteln der im Körper oder im Blut des Benutzers vorliegenden Konzentration der Substanz zu dem wenigstens einen Feedbackzeitpunkt.

Ein Ermitteln kann hierin stets ein Errechnen, Aus- und/oder Ablesen sein.

Ergänzend ist die Steuervorrichtung programmiert zum Festsetzen einer Zielkonzentration oder ihres Maximalwerts auf einen Wert jeweils unterhalb, oberhalb oder gleich der ermittelten Konzentration.

Immer wenn hierin von einer "Zielkonzentration" die Rede ist, kann dies die Konzentration sein, die einen gewünschten therapeutischen Effekt bewirkt.

In einigen Ausführungsformen ist die Erfassungsvorrichtung weiter programmiert zum Erfassen von wenigstens einer Betätigung der Abrufvorrichtung durch den Benutzer zu unterschiedlichen Betätigungszeitpunkten, insbesondere nach der Sperrdauer. Alternativ oder ergänzend ist die Erfassungsvorrichtung programmiert zum Erfassen eines, Feedbackzeitpunkts, vorzugsweise eines letzten Feedbackzeitpunkts, ab welchem weitere Betätigungen der Feedbackvorrichtung durch den Benutzer zumindest bis zum Abgabezeitpunkt der nächsten Applikationsdosis ausbleiben. Optional ist von der vorliegenden Erfindung auch das Speichern dieser Feedbackzeitpunkte umfasst.

Die Steuervorrichtung kann weiter programmiert sein zum Ermitteln von Konzentrationen der Substanz im Körper, insbesondere im Blut, etwa anhand der ausgelesenen Daten wie z. B. PK-Kurven, PK-Modellen, usw. Hierbei ist beispielsweise sowohl die Konzentration zum, vorzugsweise ersten, Feedbackzeitpunkt als auch die Konzentration zum, vorzugsweise ersten, Betätigungszeitpunkt (z. B. seit Ende der letzten Sperrdauer) oder zum letzten Feedbackzeitpunkt relevant. Diese Konzentrationen können den jeweiligen Zeitpunkten zugeordnet sein oder werden.

Ferner kann die Steuervorrichtung weiter programmiert sein zum Ermitteln oder Auswählen eines zeitlichen Konzentrationsverlaufs aus einer Gruppe von zeitlichen Konzentrationsverläufen. Beim Ermitteln des zeitlichen Konzentrationsverlaufs wird sowohl die Konzentration zum, vorzugsweise ersten, Feedbackzeitpunkt als auch die Konzentration zum, vorzugsweise ersten Betätigungszeitpunkt oder dem letzten Feedbackzeitpunkt berücksichtigt.

Wenn hierin von einer "Gruppe von zeitlichen Konzentrationsverläufen" die Rede ist, so ist dies eine Gruppe von Konzentrationsverläufen, die sich auf dieselbe Dosis (z. B. 50 µg Fentanyl) beziehen oder die hiervon abgeleitet werden. Insbesondere umfassen die Konzentrationsverläufe solche, die den zeitlichen Verlauf der Konzentration für den Mittelwert aller mittels dieser Dosis untersuchten Patienten eines Kollektivs widerspiegeln, oder den plus/minus eine oder mehrere Standardabweichungen. Eine Vielzahl solcher Verläufe oder Daten können im Datenspeicher gespeichert sein. Aus ihr kann die Gruppe der betrachteten zeitlichen Verläufe ausgewählt sein.

Ferner kann die Steuervorrichtung programmiert sein zum Berücksichtigen des so ermittelten zeitlichen individuellen Konzentrationsverlaufs beim Vorbestimmen der Höhe einer oder mehrerer der nachfolgenden Applikationsdosen, insbesondere zum Berücksichtigen der ermittelten individuellen PK-Kurve.

In manchen Ausführungsformen des Nasenapplikators ist die Steuervorrichtung weiter programmiert, um beim Vorbestimmen der Höhe der nächsten Applikationsdosis die festgesetzte Zielkonzentration oder ihren Maximalwert zu berücksichtigen. Alternativ oder ergänzend kann vorgesehen sein, dass beim Vorbestimmen der ermittelte zeitliche individuelle Konzentrationsverlauf, insbesondere die ermittelte individuelle PK-Kurve, berücksichtigt wird.

In einigen Ausführungsformen ist die Steuervorrichtung weiter programmiert, um nach der Abgabe einer Ausgangsdosis oder Initialdosis zu einem früheren Zeitpunkt, zu jeweils einem ersten (oder hier als "initial" bezeichneten) Zeitpunkt der Betätigung der Abrufvorrichtung durch den Benutzer (vorzugsweise nach Ablauf einer sich an die Abgabe der Ausgangsdosis anschließende Sperrdauer, in welcher dem Benutzer auf Betätigen der Abrufvorrichtung hin keine Applikationsdosis appliziert wird), eine, diesem Betätigungszeitpunkt zugeordnete Konzentration zu ermitteln, und zwar für jeden der (im Datenspeicher hinterlegten) Konzentrationsverläufe der/einer Gruppe zu ermitteln oder hiervon abzuleiten. Dieser erste Betätigungszeitpunkt liegt zeitlich nach dem Abgabezeitpunkt der Ausgangsdosis. Die Steuervorrichtung ist weiter programmiert, um die so ermittelten Konzentrationen jeweils als geschätzte Zielkonzentration des zugehörigen zeitlichen Konzentrationsverlaufs festzulegen. Hat es, vereinfacht gesprochen, eine Ausgangsdosis gegeben, und hat der Benutzer nach Ablauf einer auf diese folgende Sperrdauer eine weitere Applikationsdosis abrufen wollen, so werden dem Zeitpunkt, zu welchem der Benutzer versucht hat, die weitere Applikationsdosis abzurufen, für jeden der betrachteten zeitlichen Konzentrationsverlauf eine Konzentration zugeordnet. Da die so zugeordnete Konzentration rechnerisch ermittelt wurde und das Ergebnis einer Hypothese ist, welche noch einer Überprüfung und Bestätigung bedarf, wird die so ermittelte Konzentration hierin als geschätzte Zielkonzentration bezeichnet. Hat die Gruppe von betrachteten zeitlichen Verläufen drei Elemente (Verläufe), so erhält man drei geschätzte Zielkonzentrationswerte. Man stellt, anders ausgedrückt, drei Hypothesen auf. Sie besagen, wie hoch die Konzentration im Körper sein müsste, würde der erste, der zweite oder der dritte Konzentrationsverlauf auf der Gruppe für den betrachteten Benutzer zutreffen.

Eine Ausgangsdosis kann hierin auch als erste oder vorausgehende Applikationsdosis bezeichnet werden, welche zu einem Abgabezeitpunkt, der entsprechend der erste Abgabezeitpunkt sein kann, abgegeben wurde. Die Ausgangsdosis kann die tatsächlich allererste Applikation mittels der Medikamentenabgabevorrichtung sein.

Die Steuervorrichtung kann ferner zum Applizieren der auf die Ausgangsdosis folgenden ersten Applikationsdosis als Reaktion auf die Betätigung der Abrufvorrichtung zum ersten Betätigungszeitpunkt programmiert sein.

Weiter kann die Steuervorrichtung zum Ermitteln eines Zeitpunkts, zu welchem nach dem Applizieren dieser ersten Applikationsdosis die Konzentration im Körper, insbesondere im Blut, jeweils erneut auf die geschätzte Zielkonzentration des zugehörigen zeitlichen Konzentrationsverlaufs abgesunken sein wird oder sein sollte. Dieser Zeitpunkt wird hierin auch als berechneter Zielkonzentrationszeitpunkt bezeichnet. Um ihn jeweils (also für jeden Konzentrationsverlauf aus der Gruppe) zu berechnen, werden jeweils erneut dieselben zeitlichen Konzentrationsverläufe der Gruppe herangezogen, für die jeweils bereits, ein berechneter Zielkonzentrationszeitpunkt festgelegt ist. Anzumerken ist, dass Unterschiede zwischen der Höhe der Ausgangsdosis und der nachfolgenden, ersten Applikationsdosis hierbei berücksichtigt werden. Betrug die Ausgangsdosis beispielsweise 50 µg Fentanyl, so haben die drei ausgewählten zeitlichen Konzentrationsverläufe der Gruppe angegeben, wie diese 50 µg Fentanyl im Körper nachzuweisen sein könnten. Ein erster der drei Konzentrationsverläufe könnte z. B. annehmen, dass der Verlauf sich wie in einem Kollektiv durchschnittlich beobachtet verhalten wird. Ein zweiter kann z. B. annehmen, dass sich der Verlauf wie der erste Verlauf um plus eine Standardabweichung, plus 10%, usw. verhält. Ein dritter kann z. B. annehmen, dass der Verlauf sich wie der erste Verlauf um minus eine Standardabweichung, minus 10%, usw. verhält. Beträgt die nachfolgende, erste Applikationsdosis nun aber beispielsweise nicht ebenfalls 50 µg, sondern nur 30 µg Fentanyl, so werden jeweils dieselben drei ausgewählten zeitlichen Konzentrationsverläufe der Gruppe angenommen. Sie sind nur nicht mehr auf 50 µg, sondern auf 30 µg Fentanyl berechnet.

Die Steuervorrichtung kann ferner zum direkten oder indirekten Erfassen oder Feststellen des zweiten Betätigungszeitpunkts zum Abrufen einer zweiten oder auf die nachfolgenden, erste Applikationsdosis ihrerseits nachfolgende, z. B. zweite, Applikationsdosis programmiert sein.

Ergänzend kann die Steuervorrichtung programmiert sein, um einen zeitlichen Konzentrationsverlauf aus der Gruppe als individuellen zeitlichen Konzentrationsverlauf, insbesondere als individuelle PK-Kurve zu ermitteln. Hierbei wird die jeweilige Differenz zwischen dem berechneten Zielkonzentrationszeitpunkt eines jeden zeitlichen Konzentrationsverlaufs aus der Gruppe und dem zweiten Betätigungszeitpunkt berücksichtigt. Als individueller zeitlicher Konzentrationsverlauf kommt insbesondere jener zeitliche Konzentrationsverlauf in Frage, welcher die geringste Differenz zwischen seinem berechneten Zielkonzentrationszeitpunkt und dem zweiten Betätigungszeitpunkt aufweist.

Die Steuervorrichtung kann ferner programmiert sein, um beim Vorbestimmen der Höhe einer oder mehrerer der nachfolgenden Applikationsdosen den ermittelten zeitlichen individuellen Konzentrationsverlauf zu berücksichtigen, was insbesondere eine individuelle PK-Kurve oder ein individuelles PK-Modell sein kann.

In manchen Ausführungsformen ist die Steuervorrichtung weiter programmiert zum Ermitteln der im Körper oder im Blut des Benutzers vorliegenden Konzentration der Substanz. Das Ermitteln erfolgt zu wenigstens einem betrachteten Betätigungszeitpunkt oder zu wenigstens einem betrachteten Vorbestimmungszeitpunkt. Ferner ist die Steuervorrichtung programmiert, diese Konzentration als die Zielkonzentration festzusetzen.

Hierbei ist die Steuervorrichtung weiter programmiert, um am nächsten Vorbestimmungszeitpunkt die Höhe der/einer nächsten Applikationsdosis derart festzusetzen, dass die Konzentration im Körper oder im Blut des Benutzers erst nach Ablauf einer vorbestimmten Zeitdauer erneut auf die Zielkonzentration absinken wird. Diese Zeitdauer kann einstellbar sein, beispielsweise durch den behandelnden Arzt, und beginnt mit dem Abgabezeitpunkt der nächsten Applikationsdosis.

Hierzu kann analog wie oben beschrieben der unveränderliche zeitliche individuelle Konzentrationsverlauf, also die PK-Kurve des Benutzers zugrunde gelegt und/oder berücksichtigt werden.

In einigen Ausführungsformen ist die elektronische Steuervorrichtung weiter programmiert, um im Schritt des Vorbestimmens der Höhe der nächsten Applikationsdosis aus dem Datenspeicher ausgelesene Daten zu berücksichtigen. Diese Daten umfassen sowohl den Abgabezeitpunkt einer oder mehrerer der bereits abgegebenen Applikationsdosen einerseits als auch die Höhe einer oder mehrerer der bereits abgegebenen Applikationsdosen andererseits. Es ist von der vorliegenden Erfindung umfasst, dass hierbei alternativ zum Abgabezeitpunkt die zwischen dem oder den mehreren Abgabezeitpunkten der bereits abgegebenen Applikationsdosen und dem nach dem nächsten Betätigungszeitpunkt liegenden Abgabezeitpunkt verstrichenen Zeitdauer betrachtet werden kann.

In manchen Ausführungsformen umfasst das Auswerten des Betätigungsverhaltens des Benutzers, welches mittels der Erfassungsvorrichtung erfasst wird, das Erfassen von Betätigungen der Abrufvorrichtung durch den Benutzer zu Betätigungszeitpunkten innerhalb einer Sperrdauer. Dies kann in einer beliebigen, einer bestimmten, d. h, in der ersten und/oder in der zweiten usw., oder in jeder Sperrdauer erfolgen. In bestimmten Ausführungsformen ist optional auch das Speichern dieses Betätigungsverhaltens in oder auf einer hierzu geeigneten Vorrichtung, z. B. dem hierin genannten Datenspeicher, vorgesehen.

Die Steuervorrichtung ist weiter programmiert, um eine Konzentration der Substanz im Körper, insbesondere im Blut, zu ermitteln, die den jeweiligen Betätigungszeitpunkten innerhalb einer der Sperrdauern zugeordnet ist oder sein kann.

Die Steuervorrichtung ist weiter programmiert, um eine Zielkonzentration oder ihrem Mindestwert auf einen Wert jeweils oberhalb, unterhalb oder gleich der zugeordneten Konzentration festzusetzen.

In bestimmten Ausführungsformen kann das oben ausgeführte Vorgehen mehrfach wiederholt, d. h. in Schleife, ausgeführt werden. Die so angenäherte Zielkonzentration, oder der so angenäherte Mindestwert kann optional nach einer oder jeder Sperrdauer auf Null (0) zurückgesetzt werden.

In einigen Ausführungsformen ist die Erfassungsvorrichtung des erfindungsgemäßen Nasenapplikators weiter programmiert, um wenigstens eine Betätigung der Abrufvorrichtung durch den Benutzer zu wenigstens einem Betätigungszeitpunkt nach der Sperrdauer zu erfassen und optional zu speichern.

Hierbei ist die Steuervorrichtung weiter programmiert zum Ermitteln einer Konzentration im Körper, insbesondere im Blut, welche dem Betätigungszeitpunkt nach der Sperrdauer jeweils zugeordnet ist.

Weiter ist die Steuervorrichtung programmiert zum Ermitteln eines zeitlichen Konzentrationsverlaufs der Gruppe. Hierbei wird sowohl wenigstens ein, vorzugsweise der letzte, Betätigungszeitpunkt innerhalb der (beliebigen) Sperrdauer als auch wenigstens ein, vorzugsweise der erste, Betätigungszeitpunktes nach der Sperrdauer berücksichtigt.

Die Steuervorrichtung ist ferner programmiert, um beim Vorbestimmen der Höhe einer oder mehrerer der nachfolgenden Applikationsdosen den ermittelten zeitlichen individuellen Konzentrationsverlauf zu berücksichtigen, insbesondere die ermittelte individuelle PK-Kurve.

In manchen Ausführungsformen des Nasenapplikators ist die Steuervorrichtung weiter programmiert, um beim Vorbestimmen der Höhe der nächsten Applikationsdosis die festgesetzte Zielkonzentration oder ihren Mindestwert und/oder den ermittelten zeitlichen individuellen Konzentrationsverlauf, insbesondere die ermittelte individuelle PK-Kurve, zu berücksichtigen.

In einigen Ausführungsformen umfasst das Auswerten des Betätigungsverhaltens des Benutzers, das mittels der Erfassungsvorrichtung erfasst wird, das Auswerten von Betätigungen der Abrufvorrichtung durch den Benutzer zu Betätigungszeitpunkten innerhalb einer der Sperrdauern. Dies kann in einer beliebigen, einer bestimmten, d. h, in der ersten und/oder in der zweiten usw., oder in jeder Sperrdauer erfolgen. In bestimmten Ausführungsformen ist optional auch das Speichern dieses Betätigungsverhaltens in oder auf einer hierzu geeigneten Vorrichtung vorgesehen.

Weiter kann die Steuervorrichtung programmiert sein zum Ermitteln einer Zunahme der Zeitabstände zwischen aufeinanderfolgenden Betätigungszeitpunkten innerhalb einer der (betrachteten) Sperrdauern, alternativ des Ausbleibens weiterer Betätigungszeitpunkte innerhalb der betrachteten Sperrdauer. Ergänzend kann die Steuervorrichtung programmiert sein, um einen Zeitpunkt festzulegen, ab welchem das Maß der Zunahme oder das Ausbleiben vorbestimmten Kriterien genügt.

Als Kriterien kann beispielsweise festgelegt sein, dass, sobald z. B. 50%-75% von der Sperrdauer T_vainₙ verstrichen sind, man davon ausgehend darf, die Betätigungszeitpunkte auf vorbestimmte Weise wesentlich weiter auseinander liegen als zuvor, und/oder falls für eine, z. B. vorbestimmte, Zeitdauer keine Betätigung mehr erfolgt ist, davon ausgegangen wird, dass die Wirkung des therapeutischen Effekt spürbar/bemerkbar geworden ist und/oder die Zielkonzentration erreicht wurde.

Für manche Ausführungsformen kann festgelegt sein, dass, wenn rechnerisch z. B. 80% oder ein anderer, vorbestimmter Anteil der errechneten Maximalkonzentration erreicht wurde und es immer noch vergebliche Abrufversuche gibt, also der gewünschte therapeutische Effekt noch nicht erreicht wurde, das Ende der laufenden Sperrdauer vorverlegt wird, da die restlichen z. B. 20% der Konzentration nicht ausreichen werden, um den bei derzeitig angelegten Regime den Benutzer auf die Zielkonzentration (C_{ED}) zu bringen.

Für einige Ausführungsformen kann festgelegt sein, dass, wenn rechnerisch z. B. 80% der errechneten Maximalkonzentration erreicht wurde und es immer noch vergebliche Abrufversuche gibt, also der gewünschte therapeutische Effekt noch nicht erreicht wurde, der nächste Vorbestimmungszeitpunkt vorverlegt wird im Wissen, dass wahrscheinlich zum Ende der Sperrdauer ein Abrufversuch des Benutzers stattfinden wird.

Wenn diese Zeitintervalle größer werden, bedeutet dies, dass der gewünschte therapeutische Effekt, vielleicht langsam, aber auf jeden Fall anfängt einzusetzen. Somit kann im Fall, dass nach dem Ende der Sperrdauer eine Betätigung durch den Benutzer stattfindet, die Dosis kleiner ausfallen bzw. berechnet werden, wie wenn kein therapeutischer Effekt vorliegen würde.

Ferner kann die Steuervorrichtung programmiert sein zum Ermitteln einer dem Zeitpunkt zugeordneten Konzentration der Substanz im Körper, insbesondere im Blut.

Die Steuervorrichtung kann weiter programmiert sein, um die Zielkonzentration oder ihren Mindestwert auf einen Wert jeweils oberhalb, unterhalb oder gleich der zugeordneten Konzentration festzusetzen.

In manchen Ausführungsformen ist die elektronische Steuervorrichtung weiter programmiert zum Auswerten von wenigstens einer Betätigung der Abrufvorrichtung durch den Benutzer zu wenigstens einem Betätigungszeitpunkt, welcher nach der Sperrdauer liegt. Optional ist auch das Speichern der Betätigung in oder auf einer hierzu geeigneten Vorrichtung von der vorliegenden Erfindung umfasst.

Hierbei ist die Steuervorrichtung weiter programmiert, um eine Konzentration im Körper, insbesondere im Blut, zu ermitteln für den oder die Betätigungszeitpunkte nach der Sperrdauer.

Die Steuervorrichtung kann hierbei weiter programmiert sein, um einen zeitlichen Konzentrationsverlauf der/einer Gruppe unter Berücksichtigung sowohl eines, vorzugsweise des letzten, Betätigungszeitpunktes innerhalb der Sperrdauer als auch eines, vorzugsweise des ersten, Betätigungszeitpunktes nach der Sperrdauer zu ermitteln.

Ergänzend kann die Steuervorrichtung programmiert sein, beim Vorbestimmen der Höhe einer oder mehrerer der nachfolgenden Applikationsdosen den ermittelten zeitlichen individuellen Konzentrationsverlauf, insbesondere die ermittelte individuelle PK-Kurve zu berücksichtigen.

In einigen Ausführungsformen ist die Steuervorrichtung des Nasenapplikators weiter programmiert, um beim Vorbestimmen der Höhe der nächsten Applikationsdosis die festgesetzte Zielkonzentration oder ihren Mindestwert zu berücksichtigen.

Alternativ oder ergänzend kann die Steuervorrichtung den ermittelten zeitlichen individuellen Konzentrationsverlauf berücksichtigen, insbesondere die ermittelte individuelle PK-Kurve.

In manchen Ausführungsformen ist die Steuervorrichtung weiter zum Begrenzen der Höhe der nächsten Applikationsdosis und/oder zum Begrenzen der daraus resultierenden Konzentration programmiert.

Alternativ oder ergänzend ist die Steuervorrichtung zum Begrenzen der kumulierten Höhe aller innerhalb einer vorbestimmten Zeitdauer abgegebenen Applikationsdosen und/oder zum Begrenzen der daraus resultierenden Konzentrationen programmiert.

Wiederum alternativ oder ergänzend ist die Steuervorrichtung zum Begrenzen der kumulierten Höhe aller abgegebenen Applikationsdosen und/oder zum Begrenzen der daraus resultierenden Konzentrationen programmiert.

Hierzu greift die Steuervorrichtung optional auf im Datenspeicher gespeicherte Daten zurück, die die Substanz betreffen.

In einigen Ausführungsformen ist der erfindungsgemäße Nasenapplikator geeignet zur nasalen Verabreichung einer Applikationsdosis von Substanzen, welche insbesondere medizinische Wirkstoffe sind oder aufweisen.

In anderen Ausführungsformen betrifft die Erfindungen eine Medikamentenabgabevorrichtung, welche kein Nasenapplikator ist. Was hierin zum Nasenapplikator ausgeführt ist, gilt daher uneingeschränkt auch für eine Medikamentenabgabevorrichtung als einen Nasenapplikator, insbesondere wenn diese tragbar und vom Benutzer, z. B. einem Patienten, allein betätigbar ist und üblicherweise auch ohne fremde Hilfe oder besonderes Wissen betätigt wird.

Insbesondere ist eine Medikamentenabgabevorrichtung für andere Verabreichungsarten geeignet, beispielsweise für weitere transmukosale Verabreichungen, insbesondere über die Mundschleimhaut, beispielsweise in der Backentasche (bukkal) oder unter der Zunge (sublingual), für intravenöse oder auch für inhalative Verabreichungen.

In manchen Ausführungsformen ist oder umfasst der Abgabedosierer ein(en) Partikel- oder Tröpfchengenerator, beispielsweise eine Wirbelkammer, ein(en) Sprühkopf, eine Düse, eine Kanüle, ein(en) Tropfer, eine Lochplatte, ein(en) Baffle, eine poröse Membran oder dergleichen.

In einigen Ausführungsformen umfasst der Abgabedosierer einen Energiebeaufschlagungsmechanismus und/oder einen Fördermechanismus. Dieser kann mechanisch und/oder elektronisch ausgestaltet sein, z. B. als Mikropumpe, Kolbenpumpe, Rotationspumpe, Peristaltikpumpe, Kreiselpumpe oder dergleichen.

In manchen Ausführungsformen umfasst der Abgabedosierer eine analoge, insbesondere stufenlose, und/oder eine digitale (0 oder 1) Steuerung zur Dosierung. Diese kann Teil der elektronischen Steuervorrichtung des Nasenapplikators sein. Insbesondere entspricht eine Applikationsdosis oder Abgabemenge der Substanz einer Menge im Bereich zwischen 1 µl bis 300 µl pro Betätigung.

In einigen Ausführungsformen kann eine hohe Reproduzierbarkeit und Genauigkeit bei der Abgabe der Menge durch eine benutzerunabhängige Betätigung (alternativ: Auslösung) durch immer gleiche Auslöseparameter wie z. B. Auslösegeschwindigkeit, Auslösekraft, etc., Druck, Rotation, Hub, etc. gewährleistet sein.

In manchen Ausführungsformen umfasst der Nasenapplikator ein Dosierventil.

In bestimmten Ausführungsformen umfasst der Nasenapplikator einen, insbesondere abnehmbaren, Elastomerring zum Aufschieben auf einen Nasenaufsatz (auch bekannt als Nasendip). Der Elastomerring dient einem möglichst luftdichten Abschließen des Nasenlochs gegenüber einem Äußeren.

In einigen Ausführungsformen umfasst der Nasenapplikator, z. B. handelsübliche oder herkömmliche, Nasenspray-Kartuschen, die eine integrierte Förder- bzw. Pumpvorrichtung aufweisen. Mittels dieser integrierten Förder- bzw. Pumpvorrichtung können nach einer Entlüftung (Priming) verschiedene Dosiergroßen abgeben werden, beispielsweise mittels Verlängerung bzw. Verkürzung des Hubwegs dieser Vorrichtung.

In manchen Ausführungsformen wird eine Förder- bzw. Pumpvorrichtung vom Benutzer/Patienten mechanisch, also von Hand, ausgelöst. Hierzu kann beispielsweise ein Federspeicher manuell oder fremdenergetisch geladen, eingestellt und ausgelöst werden.

In einigen Ausführungsformen umfasst die Dosiserfassungseinrichtung des Nasenapplikators einen Sensor, konfiguriert zum Erfassen der abgegebenen Substanz oder deren Wirkstoffmenge. Das Erfassen der Dosis kann beispielsweise mittels direkter Messung, z. B. mittels eines Durchflusssensors, etc.) und/oder mittels abgeleiteter Messung erfolgen. Bei einer abgeleiteten Messung wird die Dosis rechnerisch ermittelt bzw. abgeleitet. Dies kann beispielsweise durch Messen von Umdrehungen (min⁻¹), Winkel, Hubweg, Weg, Hubanzahl, Zeit, Ventilbetätigungen (Anzahl und/oder Zeit) etc. erfolgen.

In manchen Ausführungsformen kann die Dosiserfassungseinrichtung beispielsweise in die Steuervorrichtung des Nasenapplikators integriert sein. In anderen Ausführungsformen liegt sie separat vor.

In einigen Ausführungsformen kann die Dosis mittels eines Flusssensors, eines Durchflusssensors oder mittels einer Tröpfchenverteilungsmessung und/oder Partikelverteilungsmessung erfolgen.

In manchen Ausführungsformen kann die Tröpfchenverteilung und/oder Partikelgrößenverteilung zur Ermittlung der Dosis gemessen werden.

In einigen Ausführungsformen kann mittels der Auslöseparameter, wie z. B. Auslösegeschwindigkeit, Auslösekraft etc., die Tröpfchengrößenverteilung und/oder Partikelgrößenverteilung eingestellt bzw. angepasst bzw. justiert werden. Dies ist insbesondere dazu vorgesehen, um auf einfache Art und Weise die Tröpfchenverteilung und/oder Partikelgrößenverteilung auf verschiedene Wirkstoffe anzupassen.

In manchen Ausführungsformen umfasst der Datenspeicher des Nasenapplikators wenigstens ein internes Speicherbauteil, welches nicht volatil und mehrfach überschreibbar bzw. beschreibbar ist und/oder ein externes Speicherbauteil, welches insbesondere austauschbar sein kann (z. B. SSD-Speicherkarte, USB-Speicher, HDD, etc.). Als externer Speicher kann alternativ oder ergänzend auch ein Netzwerk oder das Internet betrachtet werden.

In einigen Ausführungsformen kann, z. B. als finaler, Schritt des von der Steuervorrichtung getriggerten Verfahrens vorgesehen sein, am Ende der Behandlung oder Therapie ohne oder nach einer automatischen Datensicherung in oder auf einer hierzu geeigneten (weiteren)internen oder externen Speichervorrichtung die persönlichen Daten und/oder individuellen Steuerungsdaten aus dem Datenspeicher des Nasenapplikators zu löschen und/oder den Nasenapplikator auf die Werkseinstellungen zurückzusetzen. Damit kann der Nasenapplikator oder Teile hiervon nach Beendigung der Therapie des Patienten vorteilhafterweise an weitere Patienten weitergegeben werden, ohne dass Datenschutzbedenken dem entgegenstehen müssten.

In manchen Ausführungsformen kann der Datenspeicher, insbesondere der externe Datenspeicher, als digitaler Schmerzausweis implementiert sein und, beispielsweise auf Reisen oder bei Kontrollen, vom Benutzer/Patienten zum Nachweis gegenüber behandelnden Ärzten und/oder Kontrollierenden genutzt werden.

In einigen Ausführungsformen können der Benutzer/Patient im Zusammenwirken mit dem Nasenapplikator als vorprogrammiertes, selbstregulierendes System betrachtet werden, welches eine Programmierung überflüssig macht und potenzielle Fehlerquellen eliminiert. Ähnliches ist aus der Verwendung von *IV-PCA Smartpumps* zur Verabreichung von Opiaten bekannt. Das vorprogrammierte, selbstregulierende System umfasst bestimmte Dosisgrenzen (beispielsweise Dosis Limits, Konzentrationslimits, Zeitlimits, Limits basierend auf einem therapeutischen Effekt, d. h. z. B. eine geringere Dosis bei einem niedrigen Schmerzscore). Diese Dosisgrenzen können Dosisgrenzen pro Zeit, eine bestimmte Anzahl an Auslösungen oder Betätigungen, Grenzen einer Einzeldosis, ein Tagesdosislimit, ein Konzentrationslimit und/oder dergleichen sein. Bei Erreichen der Dosisgrenzen können zwei Konsequenzen erfolgen: Die Abgabe weiterer Applikationsdosen kann entweder pausiert werden oder die Dosis so runtergefahren werden, dass kein therapeutischer Effekt mehr auftreten kann. Insbesondere wird hier jedoch trotzdem ein Placebo-Effekt ermöglicht.

In manchen Ausführungsformen kann der Benutzer/Patient als Regelkreis betrachtet werden und das Auslöseverhalten des Nasenapplikators kann aufgrund der erfassten bzw. ermittelten pharmakologischen Daten entsprechend der Benutzer-/Patientenreaktion in puncto Dosis und Therapie angepasst werden. Hierzu können einerseits direkte Messwerte (Ist Werte), z. B. der Blutzuckerwert oder die Opioidkonzentration im Blut, oder andererseits indirekte Messwerte (abgeleitete und/oder geschätzte Werte), z. B. Pharmakokinetik("PK")-Kurven einer (Referenz-)Population, herangezogen werden. Vorzugsweise finden direkte Messwerte Eingang in die Anpassung des Auslöseverhaltens.

In einigen Ausführungsformen ist die elektronische Steuervorrichtung weiter programmiert, um einen Vorbestimmungszeitpunkt einer Applikationsdosis der Substanz vorbestimmen zu können, wobei subjektive therapeutische Beobachtungen eines patientenindividuellen therapeutischen Effekts, wie z. B. Schmerz, in Betracht gezogen werden. Diese Beobachtungen können das Schmerzniveau (*pain score*), den Schmerztypus, die Schmerzdauer, die Schmerzintensität und/oder die Schmerzfrequenz des Patienten sein oder umfassen.

Das bedeutet, dass der Benutzer/Patient bestimmt, was für ihn/sie noch erträglich ist bzw. wie stark er den Schmerz empfindet. Dies kann z. B. mittels manueller Eingabe auf einer eindimensionalen Ratingskala zum Feststellen der Schmerzintensität erfolgen. Solche Skalen können beispielsweise sein:
- eine numerische Ratingskala (NRS, beispielsweise von 1 (= schmerzfrei) bis 10 (= stärkste Intensität)),
- eine verbale Ratingskala (VRS, mit Beschreibungen der Schmerzintensität, beispielsweise "keine", "mäßig", "mittel", "schwer", "sehr schwer", oder auch Beschreibungen des zeitlichen Auftretens, beispielsweise "nie", "selten", "manchmal", "häufig", "immer"),
- visuelle Analogskala (VAS, beispielsweise als Linie, deren Endpunkte extreme Zustände darstellen, wie z. B. "kein Schmerz" und "unerträglicher Schmerz", auf welcher der Benutzer/Patient sein subjektives Schmerzempfinden markiert, ohne eine entsprechende Skalierung wahrzunehmen), oder eine
- *Functional Activity Scale* (FAS, beispielsweis mit den Einteilungen "keine Einschränkung" (*no limitation*), wenn der Benutzer eine bestimmte Aktivität ohne Einschränkung (z. B. aufgrund von Schmerzen) ausführen kann, "leichte Einschränkung" (*mild limitation*), wenn die Aktivität nur eingeschränkt ausgeführt werden kann (z. B. aufgrund von Schmerzen) und "erhebliche Einschränkung" (*significant limitation*)*,* wenn die Aktivität nicht ausgeführt werden kann (z. B. aufgrund von Schmerzen oder aufgrund von Nebenwirkungen einer Schmerztherapie).

In manchen Ausführungsformen können objektive und/oder subjektive Beobachtungen in Betracht gezogen werden. Diese können z. B. umfassen:
- Vitalwerte (wie Blutdruck, Herzfrequenz, Temperatur etc.),
- Pupillenreaktion,
- Delirium
- Halluzinationen
- Schwindel
- Toleranz
- Abhängigkeit
- gesteigerte Schmerzempfindlichkeit (Hyperalgesie)
- Sedation,
- Nebenwirkungen (wie Juckreiz (*Itching*), Übelkeit (*Nausea*), Erbrechen (*Vomiting*) etc.),
- physiologische Beobachtungen,
- Gesamtdosis/Volumen und/oder Gesamtkonzentration der Substanz/des Wirkstoffs,
- verbleibende Füllmenge im Behältnis.

In einigen Ausführungsformen kann die vorliegende Erfindung zu der sog. personalisierten Medizin (*Personalized Medicine*) gezählt werden, in deren Rahmen der Benutzer/Patient als vorprogrammiertes, selbstregulierendes System betrachtet und mit Dosisgrenzen (absolut) und/oder Dosisgrenzen pro Zeit, Konzentrationsgrenzen bzw. Mengengrenzen und/oder Beschränkung der Anzahl an Auslösungen und/oder Beschränkungen für eine Einzeldosis und/oder Tagesdosis, etc. sowie unten auf- und ausgeführte Alternativen eine möglichst individuelle Therapie angestrebt wird. Je mehr Input bzw. Information zum Status des Benutzers/Patienten verfügbar sind, umso individueller kann die Therapie gestaltet werden. Insbesondere, wenn hierbei Konzentrationsgrenzen betrachtet werden, können sich in bestimmten Ausführungsformen unterschiedlich hohe Dosen ergeben.

In manchen Ausführungsformen ist die elektronische Steuervorrichtung weiter programmiert, um einen Vorbestimmungszeitpunkt einer Applikationsdosis der Substanz vorbestimmen zu können, wobei alternativ oder ergänzend patientenspezifische Setup-Daten eingegeben und/oder in Betracht gezogen werden. Diese Setup-Daten können beispielsweise umfassen oder hervorgehen aus:
- Daten aus der (elektronischen) Patientenakte,
- Daten der individuellen Anamnese,
- Daten aus einem voroperativen Aufklärungsgespräch (*Pre-OP*),
- Daten die während einer Operation erhoben wurden (beispielsweise während einer Anästhesie),
- Daten, die in einem PACU (Post Anaesthesia Care Unit, Aufwachraum etc.) erhoben wurden und/oder
- individuellen anthropometrischen Patientendaten (beispielsweise Gewicht, Alter, Größe, Geschlecht etc.)

In einigen Ausführungsformen können alternativ oder ergänzend subjektive therapeutische Beobachtungen, z. B. mittels eines Schmerzscore wie hierin beschrieben, eingegeben werden und in Betracht gezogen werden.

In manchen Ausführungsformen ist die elektronische Steuervorrichtung weiter programmiert, um einen Vorbestimmungszeitpunkt einer Applikationsdosis der Substanz vorbestimmen zu können, indem alternativ oder ergänzend objektive therapeutische Beobachtungen in Betracht gezogen werden. Diese Beobachtungen können, vorzugsweise automatisch, mittels Sensordaten für Vitalwerte abgeglichen werden. Die Vitaldaten können beispielsweise umfassen:
- Herzfrequenz (HR),
- HRV,
- (partielle) Sauerstoffsättigung im Blut (SpO₂),
- Blutdruck,
- Blood pressure trending (BPT),
- elektrochemische Hautreaktionen,
- Temperatur,
- Photoplethysmogramm (PPG),
- Elektrokardiogramm (EKG),
- Atemfrequenz,
- Blutzuckerspiegel,
- Stress.

In einigen Ausführungsformen besteht die Möglichkeit, das mittels der Steuervorrichtung veranlasste Verfahren zu personalisieren, anzupassen, zu modifizieren etc. Dies kann mittels eines User-Interface, Verwaltungssoftware und/oder über Peripheriegeräte erfolgen.

In manchen Ausführungsformen erstellt das mittels der Steuervorrichtung veranlasste Verfahren Wertetabellen zur Steuerung des Nasenapplikators.

In einigen Ausführungsformen berechnet das mittels der Steuervorrichtung veranlasste Verfahren je nach Lage des Gerätes unterschiedliche Auslöseparameter für den Nasenapplikator.

In manchen Ausführungsformen können der circadiane, ultradiane und/oder infradiane Rhythmus des Benutzers/Patienten Berücksichtigung finden.

In einigen Ausführungsformen überträgt der Nasenapplikator die Daten aus dem Datenspeicher kabelgebunden und/oder drahtlos mittels entsprechender Protokolle, z. B. Wi-Fi, Bluetooth, WLAN, GPS, RFID, NFC, Barcode, QR-Code, ZigBee, Wibree, WiMAX, IrDA etc. und/oder steht mit dem Datenspeicher in Signalverbindung.

Wenn hierin von einer Signal- oder Kommunikationsverbindung zweier oder mehrerer Komponenten die Rede ist, so kann hierunter eine im Gebrauch bestehende Verbindung zu verstehen sein. Ebenso kann hierunter zu verstehen sein, dass eine Vorbereitung zu einer solchen (kabelgebunden, kabellosen oder auf andere Weise umgesetzte) Signalkommunikation besteht, beispielsweise durch eine Kopplung beider Komponenten, etwa mittels *pairing,* usw.

Unter *pairing* versteht man einen Prozess, um eine Verknüpfung zwischen Elektronikeinheiten zum Zwecke der Kommunikation herzustellen. Eines der bekanntesten Beispiele hierfür ist das Herstellen einer Bluetooth-Verbindung, mittels welcher verschiedenen Einrichtungen (z. B. Smartphone, Kopfhörer) miteinander verbunden werden. *Pairing* wird gelegentlich auch als *bonding* bezeichnet.

In manchen Ausführungsformen werden die hierin aufgeführten Daten mittels einer Schnittstelle zwischen Smart Device und Rechner übermittelt.

In einigen Ausführungsformen werden die hierin aufgeführten Daten webbasiert übermittelt, d. h. es ist keine Softwareinstallation notwendig.

In manchen Ausführungsformen werden die hierin aufgeführten Daten mittels einer verschlüsselten Datenübertragung übermittelt.

In einigen Ausführungsformen werden die hierin aufgeführten Daten vorzugsweise über eine einstellbare Reichweite hinweg übermittelt.

In manchen Ausführungsformen sind für den Nasenapplikator und die hiermit verbundenen Vorrichtungen vorzugsweise Low-Energy Lösungen vorgesehen, um möglichst wenig Strom zu verbrauchen.

In einigen Ausführungsformen ist für den Nasenapplikator und die hiermit verbundenen Vorrichtungen ein Flottenmanagement vorgesehen, d. h. ein Verwalten, Planen, Steuern und Überwachen von mehreren Nasenapplikatoren mit den verbundenen Vorrichtungen.

In manchen Ausführungsformen besteht eine Integrationsmöglichkeit des Nasenapplikators inklusive der hiermit verbundenen Vorrichtungen in eine bestehende IT-Infrastruktur. Es können hierzu Schnittstellen, z. B. mit klinischen Geräten und/oder mit anderen Medizinprodukten und/oder mit Diagnostikvorrichtungen und/oder mit einem Labor etc. vorgesehen sein.

In einigen Ausführungsformen können Schnittstellen zu elektronischen Patientenakten und/oder zu Krankenhaus Qualitätsberichten und/oder zu nationalen, europäischen und internationalen Datenerhebungsinitiativen vorgesehen sein. Somit kann auf Daten von z. B. interner und externer Forschung, auf interne und externe Umfragedaten (z. B. Patientenzufriedenheit, Nebenwirkungen etc.) zugegriffen werden. Innerhalb der Europäischen Union ist beispielweise die internationale statistische Klassifikation der Krankheiten und verwandter Gesundheitsprobleme (*International Statistical Classification of Diseases and Related Health Problems* (ICD-10) von Interesse, innerhalb Deutschlands können die diagnosebezogene Fallgruppierungen (*Diagnosis Related Groups* (DGRG)) sowie die Qualitätsverbesserung in der postoperativen Schmerztherapie (QUIPS) Vergleichsdaten liefern und in den USA die Programme der *Risk Evaluation and Mitigation Strategies* (REMS).

In manchen Ausführungsformen ist ein modulares/erweiterbares Dock oder eine Docking Station für den Nasenapplikator oder die elektronische Steuereinheit hiervon und/oder weitere Peripheriegeräte vorhanden. Das Dock oder die Docking Station dient zur Aufbewahrung und/oder Verwaltung des Nasenapplikators und/oder von Peripheriegeräten. Das Dock oder die Docking Station ist für die Aufbewahrung und/oder Verwaltung des Applikators und/oder von Peripheriegeräten geeignet. Das Dock kann z. B. zum Laden, für Software Updates, Pairing, Hardware Checks und/oder Freigaben usw. vorgesehen sein.

Das Dock oder die Docking Station zählt in manchen Ausführungsformen ebenfalls als Peripheriegerät. Optional dient das Dock oder die Docking Station in einigen Ausführungsformen jedoch nicht zum Datentransfer zwischen Dock oder Docking Station einerseits und einem mit ihr körperlich in Kontakt stehenden Nasenapplikator und/oder einem Server oder Cloudserver anderseits, in anderen hingegen schon.

In einigen Ausführungsformen sind das Dock oder die Docking Station als eigenständige IT-Infrastruktur, z. B. im Internet oder Intranet (via Hub/Router/Switch), vorgesehen. Alternativ oder ergänzend ist das Dock über entsprechende Schnittstellen in eine bestehende IT-Infrastruktur integrierbar und/oder integriert.

In einigen Ausführungsformen der vorliegenden Erfindung sind die Datenschnittstellen verkabelt, z. B. ausgestaltet als USB, USB-C, Thunderbolt, etc.

In manchen Ausführungsformen kann vorgesehen sein, mittels z. B. IBM Watson Zugang zu ausgelagerter Systemintelligenz zu schaffen und somit Ankopplung an bestehende Krankenhausinformationssysteme (KIS/PACS) über gängige Schnittstellen wie HL7 und DICOM bereitzustellen. Beispielsweise kann somit einem Techniker Zugriff auf den Nasenapplikator, auf die mit dem Nasenapplikator verbundenen Vorrichtungen, auf Interfaces/Schnittstellen und/oder auf die Software ermöglicht werden.

In einigen Ausführungsformen ist ein zentraler Server vorgesehen.

In manchen Ausführungsformen umfasst der Nasenapplikator, insbesondere dessen elektronische Steuervorrichtung, eine Energiespeichervorrichtung, insbesondere einen aufladbaren Akku oder eine Batterie.

In einigen Ausführungsformen kann die Energiespeichervorrichtung eine anwendungsfall- und/oder indikationsbezogene Batterieauslegung aufweisen. In diesen Ausführungsformen kann je nach Indikation eine unterschiedlich dimensionierte Energiespeichervorrichtung vorgesehen sein, insbesondere Energiespeichervorrichtungen mit unterschiedlicher Lebensdauer bzw. unterschiedlichen Laufzeiten. So kann postoperativ beispielsweise eine Energieversorgung von drei Tagen ausreichend sein, während bei der Behandlung von Migräne eine längere Energieversorgung (z. B. über zwei Wochen) vorteilhaft sein kann.

In manchen Ausführungsformen kann ein kabelloses Laden des Energiespeichers vorgesehen sein, beispielsweise mittels Pins (z. B. in einer Ladeschale) oder ohne Pins (z. B. Qi, mittels induktiver Energieübertragung).

In einigen Ausführungsformen kann ein verkabeltes Laden des Energiespeichers vorgesehen sein, beispielsweise mittels eines Netzteils.

In manchen Ausführungsformen können als Energiespeicher Batterien zum Wechseln vorgesehen sein, z. B. Einwegbatterien (Knopfzellen, AA, AAA, AAAA, Blockbatterien, etc.), wiederaufladbare Batterien, wiederaufladbare Batteriepacks, etc.

In einigen Ausführungsformen kann als Energiespeicher ein externer Stromanschluss vorgesehen sein.

In manchen Ausführungsformen ist das Substanzreservoir des Nasenapplikators integral vorgesehen, in anderen Ausführungsformen nicht integral, insbesondere Co-Packed, d. h. mit dem Nasenapplikator und/oder einer zugehörigen Komponente mitverpackt. Sowohl in den integralen als auch in den nicht integralen Ausführungsformen ist das Substanzreservoir (wieder-)befüllbar, insbesondere von außen.

In einigen Ausführungsformen ist das Substanzreservoir ein bereits vorgefülltes Einwegbehältnis und/oder ein vor Ort befüllbares Einwegbehältnis, z. B. gefertigt aus Glas, Kunststoff, Metall, Nicht-Eisen Metall, Verbundwerkstoffen, etc. oder aus einer Kombination dieser Materialien.

In manchen Ausführungsformen ist das Substanzreservoir vorgesehen mit einer positionsunabhängigen Auslöseeinrichtung, d. h. ein Auslösen kann mit einer Auslösefunktionalität in allen Achsen und Freiheitsgraden erfolgen. Dies kann beispielsweise durch ein kollabierbares und/oder ein energiebeaufschlagtes Behältnis und/oder dergleichen realisiert werden.

In einigen Ausführungsformen hat das Substanzreservoir ein fixes, in anderen Ausführungsformen ein variables Füllvolumen.

In manchen Ausführungsformen kann das Substanzreservoir konservierungsmittelfrei, in anderen Ausführungsformen mit konservierungsmittelhaltigen Substanzen befüllbar sein und/oder dafür geeignet sein.

In einigen Ausführungsformen ist das Substanzreservoir mit wenigstens einer Konnektierungsvorrichtung versehen, welche dazu dient das Substanzreservoir mit dem Abgabedosierer, insbesondere lösbar, zu verbinden. Dies kann mittels einer Luerverbindung, einer Nadel, einem Schraubverschluss, einer Steckverbindung, einer Klickverbindung, mittel Verpressens oder dergleichen erfolgen. Ferner können zur Verbindung auch mechanische Pins und/oder ein Formschluss von der vorliegenden Erfindung umfasst sein.

In manchen Ausführungsformen kann am Substanzreservoir eine Einrichtung, insbesondere ein Barcode und/oder eine RFID-Einrichtung, zur Erfassung und/oder Speicherung von ausgebbaren Informationen zur Rückverfolgbarkeit einer oder mehrerer Applikationen, vorgesehen sein. Diese können ferner z. B. eine Beschriftung, ein QR-Code, ein Chip, ein RFID/NFC-Tag, eine Bildaufnahme, welche jeweils eingescannt werden können, etc. oder eine Kombination hiervon sein oder umfassen. Applikationen sind beispielsweise Informationen über die Substanz wie Name, Verfallsdatum, pharmakologische Daten, Konzentration usw.

In einigen Ausführungsformen können am Substanzreservoir zur Fehlervermeidung im Sinne des Poka-Yoke- oder Schlüssel-und-Schloss-Prinzips technische Lösungen zum Einsatz kommen. Hierzu können beispielsweise eine Beschriftung, ein QR-Code, ein Barcode, ein Chip, ein RFID/NFC-Tag, eine Bildaufnahme, welche jeweils eingescannt werden können, etc. oder auch eine Kombination hiervon am Substanzreservoir vorgesehen sein. Entsprechende Steuer- und/oder Rechenvorrichtungen mit geeigneter Software können hierzu ebenfalls von der vorliegenden Erfindung umfasst sein. Damit kann u. a. vorteilhaft eine unsachgemäße Handhabung und/oder das Einsetzen eines falschen Substanzreservoirs in den Nasenapplikator vermieden werden. Mechanische Pins und/oder ein entsprechender Formschluss können ebenso diesem Ziel dienen und sind deshalb ebenfalls von der vorliegenden Erfindung umfasst.

In manchen Ausführungsformen ist das Substanzreservoir ein nicht-integrales, vorgefülltes Einwegbehältnis z. B. aus Glas, Kunststoff, Metall, Nicht-Eisen Metall, Verbundwerkstoffen, etc. oder einer beliebigen Kombination hiervon, welches getrennt vom Nasenapplikator, insbesondere von dessen Abgabedosierer, gelagert wird. Insbesondere werden hierdurch Kompatibilitätsprobleme zwischen dem/den Material(ein) des Substanzreservoirs der Substanz vermieden. Durch die getrennte Lagerung kann das Risiko, dass extrahierbare (*Extractables*) oder auslaugbare Stoffe (*Leachables*) in die Substanz migrieren, reduziert oder gar vermieden werden.

In manchen Ausführungsformen ist das Substanzreservoir ein nachfüllbares, integriertes Mehrwegbehältnis mit Filter, in anderen Ausführungsformen ohne Filter.

In einigen Ausführungsformen ist das Substanzreservoir ein externes Mehrwegbehältnis mit Filter, in anderen Ausführungsformen ohne Filter.

In manchen Ausführungsformen umfasst der Nasenapplikator ein zusätzliches Substanzreservoir, insbesondere ein Flüssigkeitsreservoir, in welchem eine weitere Substanz aufgenommen sein kann. Vorzugsweise unterscheidet sich die weitere Substanz von der ersten Substanz. Die weitere Substanz kann medizinisch oder nicht medizinisch sein, z. B. Hyaluronsäure, Ectoine, Aloe Vera, usw.

In einigen Ausführungsformen umfasst das Substanzreservoir des Nasenapplikators mehrere Kammern. In solchen Ausführungsformen kann der Nasenapplikator dazu geeignet sein, mehrere Therapien, z. B. Schmerzmittel oder Cannabinoide einerseits und z. B. und Insulin andererseits, zu unterstützen. In bestimmten Ausführungsformen kann vorgesehen sein, mit einer Substanz in der zweiten Kammer und/oder Reservoir die Substanz in der ersten Kammer und/oder Reservoir zu neutralisieren oder zu zerstören. Dies kann insbesondere bei Diebstahl oder Missbrauch von Vorteil sein.

In manchen Ausführungsformen umfasst der Nasenapplikator eine Kühlvorrichtung, geeignet um ein, mehrere oder alle Substanzreservoir(s), insbesondere dessen Inhalt, zu kühlen.

In einigen Ausführungsformen kann die Steuervorrichtung als Einwegvorrichtung vorgesehen sein, in anderen Ausführungsformen als wiederverwendbare elektronische Steuervorrichtung. Auch hybride Ausführungsformen, bei denen ein Teil der Steuervorrichtung wiederverwendbar und ein Teil als Einwegbauteil vorgesehen ist, sind von der vorliegenden Erfindung umfasst.

In manchen Ausführungsformen sammelt, speichert, überwacht, verarbeitet, berechnet, simuliert, regelt und/oder steuert die Steuervorrichtung eine, mehrere oder alle Funktionalitäten des Nasenapplikators.

In einigen Ausführungsformen wird der Nasenapplikator mittels der Steuervorrichtung angesteuert durch einen auf den Nasenapplikator aufgespielten Algorithmus (*Embedded Software) .*

In manchen Ausführungsformen kann der Abgabedosierer mittels der Steuervorrichtung separat angesteuert werden oder zusammen mit der Dosiserfassungsvorrichtung.

In einigen Ausführungsformen kann die Dosiserfassungsvorrichtung mittels der Steuervorrichtung separat angesteuert werden oder zusammen mit dem Abgabedosierer.

In manchen Ausführungsformen wird der Nasenapplikator mittels der Steuervorrichtung angesteuert durch einen dezentral gespeicherten Algorithmus, z. B. webbasiert, über einen Stationsrechner oder Smart Devices (*Non-Embedded Software*).

In einigen Ausführungsformen steht die Steuervorrichtung mit weiteren Vorrichtungen kabellos oder kabelgebunden in Signalkommunikation, beispielsweise um mit Peripheriegeräten interagieren zu können, entsprechende Sende- und/oder Empfangsvorrichtungen können vorgesehen sein. Sie können mit der Steuervorrichtung in Signalverbindung stehen. Zu den Peripheriegeräten können u. a. zählen:
- Vorrichtungen zur Patientenauthentifizierung,
- Armbänder,
- Sensoranordnungen,
- Docking Stations,
- Rechner (PC),
- Smartgeräte,
- Drucker,
- andere medizinische Geräte,
- Vorrichtungen in einem Rechenzentrum,
- Hub, Router, Accesspoint,
- Server,
- (Cloud)Server.

Das erfindungsgemäße System kann eines oder mehrere der vorstehend genannten und/oder weiterer Peripheriegeräte aufweisen.

In einigen Ausführungsformen fungieren Peripheriegeräte des Nasenapplikators als Funkzellen für ein Ortungssystem bzw. zur Generierung eines Geofence.

Ein Geofence ist ein virtueller Zaun bzw. eine virtuelle Grenze, die einen physischen Standort "umgibt". Wie ein realer Zaun trennt ein Geofence den jeweiligen Standort von der Umgebung ab - wenn auch in digitaler Art und Weise anstatt in Form einer physischen Barriere. Im Gegensatz zu einem echten Zaun kann ein Geofence optional allerdings auch Bewegungen innerhalb der "Einzäunung" erkennen.

Ein solcher virtueller Zaun kann eine beliebige Größe, Form oder Peripheriegeräte Koppelung haben. Sogar eine gerade Linie zwischen zwei Punkten kann möglich und vorgesehen sein.

Geofences werden z. B. mit Hilfe einer Kartensoftware erstellt und ermöglichen dem Benutzer, den virtuellen Zaun direkt über die gewünschte Fläche zu zeichnen und/oder mittels W-LAN-, RFID-, Bluetooth- Signalen und/oder mit anderen Peripheriegeräten eine Koppelung usw. zu erstellen. Die digitale Grenze besteht z. B. aus einer Anzahl von Koordinaten für Breite und Länge - und bei einem kreisförmigen Geofence lediglich aus einem Punkt, der das Zentrum bildet mittels W-LAN-, RFID-, Bluetooth- Signalen und/oder mit anderen Peripheriegeräten eine Koppelung usw.

Geofences lassen sich dazu verwenden, Objekte innerhalb des virtuellen Zaunes z.B. per mittels W-LAN-, RFID-, Bluetooth-Signalen und/oder andere Peripheriegeräte Koppelung etc.-Tracking zu überwachen und/oder bestimmte Aktion für einen Bereich definieren und/oder zu aktivieren. Auf diese Weise erkennt man zum Beispiel sofort, wenn ein Gerät wie die erfindungsgemäße Medikamentenabgabevorrichtung, insbesondere der Nasenapplikator, von seinem Einsatzort entfernt wird. Umgekehrt können virtuelle Zäune auch dazu eingesetzt werden, Objekte aus einem bestimmten Bereich herauszuhalten.

In manchen Ausführungsformen kann die Elektronik der Steuervorrichtung redundant ausgelegt werden, z. B. zweikanalig, per Überwachungssystem/Watchdog, etc. Dies kann insbesondere vorteilhaft dazu beitragen, eine sichere Funktionsweise zu gewährleisten, damit die Sicherheit zu erhöhen und den verschiedenen Risikoklassen in der Medizintechnik gerecht zu werden.

In manchen Ausführungsformen umfasst die Abrufvorrichtung des Nasenapplikators einen Auslösemechanismus, der seinerseits wenigstens einen der im Folgenden beschriebenen Merkmale umfasst.

So kann der Auslösemechanismus rein oder im Wesentlichen mechanisch arbeiten, z. B. ähnlich wie bei einem Insulin-Pen oder einem Respimat^{®} Soft-Mist-Inhaler.

Alternativ kann der Auslösemechanismus eine Kombination aus Mechanik (Auslösemechanismus) z.B. Herzkurve, Stellvorrichtung und/oder Übersprung und Elektronik (Ansteuerung) sein.

In einigen Ausführungsformen kann der Auslösemechanismus einen unidirektionalen, bidirektionalen, multidirektionalen, rotations-, schritt- und/oder encodergesteuerten und/oder stufenlosen Kolben und/oder eine Stellvorrichtung, etc. sein oder umfassen.

Ergänzend hierzu kann in manchen Ausführungsformen ein gegenläufiger Dosiereinstellmechanismus (vgl. Insulin Pen) vorgesehen sein. Alternativ ist auch eine elektronische Dosierung (vgl. elektronischer Insulin Pen) von der vorliegenden Erfindung umfasst.

In manchen Ausführungsformen umfasst der Auslösemechanismus eine Pumpe, z. B. eine Mikropumpe, Kolbenpumpe, Rotationspumpe, Peristaltikpumpe, Kreiselpumpe, Fliehkraftpumpe oder dergleichen.

In einigen Ausführungsformen basiert der Auslösemechanismus auf einem bidirektionalen Zweitakt-Prinzip (ansaugen/drücken), z. B. gemäß des Funktionsprinzips des Respimat^{®} 's.

In manchen Ausführungsformen kann der Auslösemechanismus eine Mikrodosierung veranlassen, z. B. im Bereich von 1 µl bis 300 µl.

In einigen Ausführungsformen kann eine abgegebene Menge (Applikationsdosis) leicht und/oder genau reproduziert werden, da die Menge bzw. der Umfang der Applikationsdosis mittels der vorliegenden Erfindung unabhängig von einer Betätigung durch den Benutzer/Patienten sind.

Eine hohe Reproduzierbarkeit und Genauigkeit der abgegebenen Menge durch eine user-unabhängige Auslösung mit stets gleichen Auslöseparametern, z. B. Kraft, Geschwindigkeit, Hub/Weg, genereller Energieeintrag kann vorteilhaft zur Patientensicherheit beitragen.

In manchen Ausführungsformen kann der Auslösemechanismus eine Auslösung durch einen mechanischen oder elektronischen Impulsgeber veranlassen. In diesen Ausführungsformen ist keine Krafteinbringung in das System nötig.

In einigen Ausführungsformen ist die Applikationsdosis der Substanz einstellbar, insbesondere variabel, insbesondere mittels Ausgestaltungen des Auslösemechanismus, wie hierin beschrieben.

In einigen Ausführungsformen umfasst der Auslösemechanismus ein Dosierventil, welches beispielsweise solange offen bleibt, wie es gedrückt gehalten wird. Auch ein Dosierventil mit Dosierkammer, ein zeitgesteuertes Ventil, oder ein durchflussgesteuertes Ventil, etc., ist von der vorliegenden Erfindung umfasst.

In manchen Ausführungsformen umfasst der Auslösemechanismus ferner eine reversible Pumprichtung (Ansaugen). Dies kann insbesondere z.B. für die Entsorgung und/oder Neutralisierung der Substanz bzw. des Wirkstoffs bei Missbrauch und/oder Diebstahl vorgesehen sein.

In einigen Ausführungsformen hat das Gehäuse des Nasenapplikators die Funktion einer Abrufvorrichtung zum Betätigen des Auslösemechanismus.

In manchen Ausführungsformen ist der Nasenapplikator für die Bedienung durch Rechtshänder geeignet und/oder angepasst, in anderen für die Bedienung durch Linkshänder. In bestimmten Ausführungsformen ist er sowohl für die Bedienung durch Links- als auch für die Bedienung durch Rechtshänder geeignet und/oder angepasst.

In einigen Ausführungsformen sind die Auslösung des Auslösungsmechanismus und/oder die Höhe der dadurch bedingten Applikationsdosis unabhängig von einer Betätigung durch den Benutzer/Patienten.

In manchen Ausführungsformen des Nasenapplikators ist vorgesehen, dass der Benutzer/Patient die Höhe einer abzugebenden Applikationsdosis selbst einstellt. Dies kann beispielsweise basierend auf Berechnungen oder Anweisungen eines Arztes erfolgen. Diese können auf unterschiedliche Art und Weise dem Benutzer/Patienten mitgeteilt werden, beispielsweise über ein Smartphone oder dergleichen. In bestimmten Ausführungsformen kann zu diesem Zweck eine Software, insbesondere eine Applikation (App), beispielsweise für ein Smartphone, von der vorliegenden Erfindung umfasst sein.

In einigen Ausführungsformen umfasst die Abrufvorrichtung zum Aktivieren des Auslösemechanismus wenigstens eine der folgenden Einrichtungen oder ist als solche ausgestaltet:
- einen integrierten Auslöseknopf, beispielsweise ausgestaltet als Softtouch-Knopf, als Druckknopf, als Schalter, als Bereich (Button) auf einem Touchscreen, als Sensor oder dergleichen;
- eine integrierte Eingabevorrichtung, beispielsweise eine Tastatur, ein Touchscreen, ein Drehknopf (oder mehrere), ein integrierter Sensor oder eine Kamera zum Erfassen von biometrischen Daten, beispielsweise Fingerabdruck, Gesicht oder Sprache, eines Codes, oder dergleichen;
- eine externe Eingabevorrichtung, die, vorzugsweise drahtlos, mit dem Nasenapplikator, insbesondere dessen Abrufvorrichtung verbunden ist, beispielsweise eine Applikation (App), eine Fernbedienung, Eingabe mittels Web oder Cloud, Rechner, Smart Device oder dergleichen.

In bestimmten Ausführungsformen kann die externe Eingabevorrichtung ausgestaltet sein analog zu den hierin genannten Beispielen der Ausführung der integrierten Eingabevorrichtung.

In manchen Ausführungsformen kann ein bestimmtes Verhalten des Benutzers/Patienten die Abrufvorrichtung steuern bzw. beeinflussen. Beispielweise kann ein zweimaliges Drücken für eine Auslösung mit dem ersten Drücken ein "Wecken" des Nasenapplikators ggf. verbunden mit einer Systemüberprüfung, einer Überprüfung der Berechtigung etc., einer Berechnung der Applikationsdosis und Bereitstellen derselben bewirken, während das zweite Drücken schließlich ein Auslösen der Applikationsdosis bewirkt.

In einigen Ausführungsformen kann der Nasenapplikator, insbesondere mittels einer seiner Vorrichtungen, eine Ausgabeinformation erzeugen, vorzugsweise automatisch, vorzugsweise nach einer Substanzdosisabgabe. Diese Ausgabeinformation kann eine Log-Datei der Behandlung sein oder umfassen. Dabei kann jedes erfassbare Signal bzw. jede erfassbare Interaktion, insbesondere jede Betätigung, geloggt werden. Alternativ oder ergänzend kann die Ausgabeinformation das Erstellen eines standardisierten und/oder auf den Benutzer/Patienten bezogenen personalisierten Berichts sein oder umfassen. Die Ausgabeinformation kann mittels eines digitalen (online) Dashboards, mittels Druckfunktionen (z. B. für das Betäubungsmittelbuch (BtM)), mittels Exports, etc. an eine hierzu jeweils geeignete Vorrichtung erfolgen, vorzugsweise drahtlos, in manchen Ausführungsformen auch kabelgebunden und/oder mit Kontaktpins.

In einigen Ausführungsformen kann der Abgabedosierer Auslösemechanismus auf andere Art und Weise aktiviert werden, beispielsweise *breath-activated* oder *nose-activated,* d. h. der Auslösemechanismus wird beispielsweise automatisch mittels eines Unterdrucksensors aktiviert, wenn der Nasenapplikator in die Nase eingeführt ist und durch die Nase eingeatmet wird. Auch eine mechanische Umsetzung dieses Auslösemechanismus ist von der vorliegenden Erfindung umfasst.

In manchen Ausführungsformen ist eine Optik in den Nasenapplikator integriert, beispielsweise zum Erfassen von biometrischen Daten, wie Fingerabdruck oder Gesicht, zum Erfassen eines QR-Codes, eines Barcodes oder dergleichen.

In einigen Ausführungsformen können die Ausgabeinformationen eine Angabe der Zeitdauer (Countdown/Timer) bis zur nächsten Freigabe sein oder umfassen. Die Anzeige bzw. der Ablauf dieser Zeitdauer kann beispielsweise optisch (z. B. mittels einer farbigen Anzeige, einer LED oder Push-Nachricht), akustisch (z. B. mittels Alarms), taktil (z.B. mittels Vibration) oder auf analoge Art und Weise erfolgen. Eine farbige Anzeige kann hierbei beispielsweise umfassen, dass der Nasenapplikator oder ein Abschnitt hiervon die Farbe ändert, um seinen Status zu signalisieren, beispielsweise auf "grün" wechselt, wenn eine neue Applikation erfolgen darf.

In manchen Ausführungsformen können die Ausgabeinformationen eine Ausgabe einer Schritt-für-Schritt Anleitung (z. B. für ein Setup oder eine weiterführende Bedienung oder dergleichen) mittels eines visuellen und/oder akustischen Mediums, beispielsweise eines Smartphones oder dergleichen, sein oder umfassen.

In manchen Ausführungsformen sind Schritt-für-Schritt Anleitungen auf dem Nasenapplikator und/oder auf entsprechenden Peripheriegeräten und/oder auf Smart Devices vorgesehen.

In einigen Ausführungsformen der vorliegenden Erfindung können Eingabe- und/oder Ausgabemöglichkeiten jeweils barrierefrei vorgesehen sein. Hierunter fallen z. B. Ein- und/oder Ausgaben in Braille, optische, akustische oder taktile Ein- und Ausgaben, z. B. Vibration, Schall etc. Dies ist insbesondere auch vorteilhaft für den Einsatz der vorliegenden Erfindung bei schlechten Sichtbedingungen und/oder bei Nacht. Diese Eingabe- und/oder Ausgabemöglichkeiten können insbesondere kontaktlose Eingabe- und/oder Ausgabemöglichkeiten sein oder umfassen.

In einigen Ausführungsformen umfasst der Nasenapplikator einen QR-Codeleser. Dieser kann zum Scannen der UDI (*"Unique Device Identification")* der Einwegkartusche, zum Erkennen des Einweggehäuses, der Applikatorelektronik, eines Einwegarmbands und/oder dessen Armbandelektronik, und/oder zum automatisierten Auslesen in einer *Docking Station* dienen.

In einigen Ausführungsformen ist von der vorliegenden Erfindung auch eine Druckerfunktionalität umfasst. Diese kann u. a. dazu dienen, einen Bericht/Report zu erstellen, Aufkleber zu fertigen (z. B. für das BtM-Buch und/oder die Patientenakte), usw.

In manchen Ausführungsformen der vorliegenden Erfindung ist ein Datenzugang direkt mittels eines eigenen Eingabe- und Ablesedisplays vorgesehen. Alternativ oder ergänzend kann ein Datenzugang indirekt mittels einer "digitalen online Verwaltungssoftware" beispielsweise mittels eines Smartphones, Tablets und/oder Rechners und/oder mittels anderer Schnittstellen vorgesehen sein.

In manchen Ausführungsformen umfasst der Nasenapplikator und/oder wenigstens eine seiner Vorrichtungen berührungsempfindliche (*touch-sensitive*) Oberflächen.

In einigen Ausführungsformen kann die vorliegende Erfindung ein in den Nasenapplikator integriertes Mikrofon und/oder ein Smartphone-Mikrofon umfassen. Dieses Mikrofon kann dazu dienen, um z. B. ein Schmerztagebuch aufzuzeichnen.

In bestimmten Ausführungsformen kann die vorliegende Erfindung eine *"shake to wake"* Funktion umfassen. Hierzu geeignete Vorrichtungen, beispielsweise ein Beschleunigungssensor, ein Gyroskop, ein Winkelsensor, ein Lagesensor und/oder dergleichen sind ebenfalls von der vorliegenden Erfindung umfasst.

In manchen Ausführungsformen kann die vorliegende Erfindung eine in den Nasenapplikator integrierte Kamera und/oder eine Smartphone-Kamera umfassen. Diese Kamera kann dazu dienen, um beispielsweise in die Nase, Ohren, usw. schauen zu können nach Übertragung an einen Arzt eine Diagnose stellen zu lassen, und dergleichen (*Digital Health Diagnostics*). Sie kann ferner dazu dienen, das Maß einer Sedierung zu erkennen, beispielsweise mittels Aufnahmen einer Pupillenreaktion des Auges.

Patienten-Authentifizierung bedeutet, dass nur der berechtigte Benutzer/Patient das Gerät bedienen kann. Ein Benutzer kann u. a. beispielsweise ein Arzt, eine Pflegekraft und/oder ein anderer Berechtigter sein.

In einigen Ausführungsformen weist der Nasenapplikator hierzu ein integriertes Authentifizierungsmodul auf, in anderen Ausführungsformen ist das Authentifizierungsmodul extern vorgesehen, in bestimmten Ausführungsformen teilintegriert. Hierbei ist der dem Authentifizierungsmodul zugrundeliegende Authentifizierungsmechanismus vorzugweise mit genau einem einzigen, konkreten Gerät gekoppelt. Hierzu erforderliche Signalübertragungen können z. B. wie hierin offenbart vorgesehen sein und erfolgen. Entsprechende Ausgestaltungen der beteiligten Signalübertragungspartner können vorgesehen sein.

In manchen Ausführungsformen kann ein integriertes, also am oder im Nasenapplikator vorgesehenes, Authentifizierungsmodul optisch wirken, z. B. mittels QR-Codeleser oder mittels Vorrichtungen zum Erkennen und Vergleichen biometrischer Daten, in anderen Ausführungsformen kann es akustisch wirken, z. B. mittels Stimmerkennung, in weiteren Ausführungsformen taktil, z. B. mittels der Eingabe einer Geheimzahl oder eines Passworts. Das Authentifizierungsmodul umfasst hierbei die jeweils zur Authentifizierung geeignete Sensoranordnung und eine Auswertevorrichtung.

In einigen Ausführungsformen kann das Authentifizierungsmodul vorgesehen sein, um mit weiteren Einrichtungen, Vorrichtungen, Geräten kompatibel zu sein.

Es kann in bestimmten Ausführungsformen vorgesehen sein, eine Authentifizierung mittels Vibration (beispielsweise mittels eines bestimmten Vibrationsmusters) oder mittels Sprach- oder Stimmerkennung durchzuführen. In weiteren Ausführungsformen kann über den GPS Standort des Nasenapplikators und/oder mittels einer Geofencing-Funktion ein Bereich festgelegt werden, in welchem das Gerät freigeschaltet ist.

In manchen Ausführungsformen kann ein teilintegriertes oder externes Authentifizierungsmodul am Patienten vorgesehen sein, in andern Ausführungsform ist es nicht am Patienten vorgesehen. Bei teilintegrierten oder externen Authentifizierungsmodulen kann der Authentifizierungsmechanismus beispielsweise auf Token, drahtloser Authentifizierung basieren. Das Authentifizierungsmodul kann eine Armbanduhr, ein RFID-Armband, ein Barcode, eine Kette, ein Ring, ein Smartphone, eine Smartwatch, ein Tablet, oder ergänzend eine Software für die vorgenannten Module (z. B. eine App), etc. sein oder umfassen. Das Authentifizierungsmodul umfasst hierbei die jeweils zur Authentifizierung geeignete Sensoranordnung und/oder Auswertungsvorrichtung, analog wie oben beschrieben.

In einigen Ausführungsformen ist das Authentifizierungsmodul mit Standard Krankenhaus-Patientenidentifikationshilfsmitteln kompatibel oder in diese integrierbar (z. B. in ein Patientenarmband) .

In manchen Ausführungsformen kann es notwendig sein, mittels des Authentifizierungsmoduls eine Authentifizierung eines Administrators und/oder einer Benutzergruppe vorzunehmen, um den Nasenapplikator in Betrieb zu nehmen bzw. das Auslösen einer Applikationsdosis zu veranlassen bzw. Anpassungen am Nasenapplikator vorzunehmen.

In manchen Ausführungsformen ist das Authentifizierungsmodul vorgesehen, um ergänzend Techniker und/oder Systemadministratoren zu authentifizieren und ihnen Zugang zur Software des Nasenapplikators, insbesondere dessen Steuervorrichtung, zu gewähren.

In einigen Ausführungsformen kann es notwendig sein, mittels des Authentifizierungsmoduls die Authentifizierung eines Administrators und/oder einer Benutzergruppe vorzunehmen, um die Steuerungsvorrichtung des Nasenapplikators per Fernbedienung oder Fernsteuerung (*remote*) zu bedienen und/oder Daten auf ihr zu überschreiben (*override-Funktion*).

Dies kann z. B. notwendig sein, um eine extra Medikamentenabgabe zu veranlassen. Die Authentifizierung kann hierbei mittels einer der hierin genannten Vorrichtungen und/oder Wirkweisen vorgenommen werden.

In manchen Ausführungsformen ist die Reichweite des Authentifizierungsmoduls variabel und/oder einstellbar, vorzugsweise stufenlos.

In einigen Ausführungsformen kann das Authentifizierungsmodul in sogenannte smarte Textilien integriert sein, beispielsweise in eine Brille, in Schuhe, in einen Patientenkittel, in ein Kissen und/oder in einen Kissenbezug und/oder in einen Bettbezug und/oder Matratze, in einen Arbeitskittel oder dergleichen.

In manchen Ausführungsformen ist das Authentifizierungsmodul eine Vorrichtung, die im Ohr getragen wird (*In-Ear Wearable*) oder dergleichen.

In einigen Ausführungsformen ist oder umfasst das Authentifizierungsmodul ein subkutanes Implantat zur Authentifizierung und eine zum Auslesen desselben geeignete Sensoranordnung.

In manchen Ausführungsformen ist oder umfasst das Authentifizierungsmodul eine Zahnkrone oder Zahnschiene zur Authentifizierung und eine zum Auslesen derselben geeignete Sensoranordnung.

In einigen Ausführungsformen ist oder umfasst das Authentifizierungsmodul eine in den Nasenapplikator integrierte Kamera und/oder eine Smartphone-Kamera, um Daten, beispielsweise biometrische Daten, zu erfassen, alternativ oder ergänzend ein Mikrofon, integriert oder extern, zum Zweck der Stimmerkennung.

In einigen Ausführungsformen umfasst der Nasenapplikator ein Monitoring-/Überwachungssystem, wobei Monitoring die Beobachtung des Patienten und Überwachen zusätzlich noch die Beobachtung von dessen Umgebung ist oder umfasst. Mittels des Systems kann beispielsweise die sogenannte Therapietreue, d. h. in welchem Ausmaß das Verhalten des Patienten mit den vereinbarten Empfehlungen/Verschreibungen eines Mediziners übereinstimmt (*Compliance*/*Adherence*), überwacht werden.

In manchen Ausführungsformen umfasst das Monitoring-/Überwachungssystem eine automatisierte Medikamentenüberwachung in Echtzeit (*realtime*). Diese kann z. B. eine verbrauchte Menge, eine Restmenge, die Anzahl verbleibende Applikationen etc. erfassen und mittels einer hierzu geeigneten Speichervorrichtung intern oder extern speichern.

In einigen Ausführungsformen umfasst das Monitoring-/Überwachungssystem eine automatisierte Therapieüberwachung in Echtzeit (*realtime*). Diese kann z. B. Applikationszeiten und Applikationsmengen, versuchte (vergebliche) Applikationen während eines Sperrintervalls etc. erfassen und mittels einer hierzu geeigneten Speichervorrichtung intern oder extern speichern.

In manchen Ausführungsformen umfasst das Monitoring-/Überwachungssystem eine automatisierte Therapietagebuchführung in Echtzeit (*realtime*). Diese kann z. B. patientenindividuell Nebenwirkungen, Gemütszustände und/oder die Stärke, Dauer und/oder Häufigkeit von Beschwerden etc. erfassen und mittels einer hierzu geeigneten Speichervorrichtung intern oder extern speichern.

In einigen Ausführungsformen kann das Monitoring-/Überwachungssystem dazu vorgesehen sein, ein Mobilitätsprotokoll zu erstellen.

In einigen Ausführungsformen umfasst das Monitoring-/Überwachungssystem des Nasenapplikators eine Kombination aus den vorgenannten Echtzeit-Überwachungen.

Eine Echtzeit-Überwachung mittels des Monitoring- und/oder Überwachungssystem des Nasenapplikators ermöglicht vorteilhafterweise eine einfache und/oder genaue Protokollierung der Therapie

In manchen Ausführungsformen umfasst die Steuervorrichtung des Nasenapplikators integrierte, optische und/oder akustische Status- und/oder Alarmanzeigen, beispielsweise LED(s), Buzzer, Beep(s)und/oder ein Display, um den Status oder den Alarm, insbesondere mittels eines Error Code oder dergleichen, anzuzeigen.

In manchen Ausführungsformen umfasst die Steuervorrichtung des Nasenapplikators externe, optische und/oder akustische Status- und/oder Alarmanzeigen, beispielsweise in oder auf Peripheriegeräten, Smart Devices und/oder Stationsrechnern. Die externen Status- und/oder Alarmanzeigen können ebenfalls LED(s), Buzzer, Beep(s)und/oder ein Display umfassen, ferner können sie als Software Dashboard (App), Push-Nachricht oder dergleichen vorgesehen sein.

In manchen Ausführungsformen ist der Nasenapplikator konfiguriert, um automatische Meldungen, insbesondere einen Hilferuf und/oder einen Alarm an Dritte, z. B. an bestimmte Personengruppen, Ärzte, Pflegepersonal, Rettungsleitstelle oder dergleichen, zu senden. Dies kann insbesondere bei ungewöhnlichem Verhalten, beispielsweise ständigen Medikamentenabrufen, Überschreiten der maximal erlaubten Medikamentenmenge (*Dosing Limit*) oder dergleichen, und/oder wenn die Vitalwerte des Benutzers/Patienten außerhalb vorbestimmter Grenzen liegen. Alternativ oder ergänzend kann eine solche Meldung mittels aktiven Betätigens eines "Notfallknopfs" durch den Benutzer/Patienten vorgesehen sein.

In einigen Ausführungsformen ist ein "sicherer" Zustand des Nasenapplikators vorgesehen. Dieser ist insbesondere im Zustand "aus" gegeben. Im "sicheren" Zustand gibt das System egal unter welchen Umständen keine Dosis ab.

In manchen Ausführungsformen umfasst der Nasenapplikator eine Patientenüberwachungssensorik. Die Sensorik ist geeignet, patientenindividuelle Daten zu erfassen. Diese Daten können objektive Daten wie Position, Bewegung und/oder Vitalwerte (z.B. HR, HRV, SpO₂, Blutdruck, elektrochemische Hautreaktion, Temperatur, Photoplethysmogram (PPG), Electrocardiogram (ECG), Blood pressure trending (BPT), Respiratory rate, Stress, etc.) oder subjektive Daten, erfasst mittels Patientenfeedback wie hierin beschrieben, sein oder umfassen.

Derartige Überwachungssysteme können die Therapietreue und somit den therapeutischer Effekt (z. B. durch Reminder, unterstützende Informationen, Wertcoupons etc., Statusabfrage, Vorschläge, Aktivitätstracker, Benachrichtigung von Angehörigen/Bekannten, etc.) vorteilhafterweise erhöhen oder optimalisieren.

In einigen Ausführungsformen kann vorgesehen sein, den Nasenapplikator mit weiteren Funktionen, wie beispielsweise Remindern, weitergehender unterstützender Information, Gamification (z. B. Punkte sammeln, TV-Minuten sammeln, Wertcoupons etc.), Statusabfragen, Vorschlägen, Aktivitätstracker, Benachrichtigung von Angehörigen/Bekannten, etc. zu ergänzen.

In manchen Ausführungsformen kann der Nasenapplikator vorgesehen sein, sogenanntes *social monitoring and communication* zu ermöglichen, d. h. ein, vorzugsweise bidirektionales, Kommunikationsmittel zu Familie, Freunden und Pflegekräften usw. bereitzustellen.

In einigen Ausführungsformen kann der Nasenapplikator zusätzlich zur medizinischen Substanz in einem weiteren Substanzreservoir ein Gegenmittel (Antidot) aufweisen, geeignet um z. B. Nebenwirkungen zu verringern bzw. zu verhindern oder eine Überdosierung zu vermeiden oder dergleichen.

In manchen Ausführungsformen ist der Nasenapplikator konfiguriert, um eine Atemgasanalyse, z. B. eine Kapnographie, durchzuführen und das Ergebnis der Analyse beispielsweise anzuzeigen, an ein Peripheriegerät zu übermitteln und/oder in einer hierzu geeigneten Speichervorrichtung zu speichern.

In einigen Ausführungsformen kann der Nasenapplikator mit Sensoranordnungen und/oder Auswerteeinrichtungen verbunden sein, mittels derer die Position des Benutzers/Patienten erfasst und gespeichert werden können. Die Position kann die Lagerung des Patienten erfassen, ob er/sie aufsteht, gestürzt ist und/oder dergleichen. Diese Sensoranordnungen können intern oder extern vorgesehen sein, vorzugsweise wie hierin ausgeführt, beispielsweise integriert in *smarte* Textilien mit integrierten Sensoren. Der Nasenapplikator kann zu diesem Zweck weiter geeignete Aktuatoren usw. aufweisen. Auf diese Art und Weise kann beispielsweise ein Mobilitätsprotokoll etc. erstellt werden.

In manchen Ausführungsformen kann die Patientenüberwachungssensorik oder Teile hiervon implantiert sein. In anderen Ausführungsformen kann sie oder Teile hiervon in eine Zahnkrone oder -schiene oder in einen Ohrring oder Ohrclip integriert sein.

In einigen Ausführungsformen kann die Patientenüberwachungssensorik oder Teile hiervon, Befindlichkeiten bzw. Zustände des Benutzers/Patienten, wie z.B. Schmerz, detektieren bzw. messbar machen. Dies kann über Körperreaktionen wie beispielsweise Hautgalvanik, Blutdruckänderung, Herzrateänderung und/oder Pupillendilatation ermittelt werden.

In manchen Ausführungsformen umfasst der Nasenapplikator ein Nasenapplikator-Ortungssystem. Dabei kann das Nasenapplikator-Ortungssystem konfiguriert sein, um den Nasenapplikator zu orten, die Substanz zu orten und/oder den Benutzer/Patienten zu orten. Dabei kann ein integriertes Echtzeit-(*Realtime*)-Ortungssystem z. B. ein Flottenmanagement oder dergleichen mittels drahtloser Konnektivität (z.B. via Wi-Fi, Bluetooth, WLAN, GPS, RFID, NFC, Barcode, QR-Code, ZigBee, Wibree, WiMAX und/oder IrDA etc.) zum Einsatz kommen. Das Nasenapplikator-Ortungssystem dient vorteilhafterweise dem Zweck, die Geräteverwaltung zu erleichtern und die Patientensicherheit zu erhöhen.

In manchen Ausführungsformen kann die Einrichtung virtueller Zonen bzw. Bereiche die Positionsbestimmung vereinfachen.

In einigen Ausführungsformen umfasst der Nasenapplikator ein Anti-Missbrauchsystem und/oder ein Anti-Diebstahlsystem.

In manchen Ausführungsformen ist das Gehäuse des Nasenapplikators unzugänglich ausgestaltet, derart, dass ein Zugang zu der oder jeder Substanz verhindert wird. Dies kann beispielsweise mittels eines Einweggehäuses realisiert werden, das beim Öffnen zerstört würde.

In einigen Ausführungsformen umfasst der Nasenapplikator eine Vorrichtung zu seinem physischen Fixieren. Dies kann beispielsweise mittels eines Kensington^{®} Schlosses, eines Sicherungskabels bzw. -Seils, Kabelschlosses, etc. ausgestaltet sein.

In manchen Ausführungsformen ist der Nasenapplikator mit einem vorbestimmten Geofence vorgesehen. In diesen Ausführungsformen ist von der vorliegenden Erfindung die Möglichkeit umfasst, die Funktionalität des Nasenapplikators außerhalb eines vordefinierten Bereichs zu beeinflussen, beispielsweise indem ein Auslösen verhindert oder der Wirkstoff der Substanz neutralisiert wird. Dies kann mittels einer Softwarelösung und/oder drahtloser Konnektivität realisiert werden. Wie hierin bereits ausgeführt, können Peripheriegeräte hierbei als Funkzelle dienen.

In einigen Ausführungsformen kann der Nasenapplikator kindersicher ausgestaltet sein. Die Kindersicherung kann beispielsweise mittels einer Hardware (z. B. Abdeckung, Verschluss und/oder Safe) und/oder mittels einer Software (z. B. Patienten Authentifizierung etc.) realisiert sein.

In manchen Ausführungsformen können Peripheriegeräte des Nasenapplikators mit Sicherheitsverschluss und/oder Manipulationsschutz vorgesehen sein, z. B. ein Armband mit eingelegten Stahldraht, Kevlar, etc.), insbesondere können Peripheriegeräte stufenlos verstellbar und somit individuell auf den Benutzer/Patienten einstellbar sein.

In einigen Ausführungsformen kann eine Echtzeit-Ortung (Realtime), insbesondere ein Geofencing, zum Flottenmanagement und/oder Sperren/Entsperren des Nasenapplikators und/oder zum Auslösen eines Neutralisierens des Wirkstoffes in der Substanz dienen.

In manchen Ausführungsformen kann der Wirkstoff in der Substanz beispielsweise durch ein flüssiges und/oder festes und/oder gasförmiges Additiv, wie z. B. eine Base, Säure, Antagonist, Gips, Zement und/oder Kälte (N₂O =̂ -89° C oder CO₂ =̂ -78,5° C) etc. neutralisiert bzw. zerstört werden.

In einigen Ausführungsformen kann der Nasenapplikator konfiguriert sein, um beispielsweise zwischen der Behandlung zweier unterschiedlicher Patienten wieder aufbereitet zu werden. Ein Wiederaufbereiten kann z. B. ein Programmieren oder Umprogrammieren, ein (neu) Laden oder ein Austauschen von Batterien oder anderen Energiespeichervorrichtungen wie hierin beschrieben, ein (erneutes) Befüllen des oder der Substanzreservoirs und/oder das Einlegen einer neuen Kartusche sein oder umfassen. Ferner kann das Wiederaufbereiten ein Überprüfen, beispielsweise in Form eines Systemchecks, ein ganz oder teilweises Entsorgen von Nasenapplikator und/oder Substanz, Hygienemaßnahmen, wie Putzen und/oder Desinfizieren, und/oder eine Wartung, welche ergänzend ein Systemupdate beinhalten kann, sein oder umfassen. Die Steuervorrichtung kann optional programmiert sein, um Schritte zum Wiederaufbereiten, wie z. B. das Löschen von Patientendaten automatisch oder auf Aufforderung zu löschen, etwa nach Auswählen eines entsprechenden Menüpunkts im Steuerungsmenü der Speichervorrichtung.

In manchen Ausführungsformen ist der Nasenapplikator ein vollständiges Einwegprodukt, welches komplett nach geltenden Regularien nach erstmaliger Verwendung am Patienten entsorgt werden muss. Dabei müssen unter Umständen der Nasenapplikator, die Substanz(en) und/oder ein eventuelles Peripheriegerät einzeln entsorgt werden.

In einigen Ausführungsformen ist der Nasenapplikator teilweise ein Einwegprodukt und weist beispielsweise eine wiederverwendbare Elektronik, Einwegkomponenten, welche mit der Substanz und/oder dem Benutzer/Patienten in Kontakt gekommen sind (z. B. ein Einweggehäuse für die Elektronik, ein Einwegnasenaufsatz, eine Einwegpumpe oder dergleichen.

In manchen Ausführungsformen kann der Nasenapplikator konfiguriert sein, um automatisiert und/oder protokolliert eine Entsorgung der Restmenge der Substanz zu umfassen. Dies kann beispielsweise durch Abpumpen in Sondermüll oder in einen Spezialbehälter bzw. ein separates Behältnis für die Medikamentenentsorgung, Ansaugen und Neutralisieren, etc. erfolgen.

In einigen Ausführungsformen umfasst der Nasenapplikator eine modulare, vorzugsweise erweiterbare *Docking Station* für die elektronische Steuervorrichtung und/oder weitere Peripheriegeräte. Die Docking Station kann beispielsweise zum Laden der Energiespeichervorrichtung, zum Übertragen von Softwareupdates auf den Nasenapplikator und/oder die Peripheriegeräte, zum Pairing von zwei beliebigen vom Nasenapplikator umfassten Vorrichtungen, zur Hardwareüberprüfung, zur Implementierung von Freigaben auf den Nasenapplikator/das Peripheriegerät usw. konfiguriert sein.

In manchen Ausführungsformen ist der Nasenapplikator konfiguriert zur automatischen (Nach-)Bestellung der Substanz(en).

In einigen Ausführungsformen enthält die Schutzkappe des Nasenapplikators antibakterielle Mittel, um den Nasenaufsatz sauber zu halten.

In manchen Ausführungsformen umfasst der Nasenapplikator eine Spülvorrichtung für den Nasenaufsatz, da sonst das Substanzvolumen im Nasenaufsatz bis zur nächsten Auslösung "stehen" bleiben würde, was eine Besiedelung mit Mikroorganismen begünstigen könnte. Dies kann vorzugsweise bei sensiblen Wirkstoffen in der Substanz vorgesehen sein.

In einigen Ausführungsformen kann ein Primingprozess vorgesehen sein, während dem z. B. die Substanz in das Substanzreservoir zurückgeführt und automatisch aufgefangen wird. Es kann in manchen Ausführungsformen vorgesehen sein, dass der Nasenapplikator eine Auslösung blockiert, wenn der Primingprozess nicht abgeschlossen ist.

In einigen Ausführungsformen kann innerhalb und/oder außerhalb des Nasenapplikators eine Datenauswertung via Software (z. B. Datenexport, Dashboard, Berichte, E-Mails, Notifications, Bildschirm, App etc.) vorgesehen sein.

In manchen Ausführungsformen kann bei der Datenauswertung vorgesehen sein, eine Datenverschlüsselung anzuwenden. Diese kann beispielsweise Verschlüsselungskey, Blockchain, Passwort, Passphrase, Token, Biometrik, und/oder Dongle, etc. sein oder umfassen.

In einigen Ausführungsformen des Nasenapplikators sind weitere Funktionalitäten zuschaltbar, beispielsweise ein Dashboard (Leitstand), von dem zentral die Vorrichtungen des Nasenapplikators angesteuert, überwacht etc. werden können, zur Therapie und/oder weitere Vorrichtungen und oder die Applikation weiterer Substanzen und/oder Funktionen zur Patientenverwaltung und/oder dergleichen.

In manchen Ausführungsformen kann der Nasenapplikator vorgesehen oder programmiert sein, interne und/oder externe Umfragen durchzuführen, z. B. zur Patientenzufriedenheit, zu Nebenwirkungen oder dergleichen. Diese Umfragen können standardisiert sein, beispielsweise gemäß der *International Statistical Classification of Diseases and Related Health Problems* (ICD-10, EU) oder gemäß *Diagnosis Related Groups* (DGRG) und/oder gemäß der *Qualitätsverbesserung in der postoperativen Schmerztherapie* (QUIPS)(Deutschland) und oder gemäß den *Risk Evaluation and Mitigation Strategies* (REMS) Programmen (USA).

In einigen Ausführungsformen umfasst der Nasenapplikator eine QIUPS Schnittstelle (Fragenbogen, Informationsmaterial etc.) zur Ermittlung des akuten und chronischen Schmerzempfindens des Benutzers/Patienten.

In manchen Ausführungsformen umfasst der Nasenapplikator eine Patientenaufklärung (online und/oder offline) zur Schulung und/oder Nachsorge (*aftercare*) des Benutzers/Patienten oder eine Möglichkeit hierzu. Diese kann beispielsweise mittels einer Online-Video-Sprechstunde, mittels eines virtuellen Assistenten, mittels einer Bedienungsanleitung, eines Beipackzettels, einer Information zu Nebenwirkungen, FAQs, Operations und/oder Produktinformationen, Formularen, Umfragen, Therapietagebuch, elektronische Patientenakte etc. erfolgen.

In manchen Ausführungsformen umfasst der Nasenapplikator eine Trainingsfunktion (online und/oder offline) für Ärzte und/oder Pflegepersonal, die z. B. mittels Remindern, neuen Schulungen, Studien, Geräteupdates, neuen Funktionalitäten, Substanzen, etc. erfüllt wird.

In einigen Ausführungsformen können mittels der vorliegenden Erfindung benutzerdefinierte Berichte erstellt werden, um die individuellen Anforderungen von Krankenhäusern oder anderen Einrichtungen und/oder Vorschriften zu erfüllen. Hierzu können beispielsweise die Vorschriften der *Food and Drug Administration* (FDA) in den USA, die Vorschriften des Bundesinstituts für Arzneimittel und Medizinprodukte (BfArM) in Deutschland oder die Vorschriften der Europäische Arzneimittel-Agentur (*European Medicines Agency,* EMA) in Europa etc. gehören.

In manchen Ausführungsformen ist der Nasenapplikator vorgesehen, IoT (*Internet of Things*) zu unterstützen, beispielsweise mittels *Healthcare Tracking, Identification*/*Authentication, Data Collection*/*Data Mining* und/oder *Sensing.* Mittels hierzu geeigneter Kommunikationsvorrichtungen, insbesondere ohne menschliches Zutun, können dann Daten über das Netzwerk übermittelt werden. Hierzu kann optional vorgesehen sein, eines oder mehrere der einzelnen Vorrichtungen und/oder Peripheriegeräte des Nasenapplikators mit eineindeutigen Identifikatoren zu versehen, damit sich die einzelnen Vorrichtungen und Peripheriegeräte in einem Netzwerk erkennen können.

In einigen der Ausführungsformen ist eines der Peripheriegeräte programmiert, um von einem ersten der Nasenapplikatoren erste Daten zu empfangen, diese zu verarbeiten, und um basierend auf dem Ergebnis der Verarbeitung zweite Daten zu erstellen und Letztere an einen zweiten der Nasenapplikatoren und/oder an den ersten Nasenapplikator zu senden. Dabei können zweite Daten z. B. ein Algorithmus sein, oder Ergebnisse, die beim Ablaufen des Algorithmus erzielt wurden.

Insbesondere können auf diese Weise Erfahrungen, die bei der Nutzung des konkreten, ersten Nasenapplikators an einem ersten Benutzer gewonnen wurden, und welche sich in den ersten Daten widerspiegeln können, vorteilhaft genutzt werden, um die spätere Nutzung dieses ersten Nasenapplikators zu verbessern. Hierzu können aufgrund der vom ersten Nasenapplikator empfangenen Daten nach deren Auswertung, Bearbeitung, Verarbeitung usw. mittels eines der Peripheriegeräte zweite Daten erstellt werden und z. B. von diesem Peripheriegerät auf den ersten Nasenapplikator übertragen werden, was von der vorliegenden Erfindung umfasst ist. Zweite Daten können Zuordnungen des konkreten ersten Benutzers zu einer Gruppe von Benutzern sein, also z. B. zu seiner Klassifizierung oder zur Klassifizierung seines Krankheitsbildes oder seines Benutzerverhaltens umfassen. Sie können als Formeln, Algorithmen oder in anderer Form vorliegen und übertragen werden. Sie können sich von einem reinen Up-date, welches ohne Betrachtung des konkreten Benutzers, seines Verhaltens und/oder seiner Charakterisierung als Patient, sondern z. B. als medizinisch unterscheidungsloser Konsument, erfolgt, unterscheiden.

Insbesondere können auf diese Weise Erfahrungen, die bei der Nutzung des konkreten, ersten Nasenapplikators an einem ersten Benutzer gewonnen wurden, und welche sich in den ersten Daten widerspiegeln können, vorteilhaft genutzt werden, um die spätere Nutzung eines zweiten Nasenapplikators zu verbessern. Hierzu können aufgrund der vom ersten Nasenapplikator empfangenen Daten nach deren Auswertung, Bearbeitung, Verarbeitung usw. mittels eines der Peripheriegeräte zweite Daten erstellt werden und z. B. von diesem Peripheriegerät auf einen zweiten Nasenapplikator übertragen werden, was von der vorliegenden Erfindung umfasst ist. Zweite Daten können wie vorstehend ausgeführt sein. Sie können dem Benutzer des zweiten Nasenapplikators dienen, Erfahrungen, welche z. B. oder u. a. durch die Benutzung des ersten Nasenapplikators durch den ersten Benutzer gewonnen wurden, für seine künftige Nutzung seines zweiten Nasenapplikators vorteilhaft für sich zu verwenden.

In manchen Ausführungsformen ist vorgesehen, dass der Einsatz eines Nasenapplikators zunächst mit einem "Basis-Algorithmus" startet.

Der anfängliche Algorithmus, hier auch "Basis-Algorithmus" genannt, startet optional mit einem voreingestellten Modell und Parameter-Set für die entsprechende Indikation, welche anhand z. B. des Alters, Ko-medikation, signifikanten Vorerkrankungen usw. initial angepasst werden kann. Nach dem Start der Behandlung oder Benutzung wird der "Basis-Algorithmus" (Modell und/oder Parameter) anhand der applizierten Dosen und/oder der daraus resultierenden Konzentrationen und vorzugsweise mithilfe des Abgleichs zwischen theoretisch errechneten nächsten Applikationszeitpunkten und den tatsächlichen nächsten Applikationszeitpunkten, des Betätigungsverhaltens des Benutzers und/oder des Inputs von Vitalwerten trainiert, um den Patienten zu charakterisieren. Hierauf basierend kann somit die Benutzung oder Behandlung dieses Patienten bei weiterer Verwendung seines Nasenapplikators optimiert werden.

Hierzu können Verfahren, Methoden und Technologien der Künstlichen Intelligenz und/oder des Machine Learnings (oder der Sonderfall des Machine Learnings, das Deep Learning) angewandt werden, um den "Basis-Algorithmus" (Modell und/oder Parameter) zu trainieren, was beispielsweise auf dem Peripheriegerät stattfinden kann, etwa unter Rückgriff auf Daten, die an einer Vielzahl von Nutzungen verschiedener Nasenapplikatoren gewonnen worden sein können.

Die somit erzeugten Behandlungsdaten (applizierte Dosen, Applikationszeitpunkte, Vitalwerte, usw.) bzw. die "Trainingsentwicklung" des "Basis-Algorithmus" (Modell und/oder Parameter) eines jeden einzelnen Benutzers können strukturiert gesammelt und am Ende der Therapie oder in Echtzeit homogenisiert in entsprechenden Datenbanken gesammelt werden. Diese Datenbank kann dann mit Hilfe von "Big Data" Analyse Methoden (z. B. Cluster Analyse, Data Mining, Kurvenanalyse, Musteranalyse, usw.) und/oder Verfahren, Methoden und Technologien der Künstlichen Intelligenz und/oder des Machine Learnings (oder des Sonderfalls des Machine Learnings, das Deep Learning) nach Gemeinsamkeiten, Mustern, Auffälligkeiten, usw. durchsucht werden mit dem Ziel aussagekräftige und umsetzbare Verbesserungen/Erkenntnisse für den "Basis-Algorithmus" abzuleiten bzw. auszuwerten und diese mittels Übertragen zweiter Daten zu implementieren.

In einem optionalen Schritt kann die "eigene" Datenbank mit anderen Datenbanken, wie z. B. Langzeit Patientendaten z. B. EHR (electronic health record), EMR (electronic medical record) und/oder HIS (healthcare information system) und/oder Gendatenbanken und/oder die Kombination von Genotyp mit Phenotypen und/oder klinische Studien und/oder Langzeitstudien z. B. Quips, Pain-Out, usw. und/oder "wearables & sensors" Daten und/oder weiteren Computermodellen wie z.B. In-silico-Medizin Computersimulationen und/oder anderen Quellen vernetzt werden. Ziel dieser Vernetzung ist es, die Datenlage/Basis (Menge) für die "Big Data" Analysemethoden bzw. die KI-Methoden und den daraus resultierenden Ergebnissen zu vergrößern bzw. zu erweitern mit denen dann der "Basis-Algorithmus" "trainiert" wird. Ziel ist es, den "trainierten neuen Basis-Algorithmus" dann für zukünftige Behandlungen zu verwenden. Er kann als zweite Daten verwendet und entsprechend übertragen werden. Alternativ oder ergänzend können zweite Daten basierend auf einem solchen "trainierten neuen Basis-Algorithmus" auf dem Peripheriegerät erzeugt und auf einen Nasenapplikator übertragen werden. Dieser Vorgang kann für jede Indikation individuell angepasst werden.

In einigen Ausführungsformen kann der Nasenaufsatz des Nasenapplikators konfiguriert sein, um Temperatur, Feuchtigkeit, Luftdurchfluss, Unterdruck usw. zu messen.

In manchen Ausführungsformen des Nasenapplikators kann dieser konfiguriert sein, Aufzeichnungen des Schlafrhythmus durchzuführen, und basierend auf dieser Aufzeichnung eine Anpassung der weitergehenden Dosierung (*Dosing Schedule*) vor/während/nach dem Schlaf vorzunehmen etc.

In manchen Ausführungsformen des Nasenapplikators kann dessen Gehäuseoberfläche einen Abperleffekt (Lotus Effekt) aufweisen, d. h. es kann selbstreinigend, antibakteriell etc. vorgesehen sein.

In einigen Ausführungsformen des Nasenapplikators kann das Gehäuse des Nasenapplikators kühlbar sein, z. B. für Insulin. Eine Kühlvorrichtung kann vorgesehen sein, ebenso wärmedämmende Materialen oder Vorkehrungen.

In manchen Ausführungsformen des Nasenapplikators kann das Gehäuse des Nasenapplikators eine Isolation gegen Kälte, Hitze, Feuchtigkeit und/oder Flüssigkeit aufweisen.

In einigen Ausführungsformen des Nasenapplikators können an diese zusätzliche Module anklickbar sein. Entsprechende Halterungen, Vorrichtungen usw. können am Nasenapplikator vorhanden sein.

In manchen Ausführungsformen des Nasenapplikators kann dieser über die AUX- oder Lightning-Buchse mit einem Smartphone, insbesondere mit dem Smartphone des Benutzers/Patienten, verbindbar sein. In diesen Ausführungsformen wird das Smartphone die Elektronik und die entsprechende App die erforderliche Software des Nasenapplikators.

In bestimmten Ausführungsformen sind der Nasenapplikator und/oder eines der Peripheriegeräte programmiert, um ein Lagerungssignal, Aufstehsignal, Sturzsignal, usw. abgeben zu können. Hierzu können smarte Textilien mit integrierten Sensoren, Aktuatoren, Haptik usw., Mobilitätsprotokoll etc. vorgesehen sein.

Der Nasenapplikator und/oder einer der Peripheriegeräte können zum Ausüben diese Funktionalitäten vorgesehen und konfiguriert sein. Sie können genutzt werden zum Abgeben eines Signals an Dritte (z. B. an einen Leitstelle, ins Stationszimmer usw. etwa nach Sturz des Benutzers), sie können aber auch genutzt werden bei der Vorbestimmung der Höhe einer oder mehrerer der nachfolgenden Applikationsdosen. Beispielsweise kann vorgesehen sein, die Abgabe eine Applikationsdosis und/oder der Höhe davon abhängig zu machen, dass der Benutzer zwischenzeitlich aufgestanden ist, also z. B. das Bett doch wenigstens für weniger Schritte oder etwas körperliche Betätigung verlassen hat.

In einigen Ausführungsformen ist der Nasenapplikators konfiguriert, um selbst, insbesondere automatisch, eine Konzentration der Substanz zu mischen und/oder das interne Substanzreservoir zu füllen und/oder mit einer Substanz aus einem zweiten Substanzreservoir zu verdünnen, z. B. 100 µg/100 µL verdünnen auf 50 µg/100 µL.

In manchen Ausführungsformen ist der Nasenapplikator geeignet zum *Energy-Harvesting,* d. h. zur Energiegewinnung z. B. durch eine oder mehrere Drehung(en) am Gehäuseboden. Somit kann ein hybrides System zwischen mechanischen und elektronischen Systemen erzeugt werden wobei die benötigte Energie jeweils bedarfsgerecht erzeugt wird.

In einigen Ausführungsformen ist die Steuervorrichtung weiter programmiert ist, um beim Vorbestimmen der Höhe der nächsten Applikationsdosis Dₙ die festgesetzte Zielkonzentration C_{ED} oder ihren Mindestwert und/oder den ermittelten zeitlichen individuellen Konzentrationsverlauf, insbesondere die ermittelte individuelle PK-Kurve PK_{Ind}, zu berücksichtigen.

In manchen Ausführungsformen die Steuervorrichtung weiter programmiert ist, um beim Vorbestimmen der Höhe der nächsten Applikationsdosis Dₙ die festgesetzte Zielkonzentration C_{ED} oder ihren Mindestwert und/oder den ermittelten zeitlichen individuellen Konzentrationsverlauf, insbesondere die ermittelte individuelle PK-Kurve PK_{Ind}, zu berücksichtigen.

In einigen Ausführungsformen ist die Steuervorrichtung weiter programmiert ist, zum Begrenzen der Höhe der nächsten Applikationsdosis Dₙ und/oder der daraus resultierenden Konzentration; zum Begrenzen der kumulierten Höhe aller innerhalb einer vorbestimmten Zeitdauer abgegebenen Applikationsdosen D₀, D₁, D₂, ..., Dₙ₋₁, Dₙ, Dₙ₊₁, ... und/oder die daraus resultierenden Konzentrationen; und/oder zum Begrenzen der kumulierten Höhe aller abgegebenen Applikationsdosen D₀, D₁, D₂, ..., Dₙ₋₁, Dₙ, Dₙ₊₁, ... die daraus resultierenden Konzentration, wobei die Steuervorrichtung hierzu optional auf im Datenspeicher gespeicherte Daten die Substanz betreffend zurückgreift.

In einigen Ausführungsformen ist eines der Peripheriegeräte programmiert, um von einem ersten der Nasenapplikatoren erste Daten zu empfangen, diese zu verarbeiten, und basierend auf dem Ergebnis der Verarbeitung zweite Daten an einen zweiten der Nasenapplikatoren zu senden.

In einigen Ausführungsformen ist eines der Peripheriegeräte programmiert, um von einem ersten der Nasenapplikatoren erste Daten zu empfangen, diese zu verarbeiten, und basierend auf dem Ergebnis der Verarbeitung zweite Daten an den ersten Nasenapplikator zu senden.

Mittels mancher erfindungsgemäßer Ausführungsformen können einer oder mehrere der hierin genannten Vorteile erzielbar sein, zu welchem Folgenden zählen:
Die Arzneitherapie wird durch die Tatsache erschwert, dass Patienten auf dieselbe Dosis derselben Substanz sehr unterschiedlich reagieren. Es finden sich Unterschiede in der Wirksamkeit, sowie im Spektrum und Schweregrad von unerwünschten Wirkungen und Nebenwirkungen.

Ein wesentlicher Anteil der interindividuellen Variabilität (d. h. Unterschiede zwischen verschiedenen Patienten) einzelner Arzneistoffe kann auf die genetische Ausstattung (Pharmakogenetik) des Patienten zurückgeführt werden. Eine identische Dosierung bei unterschiedlichen Patienten führt zu sehr unterschiedlichen Konzentrations-Zeit-Kurven (Pharmakokinetik) und damit zu sehr unterschiedlichen Wirkungen (Pharmakodynamik) und somit ist eine Dosisindividualisierung vorteilhaft. Zusätzlich zu der Interindividuellen Variabilität sollte man die intraindividuelle Variabilität d. h. Unterschiede bei einem einzelnen Patienten in eine Arzneitherapie mit berücksichtigen. Die vorliegende Erfindung kann hierzu einen Beitrag leisten.

Insbesondere bei Mehrfachgabe von Substanzen mit enger therapeutischen Breite, langer Eliminationsphase und Nebenwirkungen muss der Kumulationseffekt (Akkumulation bzw. Anreicherung der Substanz) über die Gabe mehrerer, aufeinanderfolgender Applikationsdosen berücksichtigt werden. Die vorliegende Erfindung kann erlauben, eine für den Benutzer sichere Applikation einzelner oder mehrerer solcher Applikationsdosen durch diesen selber durchführen zu lassen.

Letzteres gilt insbesondere für ältere Menschen, für die eine korrekte bzw. individuell angepasste Dosierung eine ausschlaggebende Rolle spielt. Die vorliegende Erfindung kann hierzu einen Beitrag leisten.

Das vorsichtige "Titrieren" (was in einer Bestimmungs- oder Findungsphase erfolgt) auf eine Substanzkonzentration und das Einstellen der unterschiedlichen Applikationsdosen bzw. des therapeutischen Effekts unter Vermeiden eines "Überschießens", und die damit verbundene Vermeidung von unerwünschten Nebenwirkungen kann einen weitere Vorteil darstellen.

Hierzu kann das Regeln auf die Zielkonzentration (C_{ED}) von Vorteil sein. Im Fall der Schmerztherapie steht das Erreichen der Zielkonzentration für einen Zustand, in welchem der Patient mit seine Schmerztherapie zufrieden ist. Eine über die Zielkonzentration hinausgehende Konzentration trägt nicht mehr zur therapeutischen Wirkung bei, sondern erhöht nur mehr die zu vermeidenden Nebenwirkungen.

Die vorliegende Erfindung kann es erlauben, applikationsbedingte Problemstellungen von Nasenapplikatoren, wie die unterschiedliche Nasenanatomie, der Nasenzustand, der Applikationswinkel, wie tief der einzelnen Benutzer gewöhnlich den Nasenaufsatz einführt und dergleichen, beheben: eine voreingestellte Höhe der Applikationsdosis, deren Wirkung im Körper von den vorgenannten und weiteren Faktoren abhängt, ist erfindungsgemäße nicht erforderlich. Die vorliegende Erfindung ist geeignet, auf solche, vor allem interindividuelle, Besonderheiten bei der Vorbestimmung der Höhe der Applikationsdosen einzugehen. Somit kann der gewünschte therapeutische Effekt gesichert eintreten.

Wird die Substanz, wie ebenfalls von der Erfindung umfasst, nasal appliziert, also nicht invasiv, so können Nachteile anderer Applikationsformen vermieden werden. Bei der oralen Gabe beispielsweise muss die Substanz erst über die Leber metabolisiert werden (First-Pass Effekt) bevor sie ihre Wirkung entfalten kann. Weitere Nachteile sind der verhältnismäßige langsame Wirkeintritt und die Schwierigkeit individuelle Dosen zu verabreichen. Erschwerend für die orale Gabe sind Symptomatiken wie Schluckstörung und trockener Mund. Bei der sublingualen Gabe beispielweise ist die relative kleine Absorbtionsfläche und das versehentliche Verschlucken problematisch. Die Wirkung wird ebenfalls beeinträchtigt durch zu frühes trinken, essen oder sprechen nach der sublingualen Applikation.

Die intranasale Verabreichung mittels des erfindungsgemäßen Nasenapplikators ist schmerzfrei, leicht selbstverabreichbar, und wirkt rasch.

Das substanzbezogene Sperren weiterer Applikationen während einer Sperrdauer, welche überdies variabel und erfindungsgemäß von der Sperrvorrichtung in Beginn und Dauer einstellbar ist, kann ein zeitiges Finden der Zielkonzentration erlauben, und kann eine überschießende Gabe der Substanz vorteilhaft verhindern. Die Sperrvorrichtung stellt sicher, dass die applizierte Dosis ihren vollen Effekt etablieren kann und dadurch die Wirkung vom Benutzer bewertet werden kann.

Erfindungsgemäß wird somit vorteilhaft ein selbstregulierendes System vorgeschlagen, welches sich nach einer "Titrationsphase" (welche eine Bestimmungs- oder Findungsphase ist) anhand bereits abgegebener Applikationsdosen, das Betätigungsverhaltens des Benutzers und den vergangenen Zeitintervallen individualisierte bzw. personalisierte Applikationsdosen, bei einer Mehrfachdosierung, bestimmt und abgibt, welche den Bedürfnissen des Benutzers, entsprechend bzw. den gewünschten therapeutischen Effekt bewirkend, entspricht.

Erfindungsgemäß wird der Körper des Benutzers als Regelkreis bzw. Regelstrecke verwendet, da nur der Körper des Benutzers wiedergibt was die Substanz mit dem Körper macht (Pharmakodynamik) und die jeweilige Applikationsdosis bei Anfrage/Bedarf benutzerindividuell bestimmt wird und appliziert werden kann.

Erfindungsgemäß kann ferner unter Nutzung der Eigenschaften der Pharmakokinetik der Benutzer kategorisiert und der zeitliche Verlauf der Konzentration im Körper verfolgt werden. Nach einer initialen "Titrationsphase" kann man für den entsprechenden Benutzer zuordnen, ob er innerhalb z. B. der Gruppe der Pharmakokinetik zum Mittelwert oder "-SD" oder "+SD" tendiert und/oder seine individuelle Konzentrations-Zeit-Kurve definieren. Da erfindungsgemäße auch die Zielkonzentration, bei der der gewünschte therapeutische Effekt auftritt, ermittelt werden kann, leistet die vorliegende Erfindung vorteilhaft einen Betrag zur Weiterbildung der personalisierten Medizin.

Interindividuelle Variabilität (z. B. Alter, Vorerkrankungen, Medikamentenverbrauch) und intraindividuelle Variabilität (z. B. Manipulation, Mobilisation, zirkadianer Verlauf, Verbesserung des Krankheitszustands bzw. Genesungszustands) können von der Steuervorrichtung vorteilhaft berücksichtigt werden.

Ein Tätigwerden der Ärztin oder anderes berechtigtes Personal ist vorteilhafterweise hierbei nicht erforderlich, was Zeit und Ressourcen einspart.

Schließlich kann die vorliegende Erfindung dazu beitragen, dass vorteilhafterweise eine geringere Menge an Substanz zum Erzielen desselben Effekts nötig ist.

Ein "Abfangen" der Restmedikation z. B. aus dem OP, die als Ausgangs- oder Initialdosis abgegeben worden sein kann, ist erfindungsgemäß ebenfalls vorteilhafterweise möglich.

Im Folgenden wird die Erfindung anhand exemplarischer Ausführungsformen derselben unter Bezugnahme auf die angehängte Zeichnung beschrieben. In den Figuren gilt:
- **Fig. 1**: zeigt einen Nasenapplikator als Beispiel einer Medikamentenapplikationsvorrichtung einer ersten exemplarischen Ausführungsform, sowie ein erfindungsgemäßes System;
- **Fig. 2**: zeigt schematisch und exemplarisch einen möglichen zeitlichen Verlauf einer Nutzung des erfindungsgemäßen Nasenapplikators als Beispiel einer Medikamentenapplikationsvorrichtung;
- **Fig. 3**: zeigt schematisch und exemplarisch die Höhe kumulierter Dosen verschiedener Applikationen einer Substanz an den Benutzer;
- **Fig. 4**: zeigt schematisch und exemplarisch das Ergebnis einer Vorbestimmung der Höhe der verschiedenen Applikationsdosen;
- **Fig. 5a**: zeigt schematisch anhand dreier exemplarisch ausgewählten zeitlichen Konzentrationsverläufe das Ermitteln eines individuellen Konzentrationsverlaufs für einen betrachteten Benutzer der erfindungsgemäßen Nasenapplikators; und
- **Fig. 5b**: zeigt eine Fortführung des der Erfindung in manchen Ausführungsformen zugrundeliegenden Gedankens der Fig. 5a.

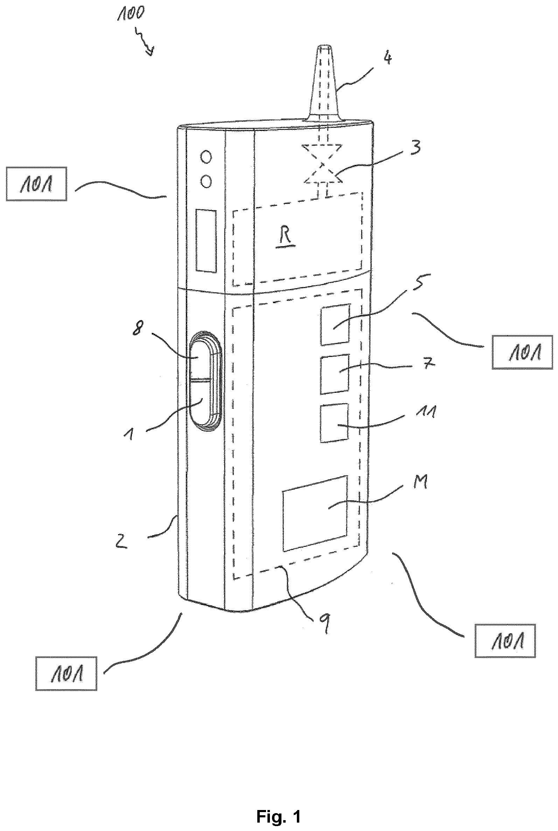

**Fig. 1** zeigt einen Nasenapplikator 100 einer ersten exemplarischen Ausführungsform in einer schematisch vereinfachten Darstellung.

Wie Fig. 1 zeigt, weist der Nasenapplikator 100 ein Gehäuse 2 auf, in oder an welchem optional eine, alle oder mehrere der im Folgenden genannten Vorrichtungen oder Komponenten unabhängig voneinander angeordnet sein können. In anderen Ausführungsformen können einzelne oder mehrerer dieser Komponenten in beliebiger Kombination auch extern, also nicht innerhalb des Gehäuses 2, vorliegen.

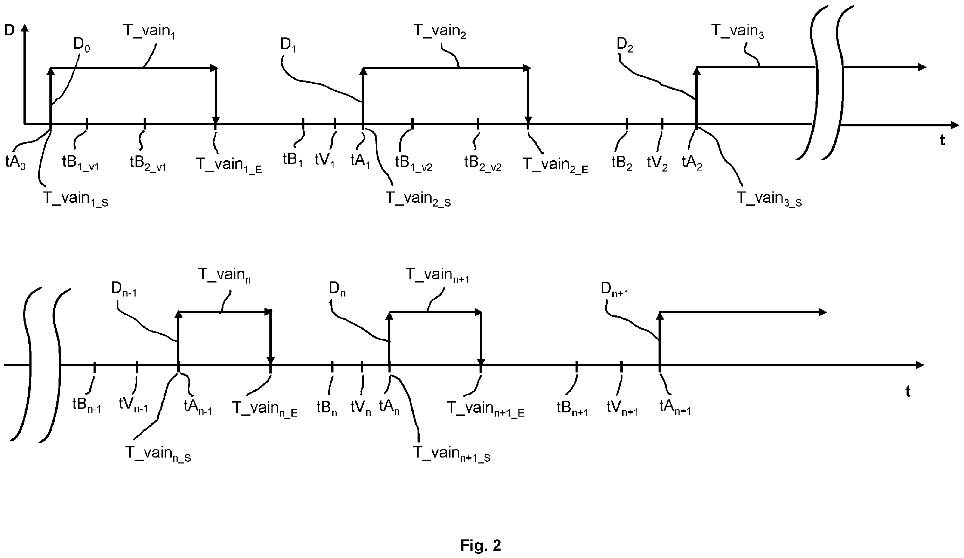

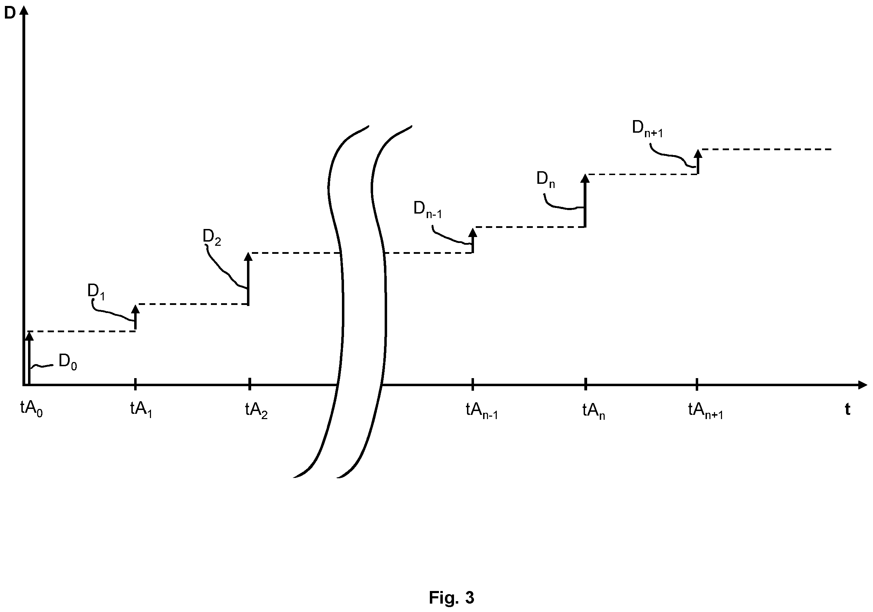

Der Nasenapplikator 100 weist eine Abrufvorrichtung 1 auf, welche der Benutzer zu betätigen hat, um sich eine Dosis D (siehe Fig. 2 oder 3) einer Substanz S aus einem Substanzreservoir R applizieren zu können. Die Substanz S kann ein medizinischer oder nicht-medizinischer Wirkstoff, insbesondere ein Schmerzmittel, sein. Die Abrufvorrichtung 1 kann zugleich eine optional vorgesehene Feedbackvorrichtung 8 sein oder aufweisen. Die Feedbackvorrichtung 8, sofern vorhanden, kann alternativ hierzu eine eigenständige Vorrichtung sein.

Hat der Nutzer die Abrufvorrichtung 1 betätigt, welche ein Schalter, ein Knopf, ein Einschub oder dergleichen sein kann, und damit ein Betätigungssignal ausgelöst, so kann ein Abgabedosierer 3 eine von ihm dosierte, also mengenmäßig oder volumenmäßig bestimmte Menge der Substanz S freigeben. Sie kann aus dem Reservoir R über das Lumen eines in ein Nasenloch einzuführenden Nasenaufsatzes 4 (in die Nase des Benutzers einströmen, einspritzen oder dergleichen. Sie kann alternativ aus dem Reservoir R über einen Tröpfengenerator in die Nase appliziert werden, der Teil des Nasenaufsatzes 4 oder einer anderen Einrichtung des Nasenapplikators 100 sein kann.

Auf das Reservoir R kann hierfür optional mittels eines Energiebeaufschlagungsmechanismus eingewirkt werden. Dieser kann mittels Druck, Kraft, Geschwindigkeit, Schwingung, Vibration, Rotation, elektrische/magnetische Kräfte, usw. wirken. Der Tröpfchengenerator, der beispielsweise Teil des Abgabedosierers 3 oder des Nasenaufsatzes 4 sein kann, kann somit beispielsweise ein Druckbeaufschlagungsmechanismus sein, der auf die Substanz S einwirkt; z. B. drückt ein Kolben in das Reservoir R, oder eine Pumpe zieht aus dem Reservoir R und beaufschlagt die Substanz S mit Druck, Kraft, Geschwindigkeit, usw. und schiebt diese durch den Tröpfchengenerator.

Der optionale Tröpfchengenerator kann mechanisch und/oder elektrisch funktionieren. Er kann z. B. als Düse, Verdampfer, Piezo-Element, usw. ausgeführt sein, oder dergleichen aufweisen. Er kann vorzugsweise einen Sprühnebel oder eine Tröpfenwolke oder eine Partikelwolke erzeugen.

Da eine solche Freigabe durch den Abgabedosierer 3 nicht zu jedem Zeitpunkt und nicht aufgrund jeder Betätigung der Abrufvorrichtung 1 und/oder nicht zu jedem Zeitpunkt möglich sein soll, ist eine Sperrvorrichtung 5 vorgesehen. Sie ist konfiguriert, um, vorzugsweise zeitgesteuert, eine Freigabe oder Abgabe mittels des Abgabedosierers 3 während einer nach vorbestimmten Kriterien hinsichtlich ihrer Dauer und/oder des Zeitpunktes ihres Beginns und/oder Endes festgelegten Sperrdauer (siehe z. B. T_vainₙ in Fig. 2) zu verhindern, nicht zuzulassen, usw., selbst wenn die Abrufvorrichtung 1 während der Sperrdauer betätigt werden sollte. Während der Sperrdauer, welche in einigen Ausführungsführungsformen von fester oder unveränderlicher Dauer ist, während sie in anderen variabel oder veränderbar ist, führt eine Betätigung der Abrufvorrichtung 1 grundsätzlich oder generell nicht zu einer Applikation, wohingegen dies für eine Betätigung nach Ablauf der Sperrdauer nicht zutreffen muss oder nicht zutrifft.

Die Menge oder das Volumen jenes Teils der Substanz S, welcher in einzelnen Ausgangs- oder Applikationsdosen (Do, D₁, D₂,..., Dₙ, ... in Fig. 2 oder in Fig. 3) wie nachfolgend erläutert ist, zu verschiedenen Abgabezeitpunkten tA₀, tA₁, tA₂, ..., tAₙ, ... abgegeben wird und/oder abgegeben wurde, wird mittels einer Dosiserfassungsvorrichtung 7 erfasst. Die Dosiserfassungsvorrichtung 7 kann konfiguriert sein, um die einzelnen Ausgangs- oder Applikationsdosen D₀, D₁, D₂,..., Dₙ, ..., welche mit dem Nasenapplikator 100 abgegeben wurden und welche variabel, also keine fixen Dosismengen sind, zu messen, festzustellen, zu speichern, aufzusummieren usw.

Die Dosiserfassungsvorrichtung 7 kann beispielsweise eine Menge, ein Volumen oder einen anderen Parameter messen, welcher eine unmittelbare Aussage über die jeweilige Applikationsdosis erlaubt. Die Dosiserfassungsvorrichtung 7 kann ergänzend oder alternativ einen Parameter messen oder ermitteln, welcher eine mittelbare Aussage über die jeweilige Applikationsdosis erlaubt, etwa einen Fluss pro Zeit, eine Anzahl von Hüben einer Pumpe, eine Anzahl von Umdrehungen einer Pumpe, oder dergleichen.

Dosislimits und/oder Konzentrationslimits, welche eine, z. B. bezogen auf eine Zeiteinheit beschränkte oder vorbestimmte, Gesamtdosis und/oder Gesamtkonzentration beschränken, können optional vorgesehen sein.

Die Dosiserfassungsvorrichtung 7 kann ergänzend oder alternativ programmiert sein, um einen Zeitpunkt, zu welchem eine oder mehrere, konkrete Applikationsdosen mittels des Nasenapplikators 100 verabreicht wurden oder werden, festzuhalten. Die Dosiserfassungsvorrichtung 7 kann weiterhin ergänzend oder alternativ programmiert sein, um eine Zeitdauer zu erfassen, beispielsweise zwischen zwei oder mehreren Abgabezeitpunkten tA₀, tA₁, tA₂,..., tAₙ, ... (siehe Fig. 2 oder Fig. 3) verabreichter Ausgangs- oder Applikationsdosen D₀, D₁, D₂,..., Dₙ, .... Weitere Zeitdauern zwischen beliebigen hierin genannten Zeitpunkten können ebenfalls erfasst werden (wie z. B. tA₁ -/"minus" T_vain_{1_E}, tA₁ - (insbesondere letztes) tB_{_v}, tB_{1_v1} - tA₀, tB_{_v} (insbesondere das letzte, also tB_{_v-1}) - tA₀, T_{_}vain_{1_E}-letzte tB_{_v}, T_{_}vain_{1_E} - tB_{1_v1}, welche deren Parameter nachstehen erläutert sind.

Eine elektronische Steuervorrichtung 9 kann vorgesehen sein, um die Funktion des Nasenapplikators 100 oder einige, beliebige oder alle der mit Bezug auf Fig. 1 genannten Vorrichtung oder Komponenten zu steuern, zu regeln und/oder zu überwachen. Sie kann, wie vorstehend ausgeführt, im Gehäuse 2 oder außerhalb vorgesehen sein. Sie kann Empfänger von Angaben über Ausgangs- oder Applikationsdosen D₀, D₁, D₂,..., Dₙ, ..., sein, die extern vorbestimmt wurden. Entsprechende kabelgebundene oder kabellose Signalverbindungen können, uni-direktional oder bi- oder mehr-direktional, zwischen der Steuervorrichtung 9 und der oder den Komponenten des Nasenapplikators 100 vorgesehen sein.

Eine Erfassungsvorrichtung 11 kann vorgesehen sein, um zu erfassen, ob, dass und/oder wann der Benutzer, der hierin ein Nutzer, Anwender oder Patient sein kann, durch Betätigen der Abrufvorrichtung 1 versucht oder initiiert hat, sich eine weitere Applikationsdosis zu applizieren.

Das Substanzreservoir R kann in jeder Ausführungsform optional austauschbar sein, beispielsweise als ein Single-Shot-Reservoir, als Reservoir mit einer ausreichenden Substanz S für eine Vielzahl von Applikationsvorgängen, usw.

Das Substanzreservoir R kann beispielsweise vorbefüllt, selbstbefüllbar, integriert, austauschbar, für einmalige Applikation und/oder für mehrfache Applikation ausgelegt sein.

Das Substanzreservoir R kann im Inneren des Gehäuses 2 oder extern hierzu angeordnet sein.

Die Substanz kann jeden Aggregatzustand aufweisen (flüssig, gasförmig, fest, etc.).

Ein Datenspeicher M zum Speichern von Daten, oder mit hierin gespeicherten Daten, kann im Inneren des Gehäuses 2 oder extern hierzu angeordnet sein.

Diese Daten können den bisherigen Gebrauch des Nasenapplikators 100 durch den Benutzer betreffen, ebenso Zeitangaben zu den hierin genannten Zeitpunkten oder Zeitdauern (synonym: Zeitintervalle) usw.; sie können das Ergebnis einer Auswertung durch die Steuervorrichtung 9 sein, usw. oder optional Daten für pharmakologische, pharmakodynamische, pharmakometrische und/oder pharmakokinetische Daten die Substanz S und/oder zugrundeliegende medizinische Umstände wie die Grunderkrankung usw. betreffend; sie können substanzbezogen und/oder vom Arzt oder anderem berechtigten Personen festgelegt sein, z. B. ein tₘₐₓ-Wert der Substanz S, ein vom Arzt korrigierter, sog. "overruled" tₘₐₓ-Wert, z. B. der Zeitpunkt oder das Ende des Zeitintervals nach Applikation, zu oder in dem die maximale Konzentration oder Blutkonzentration eintritt, die Halbwertzeit, die terminale Halbwertszeit, ein vom Arzt oder den anderen berechtigten Personen angepasster Wert, usw.

Die Daten können objektiv und/oder subjektiv sein.

Die Daten können objektive Werte (Sensorwerte) sein, z. B. Blutzuckerspiegel, Blutdruck, Herzrate, Atemfrequenz, Sauerstoffsättigung, usw.

Die Daten können subjektive Werte ("Effektwerte") sein, welche man nicht direkt messen kann. Sie werden abgeleitet anhand von erhobenen Werten wie z. B. Schmerzscore, Sedationscore, Nebenwirkungen etc.) und/oder gemessenen Werte (z. B. SpO₂, HR, HRV etc.)

Die Daten können empfohlene Konzentration, Kontraindikationen, Nebenwirkungen, Verfallsdatum, und/oder Beipackzettel, Fachinformation, Patienteninformation, Summary of Product Characteristics (SmPC), Montageanleitung, Demontageanleitung, Betriebsanweisung/Bedienungsanleitung etc. sein oder umfassen.

Die Daten können optional neben pharmakologischen und/oder pharmakokinetischen Daten, benutzerbezogene Daten wie z. B. Gewicht, Alter, Geschlecht, BMI, Vorerkrankungen, Komedikationen, Komorbiditäten, Allergien, etc. umfassen. Generell die Benutzer- und/oder Behandlungshistorie (z. B. Daten zu vorherigen Eingriffen, Prozeduren, Diagnosen, Anamnesen, Tests, erhaltene/verschriebene Medikationen, erhaltene Narkosemittel, Muskelrelaxans, Antiemetika usw.) umfassen.

Solche und andere Daten können der Steuervorrichtung 9 helfen, genauere Dosierungen zu berechnen, bzw. die Sicherheit der Therapie zu erhöhen.

Der Datenspeicher M kann ein Hardware-Memory-Bauteil, ein Flash-Speicher oder ein externer Datenspeicher sein, beispielsweise angeordnet in einer Cloud, in einem Mobilgerät oder dergleichen und/oder via Wi-Fi/ Bluetooth etc. mit weiteren Komponenten des Nasenapplikators 100 in Signalverbindung stehen.

Die Sperrvorrichtung 5 und/oder die Erfassungsvorrichtung 11 sind vorzugsweise elektronisch, z. B. mittels der Steuervorrichtung 9, verwirklichte Komponenten.

Ein Timer oder Zeitmesser, etwa eine Uhr, kann vorgesehen sein. Die einzelnen Komponenten, wie vorstehend genannt, können auf sie zurückgreifen. Es kann in manchen Ausführungsformen vorgesehen sein, den Benutzer über den Ablauf der aktuellen Sperrdauer zu informieren, beispielsweise optisch oder akustisch oder haptisch, etwa mittels Vibration.

Da in Fig. 1 eine Reihe von Peripheriegeräten 101 angedeutet sind, zeigt diese Figur auch ein erfindungsgemäßes System.

Die Peripheriegeräte 101 können gleich ausgestaltet sein, oder sich, zumindest einigen von ihnen, voneinander unterscheiden.

So können manche der Peripheriegeräte 101 Nasenapplikatoren sein, die optional wie der Nasenapplikator 100 ausgestaltet sind, und/oder als Docking Station, als Server, usw. ausgestaltet sein, vorzugsweise wie hierin beschrieben.

Optional können manche oder alle der Peripheriegeräte 101 in beliebiger Kombination miteinander oder untereinander kommunizieren, oder nicht.

In Fig. 1 ist angedeutet, dass sie alle auch mit dem Nasenapplikator 100 kommunizieren können, dies ist allerdings ebenfalls optional. In manchen Ausführungsformen können nur manchen der Peripheriegeräte 101 dies.

Weiter kann optional vorgesehen sein, dass der Nasenapplikator 100 und/oder die Peripheriegeräte 101 und/oder die Peripheriegeräte 101 über den Nasenapplikator 100 und/oder die Docking station und/oder ein anderes Peripheriegerät 101 nach extern kommunizieren können.

**Fig. 2** zeigt schematisch und exemplarisch einen möglichen zeitlichen Verlauf einer Nutzung des erfindungsgemäßen Nasenapplikators 100.

Betätigt der Benutzer die Abrufvorrichtung 1, um sich beispielsweise eine hierin als "nächste" Applikationsdosis bezeichnete Applikationsdosis Dₙ zu applizieren, so wird mittels der elektronischen Steuervorrichtung 9 oder mittels ihrer Erfassungsvorrichtung 11 festgestellt, ob dieser Zeitpunkt, zu dem der Benutzer sich diese nächste Applikationsdosis Dₙ applizieren möchte, möglichweise in eine noch andauernde Sperrdauer T_{_}vainₙ fällt oder nicht.

Fällt dieser Zeitpunkt, zu welchem die Abrufvorrichtung 1 betätigt wird, in die Sperrdauer T_{_}vainₙ, so wird dem Benutzer jedenfalls zu diesem Zeitpunkt keine Applikationsdosis Dₙ appliziert. Solche Zeitpunkte gehen aus Fig. 2 mit Bezug auf eine frühere Sperrzeit T_{_}vain₁ exemplarisch als Zeitpunkte tB_{1_v1} und tB_{2_v1} mit Bezug auf weitere Sperrzeiten z. B. als tB_{1_v2}, hervor und werden optional mit in die Vorbestimmung einbezogen.

Fällt der Zeitpunkt der Betätigung der Abrufvorrichtung 1 hingegen nicht in die Sperrdauer T_{_}vainₙ, sondern liegt nach dem Ablauf oder Ende T_{_}vain_{n_E} der Sperrdauer T_{_}vainₙ wie im Beispiel der Fig. 2 anhand des Betätigungszeitpunkts tBₙ gezeigt, dann wird dem Patienten zu einem hierin als nächster Abgabezeitpunkt tAₙ bezeichneten Zeitpunkt die nächste Applikationsdosis Dₙ appliziert.

Die Höhe der nächsten Applikationsdosis Dₙ wird zu einem hierin als nächsten bezeichneten Vorbestimmungszeitpunkt tVₙ vor ihrer Applikation von der elektronischen Steuervorrichtung 9 festgelegt oder vorbestimmt, die entsprechende Berechnungen selber ausgeführt oder Ergebnisse solcher Berechnungen übernommen haben kann.

Die Vorbestimmung der Höhe der nächsten Applikationsdosis Dₙ wird vom Nasenapplikator 100 oder seinen zu Fig. 1 genannten Komponenten, etwa der Steuervorrichtung 9, gleich ob sie vom Gehäuse 2 des Nasenapplikators 100 umfasst sind oder extern hierzu liegen, festgelegt.

Der erste Vorbestimmungszeitpunkt tVₙ kann, wie auch der Betätigungszeitpunkt tBₙ dabei zu oder vor dem ersten Abgabezeitpunkt tAₙ liegen, er kann somit mit dem ersten Abgabezeitpunkt tAₙ zusammenfallen, jedenfalls im Rahmen des technisch Möglichen, so dass zwischen erstem Vorbestimmungszeitpunkt tVₙ und nächstem Abgabezeitpunkt tAₙ ausreichend Zeit zum tatsächlichen Vorbestimmen bzw. Berechnen der Höhe der nächsten Applikationsdosis Dₙ und Zeit zum Ansprechen der entsprechenden Vorrichtungen oder Komponenten, welchen die vorbestimmte Höhe vor Applikation per Signal oder auf andere Weise mitgeteilt werden muss, verbleibt.

Bei der Vorbestimmung der Höhe der nächsten Applikationsdosis Dₙ, welche ein Beispiel der variablen Dosis D ist, also gerade nicht oder nicht ausschließlich durch Arzt oder anderen berechtigten Personen, Hersteller oder dergleichen für den Nasenapplikator 100 unveränderbar voreingestellt ist, können aus dem Datenspeicher M auslesbare Daten und/oder Daten, welche von den in Fig. 1 genannten Komponenten des Nasenapplikators 100 ermittelt wurden, berücksichtigt werden.

Zu diesen Daten zählt vorzugsweise bereits ein vor dem erfolgreichen Betätigungszeitpunkt tBₙ gelegener Ausgangszeitpunkt tA₀, zu welchem dem Benutzer möglicherweise eine Ausgangsdosis D₀ noch vor Abgabe der ersten Applikationsdosis D₁ verabreicht worden sein kann, sei es mittels des erfindungsgemäßen Nasenapplikators 100, von medizinischem Personal, z. B. noch in einem Aufwachraum postoperativ (post-OP), usw. Weiter können diese Daten die Höhe dieser optionalen Ausgangsdosis D₀ umfassen.

Im vorliegenden Fall wird angenommen, dass eine Ausgangsdosis D₀ zu einem Ausgangszeitpunkt tA₀, an den Benutzer abgegeben wurde.

Zu diesen Daten zählt vorzugsweise jeder vor dem erfolgreichen Betätigungszeitpunkt tBₙ gelegener Ausgangszeitpunkt tA₀, tA₁, tA₂, tAₙ₋₁, zu welchem dem Benutzer bereits eine Ausgangs- oder Applikationsdosis D₀, D₁, D₂, Dₙ₋₁ verabreicht wurde oder er sich selbst eine Dosis verabreicht hatte. Alternativ oder ergänzend zählen auch die seitdem jeweils verstrichene Zeit und vorzugsweise auch die jeweilige Höhe der vorstehend genannten Dosen dazu.

Betrachtet man die geplante Abgabe der nächsten Applikationsdosis Dₙ, so zählen zu diesen Daten beispielsweise zumindest die Höhe der vorangegangenen Applikationsdosis Dₙ₋₁ sowie, optional, deren Abgabezeitpunkt tAₙ₋₁ oder die seitdem verstrichene Zeit.

Das Vorbestimmen, welches zum ersten Vorbestimmungszeitpunkt tVₙ erfolgt, ergibt somit die Höhe der nächsten Applikationsdosis Dₙ. Bestimmt wird die Höhe zum nächsten Vorbestimmungszeitpunkt tVₙ, der nach dem nächsten Betätigungszeitpunkt tBₙ liegt, und nicht durch Arzt oder anderen berechtigten Personen, Hersteller oder dergleichen voreingestellt ist, was auch für alle oder manche der weiteren hierin genannten Applikationsdosen gelten kann. Das Vorbestimmen zum nächsten Vorbestimmungszeitpunkt tVₙ erfolgt unter Berücksichtigung von aus dem Datenspeicher M ausgelesenen Daten.

Nach erfolgter Applikation der nächsten Applikationsdosis Dₙ wird, vorzugsweise zum Abgabezeitpunkt tAₙ der nächsten Applikation Dₙ, erneut eine Sperrdauer T_{_}vainₙ angesetzt und/oder begonnen. Sie kann dieselbe Länge wie vorangegangene oder nachfolgende Sperrdauern haben, oder hiervon abweichen. Die Länge der jeweiligen Sperrdauer kann vorbestimmt sein und/oder z. B. vom Arzt oder anderen berechtigten Personen eingestellt worden sein. Beim Ansetzen der nächsten Sperrdauer kann einer Logik oder einem Algorithmus gefolgt werden, für welche(n) bei jedem Vorbestimmungszeitpunkt tVₙ mitberechnet werden kann.

Wie Fig. 2 zu entnehmen ist, kann sich das vorstehend zu dieser Figur Beschriebene wiederholen.

Beim Vorbestimmen der Höhe einer übernächsten Applikationsdosis Dₙ₊₁ können nun aber, optional, neben den Höhen und/oder den Abgabezeitpunkten der vorstehend genannten Applikationsdosen und ggf. auch der Ausgangsdosis D₀ dann erstmals auch die Höhe und/oder der Applikationszeitpunkt tAₙ der hierin als nächsten Applikationsdosis bezeichneten, dann aber bereits applizierten Applikationsdosis Dₙ berücksichtigt werden, welche zwischenzeitlich als Daten erfasst und ebenfalls im Datenspeicher M vorgehalten werden können.

Das Vorbestimmen der Höhe der übernächsten Applikationsdosis Dₙ₊₁, welches zum Vorbestimmungszeitpunkt tVₙ₊₁ erfolgt, kann daher nun auch die Länge der Zeitspanne, die zwischen dem übernächsten Abgabezeitpunkt tAₙ₊₁ und dem nächsten Abgabezeitpunkt tAₙ sowie die Längen der Zeitspannen, die zwischen dem übernächsten Abgabezeitpunkt tAₙ₊₁ und dem Ausgangszeitpunkt tA₀ oder früheren Abgabezeitpunkten tA₁, tA₂ liegen, berücksichtigen.

**Fig. 3** zeigt schematisch und exemplarisch die applizierten Einzeldosen D₀, D₁, D₂, ..., Dₙ₋₁, Dₙ, Dₙ₊₁, ... und die über den Verlauf der Zeit t kumulierten Dosen der Substanz S, deren Höhen und/oder Abgabezeitpunkte tA₀, tA₁, tA₂, ..., tAₙ₋₁, tAₙ, tAₙ₊₁, ...usw. in die Vorbestimmung jeweils späterer Applikationsdosen einfließen können.
Die Zeitachse t sowie ihre Skalierung entsprechen dabei der in Fig. 2 verwendeten oder sind gedanklich als identisch zu anzunehmen. Der Zeitpunkt tA₁ und der Zeitpunkt tA₂ entsprechen somit den in Fig. 2 gezeigten absoluten Zeitpunkten tA₁ und tA₂.

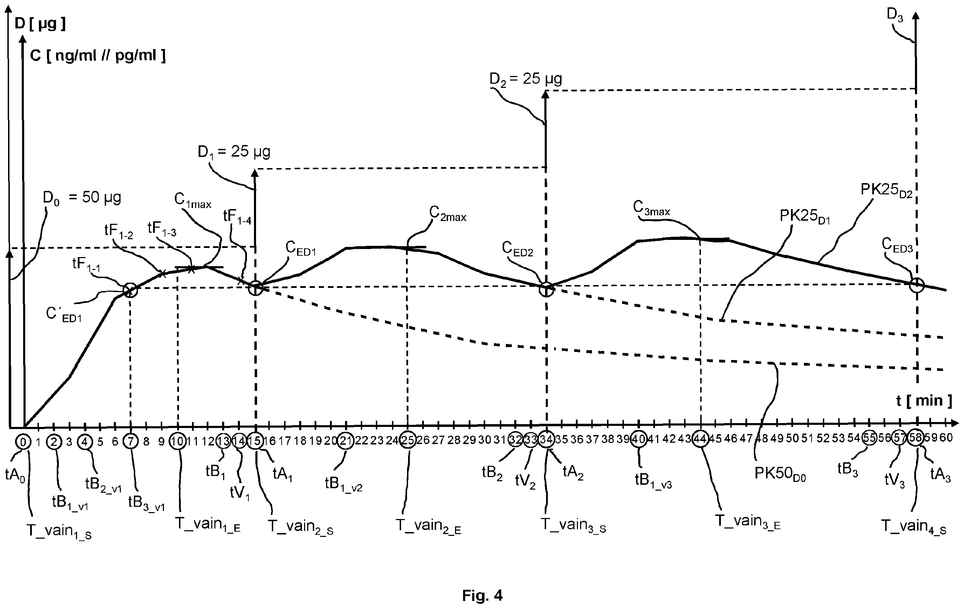

**Fig. 4** beschreibt schematisch und exemplarisch die hierin auch als "Titrationsphase" (welche eine Bestimmungs- oder Findungsphase ist) bezeichnete Phase in der Versorgung des Benutzers mit der Substanz S, in welcher die, den vom Benutzer gewünschten, therapeutischen Effekt bewirkende Zielkonzentration bestimmt wird. Auf ihr kann anschließend die Höhe folgender Applikationsdosen bestimmt werden und/oder zeitgesteuert ein individueller, dosis- bzw. konzentrationsbasierter Behandlungsplan aufgestellt werden.

Fig. 4 zeigt schematisch und exemplarisch das Ergebnis einer Vorbestimmung der Höhe der verschiedenen Applikationsdosen D₁, D₂ und weiterer, die jeweils ein Beispiel für die jeweils nächste Applikationsdosis Dₙ, wie hierin verwendet sein können.

Zur Vorbestimmung der jeweiligen Applikationsdosis D₁, D₂, und weiterer wird in der hier diskutierten Ausführungsform auf Daten aus dem Datenspeicher M zurückgegriffen, welche pharmakologische, pharmakodynamische, pharmakometrische und/oder pharmakokinetische Daten umfassen. Die Daten können Modelle, insbesondere PK-Modelle, sein, ebenso PK-Kurven. Sie sind vorzugsweise patientenindividuell.

Zu diesen pharmakologischen, pharmakodynamischen, pharmakometrischen und/oder pharmakokinetischen Daten oder Modellen zählen im Beispiel der Fig. 4 hierin als PK-Kurven bezeichnete zeitliche Verläufe von Konzentrationen der Substanz S im Körper des Benutzers nach den jeweils applizierten Dosen. Die abgebildeten Kurven spiegeln den zeitlichen Verlauf der Konzentration von einer konkreten Substanz S, welche aus der applizierten Dosis resultieren, und der Höhe der applizierten Dosen wider. Die Kurven spiegeln zudem die Prozesse der Resorption, Verteilung, Metabolisierung und Elimination im Körper wider. Derartige Pharmakokinetik Modelle folgen statistischen Verteilungen z. B. unter Berücksichtigung von mit Mittelwert und Standardabweichung und sind zumeist patientenindividuell, d. h. sie können von Patient A zu Patient B unterschiedlich verlaufen, weshalb sie auch für den unterschiedlichen Benutzer oder Benutzerkollektive entsprechend unterschiedliche statistischen Verteilungen im Datenspeicher M gespeichert sein können. Da der Verlauf solcher PK-Kurven vom Applikationsweg und von der Dosis abhängt, sind die beiden in Fig. 4 mit Bezugszeichen versehenen PK-Kurven einmal als PK50 für die intranasale Gabe von 50 µg Fentanyl und einmal als PK25 für die intranasale Gabe von 25 µg Fentanyl bezeichnet.

Zu pharmakologischen, pharmakodynamischen, pharmakometrischen und/oder pharmakokinetischen Daten, wie sie im Datenspeicher M gespeichert sein können, zählen im Beispiel der Fig. 4 optional auch Angaben zur Substanz S wie tₘₐₓ, welche den Zeitpunkt angeben können, zu welchem nach Applikation einer Dosis D die höchste Konzentration C(t) im Körper, etwa im Blut, vorliegt. Die Dauer von tₘₐₓ kann, zumindest anfänglich oder im Nachgang zu einer ersten Dosis, hier der Ausgangsdosis D₀, als Sperrdauer verwendet werden. Ebenso kann die Sperrdauer an tₘₐₓ angelehnt sein, indem die Sperrdauer z. B. nur 90% von tₘₐₓ beträgt, oder um eine Anzahl von Minuten kürzer oder länger als tₘₐₓ kann bestimmt wird, usw. Ebenso sind Werte über 100% von tₘₐₓ möglich, z. B. 110% von tₘₐₓ, was also nach tₘₐₓ liegt, wenn man einen Delay miteinberechnen möchte, in dem die verabreichte Substanz vom Blut an den Wirkort gelangt, oder wenn tₘₐₓ 13 Minuten beträgt, die Sperrdauer aus praktikablen Gründen jedoch auf z. B. 10 Minuten festgelegt werden soll.

Die elektronische Steuervorrichtung 9 kann also programmiert sein, um auch solche im Datenspeicher M gespeicherten Daten die Substanz S betreffend auszulesen und diese beim Vorbestimmen der nächsten Applikationsdosis Dₙ der Substanz S zu berücksichtigen.

Wie in Fig. 4 schematisch und exemplarisch dargestellt wurde dem Benutzer zum Ausgangszeitpunkt tA₀ zunächst eine Ausgangsdosis D₀ von 50 µg intranasalem Fentanyl verabreicht. Die Konzentration flutet bis zu einem ersten Maximum C₁ₘₐₓ an, weshalb für diesen Zeitpunkt auch das Sperrdauerende T_{_}vain_{1_E} der ersten Sperrdauer T_{_}vain₁ festgelegt ist, deren Sperrdauerbeginn T_{_}vain_{1_s} zu tA₀ liegt.

Würde dem Benutzer ausschließlich die Ausgangsdosis D₀ appliziert werden, so würde die Konzentration C(t) über die Zeit abnehmen und sich bei Annahme der in Fig. 4 gestrichelt gezeigten PK-Kurve PK50 nach etwa 30 Minuten halbiert haben.

Da eine solche Halbierung jedoch offensichtlich nicht den Bedürfnissen des Benutzers entspricht, der zum Betätigungszeitpunkt tB₁ die Abrufvorrichtung 1 betätigt hat, wird ihm zum ersten Abgabezeitpunkt tA₁ eine erste Applikationsdosis D₁ in Höhe von beispielsweise 25 µg Fentanyl verabreicht. Das zum Vorbestimmungszeitpunkt tv₁ erfolgte Vorbestimmen dieser Höhe hat ergeben, dass 25 µg Fentanyl angesichts des Verlaufs der PK50-Kurve genügen, um im Wechselspiel zwischen abnehmender Wirkung oder Konzentration der Ausgangsdosis D₀ bei gleichzeitig ansteigender Wirkung oder Konzentration der ersten Applikationsdosis D₁ eine Konzentration erzielt werden kann, die einerseits nicht über der anfangs als genügend eingestuften Ausgangsdosis D₀ von 50 µg Fentanyl und andererseits nicht unter einer gewünschten therapeutischen Effekt bewirkenden Konzentration C_{ED} von etwa 45 µg Fentanyl liegt.

Die den vom Benutzer gewünschten therapeutischen Effekt bewirkende Konzentration C_{ED} von etwa 45 µg Fentanyl erkennt die Steuervorrichtung 9 darin, dass der Benutzer ab dem Betätigungszeitpunkt tB₁, der nur kurz vor dem ersten Abgabezeitpunkt tA₁ liegt, den gewünschten therapeutischen Effekt offensichtlich vermisst hat, was ihn zum Betätigen der Abrufvorrichtung 1 gebracht hat.

Wie Fig. 4 zeigt, versucht der Benutzer während der Sperrdauer T_{_}vain₁ zwei weitere Applikationen anzufordern (zu den Zeitpunkten tB_{1_v1} und tB_{2_v1}), da der gewünschte therapeutische Effekt anscheinend nicht oder eben noch nicht eingetreten ist. Diese Applikationen werden verweigert, da die Konzentration der Ausganzdosis D₀ noch nicht ihr Maximum erreicht hat, jedenfalls aber die Sperrdauer T_{_}vain₁ noch nicht beendet ist. Ihr Maximum wird sie erst gegen Ende der Sperrdauer T_{_}vain_{1_E} erreichen. Da man nun erkennen kann, dass der Betätigungszeitpunkt tB₁ erst einige Zeit nach dem Ende der Sperrdauer T_{_}vain_{1_E} erfolgt, kann das System anhand der PK-Kurve eine Konzentration ableiten in der, der gewünschte therapeutische Effekt auftritt. In diesem Fall ergibt eine Ausgangsdosis D₀ von 50 µg Fentanyl ein therapeutisches Zeitfenster von 8 Minuten (von Minute 7 bis Minute 15).

Man kann in Fig. 4 auch erkennen, dass die Konzentration für den gewünschten therapeutischen Effekt bereits bei Minute 7 erreicht ist. Dies kann man daran erkennen, dass ab diesen Zeitpunkt keine weiteren vergeblichen Betätigungszeitpunkte tB_{m_vn} während der Sperrdauer T_{_}vain₁ mehr stattfinden. Wie man sehen kann hat die Maximalkonzentration der Ausgangsdosis D₀ jedoch noch nicht ihr Maximum erreicht. Man kann die Konzentration, welche bei dem letzten vergeblichen Betätigungszeitpunkt tB_{3_v1} vorherrscht, zur Verifikation benutzen, indem man diese vorherrschende Konzentration mit dem erwarteten Betätigungszeitpunkt nach T_{_}vain_{1_E} abgleicht.

Die den gewünschten therapeutischen Effekt bewirkende Konzentration C_{ED} ab dem ersten Betätigungszeitpunkt tB₁ kennend, kann die Steuervorrichtung 9 die Höhe der ersten Applikationsdosis D₁ nun selbständig vorbestimmen. Sie kann eine Höhe von 25 µg Fentanyl vorbestimmen, im Wissen, dass das Abklingen der Konzentration C(t) aufgrund der Ausgangsdosis D₀ und das gleichzeitige Anfluten der Konzentration C(t) aufgrund der ersten Applikationsdosis D₁ zu einem zweiten Maximum C₂ₘₐₓ führen wird, welches nicht über einem therapeutisch vertretbaren Maximum liegen wird.

Anhand der Pharmakokinetischen Modelle (PK-Kurven) kann die Steuervorrichtung 9 optional eine Zeit berechnen, in der die Konzentration der Dosis D₁ unter den gewünschten therapeutischen Effekt fallen wird. In Fig. 4 findet dies nach weiteren 19 Minuten oder 34 Minuten seit tA₀ statt.

Ist aus vorstehenden Überlegungen die den gewünschten therapeutischen Effekt bewirkende Konzentration C_{ED} und/oder das jeweils nächste Maximum Cₙₘₐₓ bekannt, so kann aus der Kenntnis des Zeitpunkts, zu welchem die Konzentration C_{ED} oder das jeweils nächste Maximum Cₙₘₐₓ eintreten oder erreicht sein wird, die jeweils nächste Sperrdauer berechnet werden. So kann die Sperrdauer T_{_}vain₁ beispielsweise dem Datenspeicher M im Zusammenhang mit der Höhe der Ausgangsdosis D₀ entnommen werden. Das Ende T_{_}vain_{2_E} der folgenden, zweiten Sperrdauer T_{_}vain₂ kann hingegen aus der Kenntnis des Zeitpunkts von C₂ₘₐₓ gewonnen werden, beiden können identisch miteinander sein. Alternativ kann optional zum Zeitpunkt, tA₁, zu welchem die Konzentration C_{ED} erzielt ist, bereits der Zeitpunkt errechnet werden, zu welchem die Konzentration C(t) erneut auf die Konzentration C_{ED} abgesunken sein wird. Die Hälfte der Differenz zwischen diesen beiden Zeitpunkten kann, ebenso wie ein anderer Anteil hiervon (z. B. 40 %, 35 %, usw.), als Dauer der jeweils nächsten Sperrdauer festgesetzt werden.

Alternative Vorgehensweisen sind ebenso umfasst. So könnte generell auch die errechnete Zeit zur nächsten Unterschreitung von C_{ED} als Sperrdauer gewählt werden, oder kurz davor. Dies wäre in Fig. 4 bei 58 Minuten ab tA₀ der Fall. Also könnte die Steuervorrichtung 9 bei tA₂, hier also tV₂, die Sperrdauer zur nächste Zielkonzentration C_{ED} (bei Minute 58 in Fig. 4) setzen.

Die Höhen der weiteren Applikationsdosen wie z. B. D₂ (in diesen beispielhaften Fall jeweils 25 µg Fentanyl) würden ähnlichen Überlegungen folgend wie zu D₁ vorbestimmt: sie wurden unter Berücksichtigung der Höhe von bereits verabreichten Applikationsdosen sowie der seit ihrer Applikation vergangenen Zeit festgelegt; für die zweite Applikationsdosis D₂ wurde dabei auf eine Restkonzentration, welche auf die Ausgangsdosis D₀ und die erste Applikationsdosis D₁ zurückzuführen ist, abgestellt, für die dritte, hier nicht gezeigte Applikationsdosis würde auf die Restkonzentration, welche auf die Ausgangsdosis D₀, die erste Applikationsdosis D₁ und die zweite Applikationsdosis D₂ zurückzuführen ist, abgestellt bzw. abgestellt werden. Eine Restdosis ist dabei mitbestimmt durch die Höhe der jeweils vorangegangenen Applikation sowie der seit ihrer Applikation vergangenen Zeit.

Die Höhe der nächsten Applikationsdosis Dₙ kann derart vorbestimmt werden, dass die Konzentration im Körper, insbesondere im Blut, des Benutzers erst nach Ablauf einer vorbestimmten Zeitdauer, einstellbar z. B. durch den Arzt oder andere berechtigten Personen, beginnend ab dem Abgabezeitpunkt tAₙ der nächsten Applikationsdosis Dₙ, erneut auf die Zielkonzentration C_{ED} absinken wird.

Wie Fig. 4 zeigt, betätigt der Benutzer zu Betätigungszeitpunkten tB_{1_v1}, tB_{2_v1} und tB_{3_v1} also während der Sperrdauer T_{_}vain₁, die Abrufvorrichtung 1. Da diese drei Betätigungen aber innerhalb der Sperrdauer T_{_}vain₁ liegen, kann der Benutzer hiermit keine Applikationsdosis D erwirken. Dennoch registriert die Steuervorrichtung 9 jedoch die drei vergeblichen Betätigungsversuche und ihre Betätigungszeitpunkte. Sie liegen im Beispiel der Fig. 4 bei Minute 2 (tB_{1_v1}) bzw. bei Minute 4 (tB_{2_v1}) bzw. bei Minute 7 (tB_{3_v1}), und können optional im Datenspeicher M gespeichert werden. Werden die vergeblichen Betätigungsversuche darauf analysiert, etwa in welchem zeitlichen Abstand sie zueinander stehen, wann sie aufhören usw., so kann in erster aber ausreichender Näherung die vom Benutzer gewünschte, den beabsichtigten therapeutischen Effekt bewirkende Konzentration C_{ED} erkannt oder festgelegt werden.

In Fig. 4 sind Feedbackzeitpunkte tF_{1_1}, tF_{1_2}, tF_{1_3} und tF_{1_4} angegeben. Sie stehen für Momente, in welchen der Benutzer eine Feedbackvorrichtung 8 betätigt, um mittels Feedbacks anzudeuten, dass mit der von ihm verspürten Wirkung der zum Feedbackzeitpunkt jeweils vorliegenden Konzentration zufrieden oder nicht zufrieden ist. Er kann dem Nasenapplikator somit z. B. signalisieren, dass er derzeit schmerzfrei ist, dass er keine höhere Konzentration im Körper bevorzugen würde, usw.

In Fig. 4 sieht man bei der PK50_{D0} Kurve, dass man ab ca. Minute 45 eine "Gerade hat", sprich das Gefälle konstant bleibt (pseudo-equilibrium "Lambda").

Die Eliminationshalbzeit ist nun die Zeit in der sich, die vorherrschende Konzentration bei Minute 45, halbiert.

Laut Literatur liegt die Eliminationshalbzeit von Fentanyl zwischen 3 bis 12 Stunden, abhängig von der Gewöhnung.

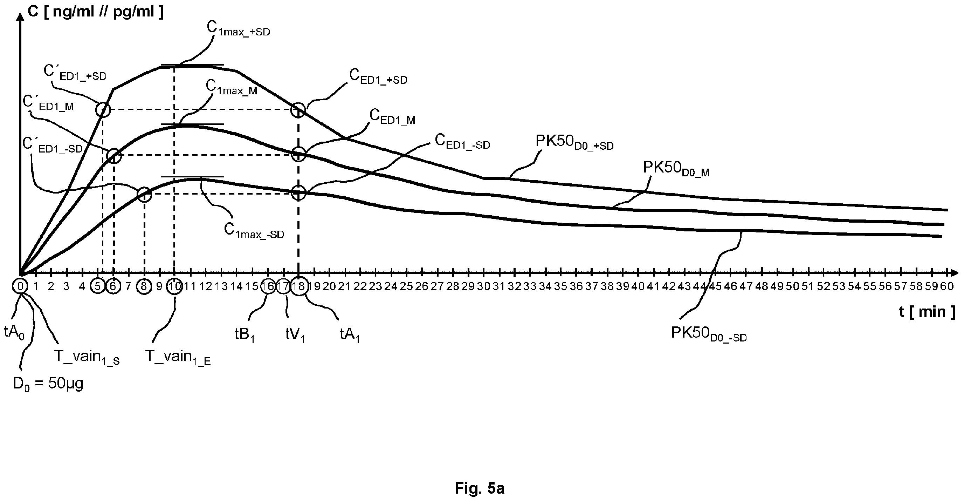

**Fig. 5a** zeigt exemplarisch und schematisch dargestellt mittels dreier exemplarischer PK-Kurven PK50_{D0_+SD}, PK50_{D0_M} und PK50_{D0_-SD} die statistische Verteilung (Mittelwert und Standardabweichung) der möglichen Konzentrationsverläufe im Körper des Benutzers nach Gabe von 50 µg intranasalem Fentanyl, welche an einer ausreichend großen Population gewonnen wurden. Es mag mehr als nur drei zwischen den dargestellten Grenzbereichen liegende Kurven geben, d. h. die entsprechende Untersuchung des Kollektivs/der Population könnte zu mehr als nur drei voneinander unterscheidbare PK-Kurven geführt haben. Sie alle, oder die ihnen zugrundeliegenden Modelle können im Datenspeicher M gespeichert sein und/oder von der Steuervorrichtung 9 berücksichtig werden. Allein aus Gründen der Vereinfachung werden hier nur drei PK-Kurven gezeigt und diskutiert.

Allgemein gilt, dass gleichhohe Dosen einer Substanz patientenindividuell zu unterschiedlichen Konzentrationen C(t) im Körper führen können. Dies wird durch verschiedene Faktoren beeinflusst, wie der Pharmakogenetik, die Intraindividualität, das Alter, das Gewicht, das Geschlecht, die Vormedikation, die Vorerkrankungen, etc. Ferner können verschiedene Nasenanatomien, der Nasenzustand, der Applikationswinkel, wie tief man den Nasenaufsatz einführt und wie man das, manuell ausgelöste, Nasenspray bedient eine Rolle spielen für den Verlauf der jeweiligen PK-Kurve, welcher die Konzentration C(t) über die Zeit im Körper beeinflusst.

Durch Verwendung von Populations-Pharmakokinetik Modellen wie der in Fig. 5a gezeigten PK-Kurven PK50_{D0_+SD} (Mittelwert zuzüglich einer Standardabweichung), PK50_{D0_M} (Mittelwert) und PK50_{D0_-SD} (Mittelwert abzüglich einer Standardabweichung) lassen sich solche Unterschiede abbilden.

Wie man erkennt führt dieselbe Dosis von 50 µg intranasalem Fentanyl für diese drei exemplarisch ausgewählten Kurven zu drei unterschiedlichen Konzentrationsmaxima C_{1max_-SD}, C_{1max_M} und C_{1max_+SD} gekennzeichnet. Eine Vielzahl weiterer Kurven ließe sich ebenfalls darstellen. Sie hätten wiederum andere Konzentrationsmaxima. Der Zeitpunkt bis zur maximalen Konzentration unterscheidet sich bei den drei gezeigten Kurven und auch bei jene nicht dargestellten aus der Vielzahl von Kurven jedoch nicht oder wenn, nur marginal. Das sich am Zeitpunkt, zu welchem das Konzentrationsmaximum C_{1max_-SD}, C_{1max_M} und C_{1max_+SD} erreicht wird, orientierende Ende T_{_}vain_{1_E} der Sperrdauer T_{_}vain₁ endet daher für jede der drei Kurven der Fig. 5a zum selben Zeitpunkt.

Nimmt man an, dass zum Zeitpunkt tA₀ die Ausgangsdosis D₀ (D₀=50 µg) appliziert wird, so kann die Steuervorrichtung 9 anhand der Populations-Pharmakokinetik-Modelle die Maximalkonzentrationen C_{1max_-SD}, C_{1max_M} und C_{1max_+SD} (und viele weitere) berechnen. Da die Zeit bis zum Erreichen dieser Maximalkonzentrationen aber bei allen Varianten gleich ist setzt Steuervorrichtung 9 die erste Sperrdauer (T_{_}vain₁, siehe Fig. 5a) für alle drei PK-Kurven gleich der Zeit bis zum Erreichen der Maximalkonzentration, welcher in diesem Fall exemplarisch auf 10 Minuten festgelegt ist. Das Erreichen der Maximalkonzentration kann in einem Bereich stattfinden. In dem vorliegenden Fall von intranasalen Fentanyl liegt dieser zwischen 10 und 12,8 Minuten. Da der Konzentrationsanstieg in dem Bereich von 10 bis 12,8 Minuten nur sehr gering ist, rundet man die Zeit auf ein praktikables Maß wie hier z. B. 10 Minuten. Man könnte selbstverständlich auch 11, 12 oder 13 Minuten wählen. Somit wird, wenn bei t_{A0} die Ausgangsdosis D₀ (D₀=50 µg) appliziert wird, die daraus jeweils resultierenden Maximalkonzentration berechnet, die Anflutzeit bis zum Erreichen der Maximalkonzentration C_{1max_-SD}, C_{1max_M} oder C_{1max_+SD} berechnet, die Länge der Sperrdauer T_{_}vain₁ (hier 10 min) berechnet und T_{_}vain_{1_s} und T_{_}vain_{1_E} gesetzt.

Entsprechend der unterschiedlichen Verläufe der drei PK-Kurven PK50_{D0_+SD}, PK50_{D0_M} und PK50_{D0_-SD} würde man zum ersten Betätigungszeitpunkt tBi, zu welchem Konzentration unter jene den Benutzer erwünschten therapeutisch Effekt bewirkende, Konzentration fällt, zu welchem der Patient auf Anforderung hin (Zeitpunkte die Betätigung durch den Patienten, das Vorbestimmen usw. betreffend sind hier aus Gründen der Vereinfachung nicht dargestellt oder fallen auf den ersten Abgabezeitpunkt, was in der Praxis tatsächlich auch der Fall sein kann) die erste Applikationsdosis D₁ erhalten würde, eine jeweils unterschiedliche Konzentration C_{ED1_+SD}, C_{ED1_M}, C_{ED1_-SD} errechnen (oder mit Blick auf die wie in Fig. 5a geplotteten PK-Kurven ablesen), welche jeweils den erwünschten therapeutischen Effekt bewirkt. Diese Konzentration ist hierin auch als Zielkonzentration oder C_{ED} bezeichnet. Ihr Wert beruht u. a. auf der Pharmakodynamik.

Es sei angemerkt, dass der Betätigungszeitpunkt tBₙ dem Vorbestimmungszeitpunkt tVₙ und/oder dem Abgabezeitpunkt tA₁ entsprechen kann. Es kann also z. B. gelten: tB₁=tV₁=tA₁.

Fig. 5a ist zu entnehmen, dass die Konzentration C_{ED1_+SD}, C_{ED1_M}, C_{ED1_-SD} bereits vor dem Abgabezeitpunkt t_{A1} als C'_{ED1_+SD}, C'_{ED1_M}, C'_{ED_-SD} bei 5, 6 bzw. 8 min vorgelegen hatten. Dies kann auch mittels der ausbleibenden tB_{_v}'s abgeleitet werden.

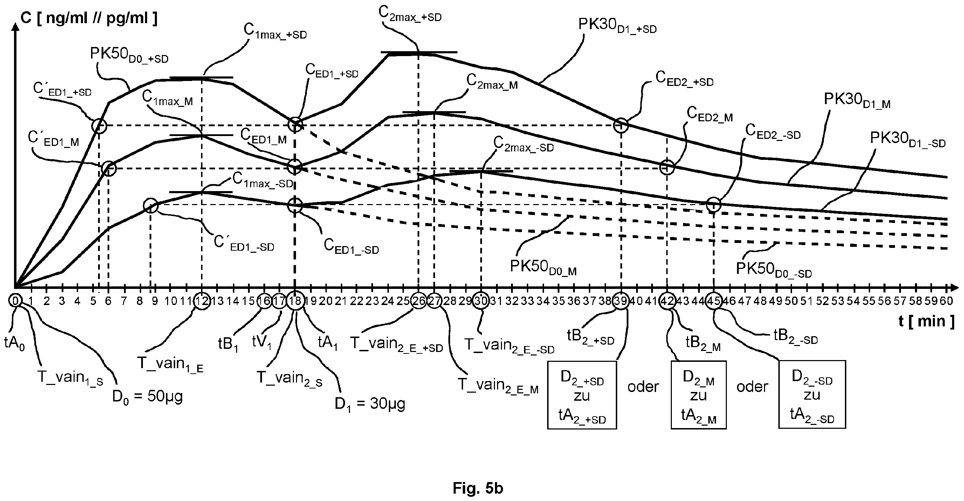

Vorstehende Ausführungen sind Basis für eine Ausführungsform, welche in Bezug auf Fig. 5b diskutiert ist, und in welcher der Nasenapplikator 100 das Betätigungsverhalten des Benutzers auf eine konkrete Weise in Betracht zieht.

Wenn man in Fig. 5b von t_{A0} ausgehend eine Linie (Gerade durch zwei Punkte) zu den entsprechenden Konzentrationen C_{1max_-SD}, C_{1max_M} und C_{1max_+SD} zieht, so erhält man drei Geraden von jeweils unterschiedlicher Steigung. Wenn man jetzt anhand der Zeitabstände den Konzentrationsanstieg betrachtet, so könnte man bereits eine Einschätzung vornehmen, welche Kurve der Benutzer möglicherweise zuzuordnen ist, denn die PK50_{D0_+SD} Kurve hat im gleichen Zeit Intervall den größten Konzentrationsanstieg und die PK50_{D0_-SD} Kurve den geringsten. Da der gewünschte therapeutische Effekt von der Konzentration abhängt, kann man eine erste Einschätzung vornehmen.

**Fig. 5b** zeigt schematisch das exemplarische Vorgehen beim Ermitteln, Auswählen und/oder Festlegen einer individuellen PK-Kurve PK_{Ind}, welche den Wirkstoff-Konzentrations-Verlauf von 50 µg intranasalem Fentanyl eines konkreten, individuellen Benutzers widergibt. Anzumerken ist an dieser Stelle, dass das Ziel beim Ermitteln eines individuellen zeitlichen Konzentrationsverlaufs, wie hierin verwendet, nicht immer eine individuelle PK-Kurve des Benutzers sein muss. Es kann erfindungsgemäß auch genügen, wenn man sich z. B. an der statistischen Mittelwert-Kurve PK50_{D0_M} orientiert oder sich bzw. Grenzbereich-Kurven wie z. B. die Kurve "Mittelwert plus Standardabweichung" oder die Kurve "Mittelwert minus Standardabweichung" oder andere nähert und weiß, in welchem Bereich der Benutzer sich bewegt.

Fig. 5b umfasst die Darstellung der Fig. 5a, ergänzt diese jedoch ab dem ersten Abgabezeitpunkt tA₁ um Gabe einer ersten Applikationsdosis D₁ in Höhe von exemplarisch 30 µg Fentanyl.

Ab dem ersten Abgabezeitpunkt tA₁ werden jeweils die vor diesem Zeitpunkt angenommenen PK-Kurven PK30_{D1_+SD}, PK30_{D1_M} und PK30_{D1_-SD} angelegt. Ausgehend von der in Fig. 5b ermittelten Zielkonzentrationen C_{ED1_+SD}, C_{ED1_M}, C_{ED1_-SD} ermittelt die Steuervorrichtung 9 bzw. der Algorithmus eine Vielzahl von möglichen Dosen und davon abgeleiteten Konzentrationen C(t), welche eine Beurteilung/Kategorisierung erlauben, sprich wann die erwarteten Zeitpunkte der nächsten Betätigung zum Abrufen einer weiteren Applikationsdosis eintreten müssten. In diesem Fall hat das System 30 µg festgelegt, und das System kumuliert die vorhergehenden Kurven und projiziert ausgehend von den ermittelten Zielkonzentrationen C_{ED18+SD}, C_{ED1_M}, C_{ED1_-SD} die jeweiligen Kurvenverläufe in die Zukunft. Es wird ermittelt, zu welchem Zeitpunkt die Zielkonzentrationen C_{ED2_+SD}, C_{ED2_M}, C_{ED2_SD} rechnerisch wieder unterschritten werden sollten. Im Beispiel der Fig. 5b sind sie mit tB_{2_+SD}, tB_{2_M} und tB_{2_-SD} gekennzeichnet. Anhand dieser Überprüfung kann die Steuervorrichtung 9 feststellen, in welchem Bereich oder auf welche Kurve der Benutzer sich befindet. Über diese und weitere Iterationen kann der Benutzer besser eingestuft werden.

Drei mögliche Fälle werden nachfolgend betrachtet:
Fall 1: Trifft auf die Konzentrationsveränderung im Körper des Benutzers die Populations-Pharmakokinetik Kurve PK30_{D1_M} (Mittelwert) zu, so liegt er gute 24 Minuten oberhalb der erwünschten therapeutischen Effekt-Konzentration, nämlich von Minute 18 bis Minute 42. Er würde erst bei Minute 42 zum Zeitpunkt tB_{2_M} eine Betätigung abgeben, was nach einer Vorbestimmung schließlich zu einer erneuten Abgabe einer (zweiten) von ihm angeforderte Applikationsdosis D_{2_M} zum Applikationszeitpunkt tA_{2_M} führt.
Fall 2: Trifft auf die Konzentrationsveränderung im Körper des Benutzers die Populations-Pharmakokinetik Kurve PK30_{D1_+SD} (Mittelwert zuzüglich einer Standardabweichung) zu, so liegt er gute 20 Minuten oberhalb der erwünschten therapeutischen Effekt-Konzentration, nämlich von Minute 18 bis Minute 38. Er würde erst bei Minute 38 zum Zeitpunkt tB_{2_+SD} eine Betätigung abgeben, was nach einer Vorbestimmung schließlich zu einer erneuten Abgabe einer (zweiten) von ihm angeforderte Applikationsdosis D_{2_+SD} zum Applikationszeitpunkt tA_{2_+SD} führt.
Fall 3: Trifft auf die Konzentrationsveränderung im Körper des Benutzers die Populations-Pharmakokinetik Kurve PK30_{D1_-SD} (Mittelwert abzüglich einer Standardabweichung) zu, so liegt er gute 27 Minuten oberhalb der erwünschten therapeutischen Effekt-Konzentration, nämlich von Minute 18 bis Minute 45. Er würde erst bei Minute 45 zum Zeitpunkt tB_{2_-SD} eine Betätigung abgeben, was nach einer Vorbestimmung schließlich zu einer erneuten Abgabe einer (zweiten) von ihm angeforderten Applikationsdosis D_{2_-SD} zum Applikationszeitpunkt tA_{2_-SD} führt.

Die Steuervorrichtung 9 erkennt anhand der Betätigungszeitpunkte tB_{2_+SD}, tB_{2_M}, bzw. tB_{2_-SD} also, ob die individuelle PK-Kurve des Benutzer nun die PK-Kurve PK30_{D1_+SD}, die PK-Kurve PK30_{D1_M} oder die PK-Kurve PK30_{D1-SD} (oder eine der unzähligen weiteren PK-Kurven, welche die Steuervorrichtung 9 ebenfalls betrachten kann) ist. Liegt der Abrufzeitpunkt bei tA_{2_+SD}, so trifft die PK-Kurve PK30_{D1_+SD} zu. Liegt der Abrufzeitpunkt bei tA_{2_M}, so trifft die PK-Kurve PK30_{D1_M} zu. Liegt der Abrufzeitpunkt bei tA_{2_-sD}, so trifft die PK-Kurve PK30_{D1_-SD} zu.

Ist eine erste direkte Zuordnung zwischen einem zeitlichen Verlauf und den betrachteten Benutzer nicht möglich, so nähert sich die Steuervorrichtung 9 mittels Ausschlussverfahrens dennoch dem Ziel und kategorisiert/bestimmt für den konkreten Benutzer anhand seines Betätigungsverhaltens, welcher zeitliche Konzentrationsverlauf oder welche Kurve ihn am besten widerspiegelt. Nach der "Titrationsphase" (welche eine Bestimmungs- oder Findungsphase ist), welche in Fig. 5b gezeigt und sich über die in Fig. 5b möglicherweise noch hinausgehende Zeiten erstrecken kann, kann eine Kategorisierung bzw. Einordnung des Benutzers erfolgen. Dem anschließend kann die Höhe folgender Applikationsdosen bestimmt werden und/oder zeitgesteuert ein individueller, dosis- bzw. konzentrationsbasierter Behandlungsplan aufgestellt werden.

Folgt man vorstehendem Vorgehen, so kann durch Postulieren einer oder beliebig vieler PK-Kurven als mögliche, individuelle PK-Kurve des Benutzers a priori oder als eine Vielzahl von Hypothesen (wie: auf den Benutzer trifft die PK-Kurve PK30_{D1_+SD} zu, oder auf den Benutzer trifft die PK-Kurve PK30_{D1_M} zu, oder auf den Benutzer trifft die PK-Kurve PK30_{D1_-SD} zu) bereits im Anschluss an eine erste Ausgangs- oder Applikationsdosis D₀, D₁, D₂, usw. oder an eine nächste Applikationsdosis Dₙ ganz allgemein die Richtigkeit der zutreffenden der aufgestellten Hypothesen überprüft werden. Dabei gelangt man zu einem individuelles pharmakokinetisches Modell für den Benutzer, das als Grundlage für zukünftige Applikationsdosen genutzt werden kann. Im Beispiel der Fig. 5b wird die PK-Kurve PK30_{D1_+SD} bei Betätigungszeitpunkt tB_{2_+SD} als PK-Kurve PK_{Ind} festgelegt und bei künftigen Vorbestimmungen von Applikationsdosen den erforderlichen Berechnungen zugrunde gelegt.

Eine Überprüfung darauf, dass das so bestimmte, individuelle pharmakokinetische Modell tatsächlich zutrifft, kann im Laufe der weiteren Behandlung und im Zusammenhang mit weiteren Applikationsdosen Dₙ, Dₙ₊₁, usw. jederzeit optional erfolgen.

### Bezugszeichen

- 100: Nasenapplikator
- 101: Peripheriegerät
- 1: Abrufvorrichtung
- 2: Gehäuse
- 3: Abgabedosierer
- 4: Nasenaufsatz
- 5: Sperrvorrichtung
- 7: Dosiserfassungsvorrichtung
- 8: Feedbackvorrichtung
- 9: Elektronische Steuervorrichtung
- 11: Erfassungsvorrichtung
- C_{ED1}: Zielkonzentration, für den gewünschten Effekt/Wirkung erforderliche Konzentration zum Betätigungszeitpunkt tB₁
- C_{ED2}: Zielkonzentration, für den gewünschten Effekt/Wirkung erforderliche Konzentration zum Betätigungszeitpunkt tB₂
- C_{ED3}: Zielkonzentration, für den gewünschten Effekt/Wirkung erforderliche Konzentration zum Betätigungszeitpunkt tB₃
- C_{ED1_+SD}: Erste festgesetzte Zielkonzentration, für den gewünschten Effekt/Wirkung erforderliche Konzentration auf der PK-Kurve PK50_{D0_+SD} zum Betätigungszeitpunkt tB₁
- C_{ED1_M}: Erste festgesetzte Zielkonzentration, für den gewünschten Effekt/Wirkung erforderliche Konzentration auf der PK-Kurve PK50_{D0_M} zum Betätigungszeitpunkt tB₁
- C_{ED1_-SD}: Erste festgesetzte Zielkonzentration, für den gewünschten Effekt/Wirkung erforderliche Konzentration auf der PK-Kurve PK50_{DO_-SD} zum Betätigungszeitpunkt tB₁
- C_{ED2_+SD}: Zweite festgesetzte oder erste geschätzte Zielkonzentration, für den gewünschten Effekt/Wirkung erforderliche Konzentration auf der PK-Kurve PK30_{D1_+SD} zum Betätigungszeitpunkt tB₂
- C_{ED2_M}: Zweite festgesetzte oder erste geschätzte Zielkonzentration, für den gewünschten Effekt/Wirkung erforderliche Konzentration auf der PK-Kurve PK30_{D1_M} zum Betätigungszeitpunkt tB₂
- C_{ED2_-SD}: Zweite festgesetzte oder erste geschätzte Zielkonzentration, für den gewünschten Effekt/Wirkung erforderliche Konzentration auf der PK-Kurve PK30_{D1_-SD} zum Betätigungszeitpunkt tB₂
- C' _{ED1}: Erste vor dem Abgabezeitpunkt tB₁ ermittelte Zielkonzentration, für den gewünschten Effekt/Wirkung erforderliche Konzentration auf der PK-Kurve PK50_{D0}
- C' _{ED1_+SD}: Erste vor dem Abgabezeitpunkt tB₁ ermittelte Zielkonzentration, für den gewünschten Effekt/Wirkung erforderliche Konzentration auf der PK-Kurve PK50_{D0_+SD}
- C' _{ED1_M}: Erste vor dem Abgabezeitpunkt tB₁ ermittelte Zielkonzentration, für den gewünschten Effekt/Wirkung erforderliche Konzentration auf der PK-Kurve PK50_{D0_M}
- C'_{ED1_-SD}: Erste vor dem Abgabezeitpunkt tB₁ ermittelte Zielkonzentration, für den gewünschten Effekt/Wirkung erforderliche Konzentration auf der PK-Kurve PK50_{D0_-SD}
- C(t): Konzentration zum Zeitpunkt t
- Cₙₘₐₓ: Konzentrationsmaximum
- C₁ₘₐₓ: Erstes Maximum auf der PK-Kurve PK50_{D0}
- C₂ₘₐₓ: Zweites Maximum auf der PK-Kurve PK25_{D1}
- C₃ₘₐₓ: Drittes Maximum auf der PK-Kurve PK25_{D2}
- C_{1max_+SD}: Erstes Konzentrationsmaximum auf der PK-Kurve PK50_{D0_+SD}
- C_{1max_M}: Erstes Konzentrationsmaximum auf der PK-Kurve PK50_{D0_M}
- C_{1max_-SD}: Erstes Konzentrationsmaximum auf der PK-Kurve PK50_{D0_-SD}
- C_{2max_+SD}: Zweites Konzentrationsmaximum auf der PK-Kurve PK30_{D1_+SD}
- C_{2max_M}: Zweites Konzentrationsmaximum auf der PK-Kurve PK30_{D1_M}
- C_{2max_-SD}: Zweites Konzentrationsmaximum auf der PK-Kurve PK30_{D1_-SD}
- D: Dosis
- D₀: Ausgangsdosis; Initialdosis
- D₁: Erste Applikationsdosis, vorangehende Applikationsdosis
- D₂: Zweite Applikationsdosis, nachfolgende Applikationsdosis
- D₃: Dritte Applikationsdosis
- Dₙ₋₁: Die der nächsten Applikationsdosis vorangehende Applikationsdosis
- Dₙ: Nächste Applikationsdosis
- Dₙ₊₁: Die der nächsten Applikationsdosis nachfolgende oder übernächste Applikationsdosis
- M: Datenspeicher
- PK25: PK-Kurve für 25 µg Fentanyl Einzeldosis
- PK50: PK-Kurve für 50 µg Fentanyl Einzeldosis
- PK25_{Dn}: PK-Kurve für 25 µg Fentanyl Mehrfachdosis
- PK50_{Dn}: PK-Kurve für 50 µg Fentanyl Mehrfachdosis
- PK50_{+SD}: PK-Kurve für 50 µg Fentanyl Einzeldosis, Mittelwert über alle betrachteten Individuen des Kollektivs zuzüglich einer Standardabweichung
- PK50_{M}: PK-Kurve für 50 µg Fentanyl Einzeldosis, Mittelwert über alle betrachteten Individuen des Kollektivs
- PK50_{-SD}: PK-Kurve für 50 µg Fentanyl Einzeldosis, Mittelwert über alle betrachteten Individuen des Kollektivs abzüglich einer Standardabweichung
- PK50_{Dn_+SD}: PK-Kurve für 50 µg Fentanyl Mehrfachdosis, Mittelwert über alle betrachteten Individuen des Kollektivs zuzüglich einer Standardabweichung
- PK50_{Dn_M}: PK-Kurve für 50 µg Fentanyl Mehrfachdosis, Mittelwert über alle betrachteten Individuen des Kollektivs
- PK50_{Dn_-SD}: PK-Kurve für 50 µg Fentanyl Mehrfachdosis, Mittelwert über alle betrachteten Individuen des Kollektivs abzüglich einer Standardabweichung
- PK30_{Dn_+SD}: PK-Kurve für 30 µg Fentanyl Mehrfachdosis, Mittelwert über alle betrachteten Individuen des Kollektivs zuzüglich einer Standardabweichung
- PK30_{Dn_M}: PK-Kurve für 30 µg Fentanyl Mehrfachdosis, Mittelwert über alle betrachteten Individuen des Kollektivs
- PK30_{Dn_-SD}: PK-Kurve für 30 µg Fentanyl Mehrfachdosis, Mittelwert über alle betrachteten Individuen des Kollektivs abzüglich einer Standardabweichung
- PK_{Ind}: Individuelle PK-Kurve
- R: Substanzreservoir
- S: Medizinisches Substanz
- T_{_}vainₙ: Sperrdauern
- T_{_}vain_{n_S}: Beginn der Sperrdauer
- T_{_}vain_{n_E}: Ende der Sperrdauer
- tA₀: Ausgangszeitpunkt; Abgabezeitpunkt der Ausgangsdosis; Initialdosis
- tA₁,..., tAₙ, tAₙ₊₁: Abgabezeitpunkt 1 bis n+1
- tA_{2_+SD}: Abgabezeitpunkt Mittelwert über alle betrachteten Individuen des Kollektivs zuzüglich einer Standardabweichung
- tA_{2_M}: Abgabezeitpunkt Mittelwert über alle betrachteten Individuen des Kollektivs
- tA_{2_-SD}: Abgabezeitpunkt Mittelwert über alle betrachteten Individuen des Kollektivs Mittelwert abzüglich einer Standardabweichung
- tB₁,..., tBₙ, tBₙ₊₁: Betätigungszeitpunkt 1 bis n+1
- tB_{2_+SD}, tB_{2_M}, tB_{2_-SD}: Berechneter Zielkonzentrationszeitpunkt
- tB_{1_v1}, tB_{2_v1}: Vergebliche Betätigungszeitpunkte während der Sperrdauer T_{_}vain₁
- tB_{1_v2}, tB_{2_v2}: Vergebliche Betätigungszeitpunkte während der Sperrdauer T_{_}vain₂
- tB_{m_vn}: Vergebliche Betätigungszeitpunkte während der Sperrdauer T_{_}vainₙ
- tF_{n_1}: Erster Feedbackzeitpunkt
- tF_{n_2}, tF_{n_3}: Feedbackzeitpunkte
- tF_{n_4}: Letzter Feedbackzeitpunkt
- tV₁,..., tVₙ, tVₙ₊₁: Vorbestimmungszeitpunkte 1 bis n+1

## Patentansprüche

1. Nasenapplikator (100) zum nasalen Verabreichen wenigstens einer medizinischen Substanz (S), insbesondere einem Schmerzmittel, mit einem Gehäuse (2), aufweisend oder verbunden jeweils mit
- einem Substanzreservoir (R) zum Vorhalten einer Menge der Substanz (S);
- einer Abrufvorrichtung (1) zum Betätigen durch den Benutzer mit dem Ziel, eine nächste Applikationsdosis (Dₙ) der Substanz (S) abzurufen;
- einem Abgabedosierer (3) zum Abgeben von Applikationsdosen (D₀, D₁, D₂, ..., Dₙ₋₁, Dₙ, Dₙ₊₁, ...) auf eine Betätigung der Abrufvorrichtung (1) hin zu jeweils einem Abgabezeitpunkt (tAo, tA₁, tA₂, ..., tAₙ₋₁, tAₙ, tAₙ₊₁, ...);
- einer Sperrvorrichtung (5) zum zeitweisen Sperren des Abgabedosierers (3) während einer zu oder nach dem jeweiligen Abgabezeitpunkt (tAo, tA₁, tA₂, ..., tAₙ₋₁, tAₙ, tAₙ₊₁, ...) der abgegebenen Applikationsdosis (D₀, D₁, D₂, ..., Dₙ₋₁, Dₙ, Dₙ₊₁, ...) erneut beginnenden Sperrdauer (T_vain₁, T_vain₂, ..., T_vainₙ₋₁, T_vainₙ, T_vainₙ₊₁, ...) mit einem Sperrdauerbeginn (T_vain_{1_s}, T_vain_{2_s}, ..., T_vain_{n-1_s}, T_vain_{n_s}, T_vain_{n+1_s}, ...) und einem Sperrdauerende (T_vain_{1_E}, T_vain_{2_E}, ..., T_vain_{n-1_E}, T_vain_{n_E}, T_vain_{n+1_E}, ...);
- einer Dosiserfassungsvorrichtung (7) zum Erfassen der Höhe der mittels des Abgabedosierers (3) zum Abgabezeitpunkt (tA₀, tA₁, tA₂, ..., tAₙ₋₁, tAₙ, tAₙ₊₁, ...) jeweils abgegebenen Applikationsdosis (D₀, D₁, D₂, ..., Dₙ₋₁, Dₙ, Dₙ₊₁, ...) ;
- einer elektronischen Steuervorrichtung (9);
- einem Datenspeicher (M) zum Speichern zumindest der Abgabezeitpunkte (tA₀, tA₁, tA₂, ..., tAₙ₋₁) einer oder mehrerer bereits abgegebener Applikationsdosen (D₀, D₁, D₂, ..., Dₙ₋₁) und/oder der Höhen dieser Applikationsdosen (D₀, D₁, D₂, ..., Dₙ₋₁) ;
**gekennzeichnet durch**
- eine Erfassungsvorrichtung (11) zumindest zum Erfassen eines zu einem Betätigungszeitpunkt (tBₙ) erfolgten Betätigungsverhaltens des Benutzers die Abrufvorrichtung (1) betreffend mit dem Ziel, eine nächste oder weitere Applikationsdosis (Dₙ) zu applizieren;
und **dadurch, dass** die elektronische
Steuervorrichtung (9) programmiert ist zum
- Auslesen von im Datenspeicher (M) gespeicherten Daten;
- Auswerten des mittels der Erfassungsvorrichtung (11) erfassten Betätigungsverhaltens des Benutzers;
- Vorbestimmen, zu einem Vorbestimmungszeitpunkt (tVₙ), der Höhe der nächsten oder der weiteren Applikationsdosis (Dₙ), welche an den Benutzer für den Fall, dass dieser außerhalb einer Sperrdauer (T_vainₙ) die Abrufvorrichtung (1) betätigt, zu einem nächsten Abgabezeitpunkt (tAₙ) abgebbar ist, wobei der Vorbestimmungszeitpunkt (tVₙ) zu oder nach dem Betätigungszeitpunkt (tBₙ) liegt, und wobei das Vorbestimmen erfolgt unter Berücksichtigung der aus dem Datenspeicher (M) ausgelesenen Daten, wobei diese Daten zumindest a) den Abgabezeitpunkt (tAo, tA₁, tA₂, ..., tAₙ₋₁) einer oder mehrerer der bereits abgegebenen Applikationsdosen (D₀, D₁, D₂, ..., Dₙ₋₁), oder die zwischen dem oder den mehreren Abgabezeitpunkten (tAo, tA₁, tA₂, ..., tAₙ₋₁) der bereits abgegebenen Applikationsdosen (D₀, D₁, D₂, ..., Dₙ₋₁) und dem nach dem nächsten Betätigungszeitpunkt (tBₙ) liegenden Abgabezeitpunkt (tAₙ) verstrichenen Zeitdauer, einerseits und/oder b) die Höhe einer oder mehrerer der bereits abgegebenen Applikationsdosen (D₀, D₁, D₂, ..., Dₙ₋₁) andererseits umfasst.

2. Nasenapplikator (100) nach Anspruch 1, wobei
- der Datenspeicher (M) weiter pharmakologische, pharmakodynamische, pharmakometrische und/oder pharmakokinetische Daten die Substanz (S) betreffend oder andere der Substanz zugeordnete Daten umfasst, wobei
- die elektronische Steuervorrichtung (9) weiter programmiert ist zum zusätzlichen Auslesen von im Datenspeicher (M) gespeicherten Daten die Substanz (S) betreffend oder der Substanz zugeordneten Daten, und wobei
- das Vorbestimmen der nächsten Applikationsdosis (Dₙ) der Substanz (S) erfolgt unter zusätzlicher Berücksichtigung der zusätzlich ausgelesenen Daten.

3. Nasenapplikator (100) nach Anspruch 1 oder 2, wobei die elektronische Steuervorrichtung (9) weiterhin programmiert ist zum Veranlassen der weiteren Schritte:
- Abgeben der nächsten Applikationsdosis (Dₙ) in der vorbestimmten Höhe zum entsprechenden Abgabezeitpunkt (tAₙ) mittels des Abgabedosierers (3); und nachfolgend
- Speichern der Höhe und/oder einer daraus resultierenden Konzentration der Applikationsdosis der abgegebenen nächsten Applikationsdosis (Dₙ) im Datenspeicher (M) und Veranlassen der Sperrvorrichtung (5) zum zeitweisen Sperren des Abgabedosierers (3) für eine ab oder nach dem Abgabezeitpunkt (tAₙ) dieser nächsten Applikationsdosis (Dₙ) beginnenden Sperrdauer (T_vainₙ₊₁) mit einem Sperrdauerbeginn (T_vain_{n+1_s}) und Sperrdauerende (T_vain_{n+1_E}) und/oder Speichern des Abgabezeitpunkts (tAₙ) der abgegebenen, nächsten Applikationsdosis (Dₙ) im Datenspeicher (M).

4. Nasenapplikator (100) nach Anspruch 3, wobei die elektronische Steuervorrichtung (9) weiter programmiert ist, die Abfolge der weiteren Schritte des Anspruchs 3 auch im Zusammenhang mit einer oder mehreren der auf die nächste Applikationsdosis (Dₙ) folgenden Abgaben von weiteren Applikationsdosen (Dₙ₊₁) auszuführen oder zu veranlassen.

5. Nasenapplikator (100) nach Anspruch 4, wobei die elektronische Steuervorrichtung (9) weiterhin programmiert ist, aus dem Datenspeicher (M) Daten auszulesen, welche z. B. PK-Kurven (PK25, PK50) und/oder Modelle umfassen, und welche einen zeitlichen Verlauf zwischen Konzentration (C(t)) im Körper, insbesondere Blut, des Patienten abbilden, und um basierend auf diesen ein nächstes Maximum (C₁ₘₐₓ, C₂ₘₐₓ, Cₙₘₐₓ) der Konzentration (C(t)) zu errechnen.

6. Nasenapplikator (100) nach Anspruch 5, wobei die elektronische Steuervorrichtung (9) weiter programmiert ist, beim Vorbestimmen der nächsten Applikationsdosis (Dₙ) basierend auf im Datenspeicher (M) gespeicherten Daten, insbesondere PK-Kurven (PK25, PK50) oder Modelle, und einem im Datenspeicher (M) vorgehaltenen, therapeutischen Maximum die Höhe der nächsten Applikationsdosis (Dₙ) derart zu bestimmen, dass das nächste Maximum (C₁ₘₐₓ, C2max, Cₙₘₐₓ) das vorgegebene Maximum nicht übersteigen wird.

7. Nasenapplikator (100) nach einem der vorangegangenen Ansprüche, wobei
- das Auswerten des erfassten Betätigungsverhaltens des Benutzers das Feststellen des Betätigungszeitpunkts (tBₙ) zum Abrufen der nächsten Applikationsdosis (Dₙ), oder des zwischen diesem Betätigungszeitpunkt (tBₙ) und dem Sperrdauerende (T_vain_{n_E}) der zuletzt abgelaufenen Sperrdauer (T_vainₙ) liegenden Zeitspanne, umfasst; wobei
- das Vorbestimmen das Berücksichtigen des festgestellten nächsten Betätigungszeitpunkts (tBₙ) oder der festgestellten Zeitspanne umfasst.

8. Nasenapplikator (100) nach einem der vorangegangenen Ansprüche, weiter aufweisend
- eine Feedbackvorrichtung (8) zum Betätigen durch den Benutzer;
wobei die Erfassungsvorrichtung (11) programmiert ist zum Erfassen einer zu wenigstens einem, vorzugsweise ersten, Feedbackzeitpunkt (tF_{n_1}) erfolgten Betätigung der Feedbackvorrichtung (8);
wobei Steuervorrichtung (9) programmiert ist zum Ermitteln der zu dem wenigstens einen Feedbackzeitpunkt (tF_{n_1}) im Körper oder im Blut des Benutzers vorliegenden Konzentration der Substanz (S), und zum Festsetzen einer Zielkonzentration (C_{ED}) oder ihres Maximalwerts auf einen Wert jeweils unterhalb der ermittelten Konzentration.

9. Nasenapplikator (100) nach Anspruch 8,
wobei die Erfassungsvorrichtung (11) weiter programmiert ist zum Erfassen von wenigstens einer Betätigung der Abrufvorrichtung (1) durch den Benutzer zu Betätigungszeitpunkten (tB₁, tB₂, ..., tBₙ₋₁, tBₙ, tBₙ₊₁, ...) nach der Sperrdauer (T_vain₁, T_vain₂, ..., T_vainₙ₋₁, T_vainₙ, T_vainₙ₊₁, ...) und/oder zum Erfassen eines, vorzugsweise letzten, Feedbackzeitpunkts (tF_{n_4}), ab welchem weitere Betätigungen der Feedbackvorrichtung (8) durch den Benutzer ausbleiben, vorzugsweise letzten, ausbleibenden Betätigungsverhaltens des Benutzers;
wobei die Steuervorrichtung (9) weiter programmiert ist
- zum Ermitteln sowohl einer dem, vorzugsweise ersten, Feedbackzeitpunkt (tF_{n_1}) als auch einer dem, vorzugsweise ersten Betätigungszeitpunkt (tB₁, tB₂, ..., tBₙ₋₁, tBₙ, tBₙ₊₁, ...) oder dem letzten Feedbackzeitpunkt (tF_{n_4}) jeweils zugeordneten Konzentration (C(t)) im Körper, insbesondere im Blut;
- zum Ermitteln eines zeitlichen Konzentrationsverlaufs aus einer Gruppe von derselben Dosis zugeordneten zeitlichen Konzentrationsverläufe, insbesondere Modellen oder PK-Kurven (PK50_{+SD}, PK50_{M} und PK50_{-SD}), aus einer Vielzahl von im Datenspeicher (M) gespeicherten, derselben Dosis zugeordneten zeitlichen Konzentrationsverläufen, insbesondere PK-Modelle oder PK-Kurven, unter Berücksichtigung sowohl dem, vorzugsweise ersten, Feedbackzeitpunkt (tF_{n_1}) als auch einer dem, vorzugsweise ersten Betätigungszeitpunkt (tB₁, tB₂, ..., tBₙ₋₁, tBₙ, tBₙ₊₁, ...) oder dem letzten Feedbackzeitpunkt (tF_{n_4}) geordneten Konzentration; und
- zum Berücksichtigen des so ermittelten zeitlichen individuellen Konzentrationsverlaufs, insbesondere die ermittelte individuelle PK-Kurve (PK_{Ind}), beim Vorbestimmen der Höhe einer oder mehrerer der nachfolgenden Applikationsdosen (D₃, Dₙ, Dₙ₊₁, ...).

10. Nasenapplikator (100) nach Anspruch 8 oder 9, wobei die Steuervorrichtung (9) weiter programmiert ist,
- beim Vorbestimmen der Höhe der nächsten Applikationsdosis (Dₙ) die festgesetzte Zielkonzentration (C_{ED}) oder ihren Maximalwert und/oder den ermittelten zeitlichen individuellen Konzentrationsverlauf, insbesondere die ermittelte individuelle PK-Kurve (PK_{Ind}), zu berücksichtigen.

11. Nasenapplikator (100) nach einen der vorangegangenen Ansprüche, wobei die Steuervorrichtung (9) weiter programmiert ist,
- zum Ermitteln, nach erfolgter Abgabe einer Ausgangsdosis (D₀), welche zu einem Abgabezeitpunkt (tA₀) abgegeben wurde, an oder für einen ersten Betätigungszeitpunkt (tB₁), welcher nach dem Abgabezeitpunkt (tA₀) der Ausgangsdosis (D₀) liegt, für eine Gruppe von derselben Dosis zugeordneten zeitlichen Konzentrationsverläufe, insbesondere Modellen oder PK-Kurven (PK50_{+SD}, PK50_{M} und PK50_{-SD}), aus einer Vielzahl von im Datenspeicher (M) gespeicherten, derselben Dosis zugeordneten zeitlichen Konzentrationsverläufen, insbesondere PK-Modelle oder PK-Kurven, jeweils eine dem ersten Betätigungszeitpunkt (t_{B1}) zugeordnete Konzentration (C_{ED18_+SD}, C_{ED1_M}, C_{ED1_-SD}), und zum Festsetzen der so ermittelten Konzentrationen (C_{ED1_+SD}, C_{ED1_M}, C_{ED1_-SD}) jeweils als geschätzte Zielkonzentration des zugehörigen zeitlichen Konzentrationsverlaufs;
- zum Applizieren der auf die Ausgangsdosis (D₀) folgenden ersten Applikationsdosis (D₁) als Reaktion auf die Betätigung der Abrufvorrichtung (1) am ersten Betätigungszeitpunkt (tB₁);
- zum Ermitteln für die zeitlichen Konzentrationsverläufe der Gruppe, zu welchem Zeitpunkt nach dem Applizieren der ersten Applikationsdosis (D₁) die Konzentration im Körper, insbesondere im Blut, jeweils erneut auf die geschätzte Zielkonzentration des zugehörigen zeitlichen Konzentrationsverlaufs abgesunken sein wird, und zum Festsetzen, jeweils für die zeitlichen Konzentrationsverläufe der Gruppe, des ermittelten Zeitpunkts als berechneten Zielkonzentrationszeitpunkt (tB_{2_+SD}, tB_{2_M}, tB_{2_-SD}) ;
- zum Erfassen oder Feststellen des zweiten Betätigungszeitpunkts (tB₂) zum Abrufen einer zweiten oder nachfolgenden Applikationsdosis (D₂);
- zum Ermitteln eines zeitlichen Konzentrationsverlaufs aus der Gruppe als individuellen zeitlichen Konzentrationsverlauf, insbesondere als individuelle PK-Kurve (PK_{Ind}), unter Berücksichtigung der jeweiligen Differenz zwischen dem berechneten Zielkonzentrationszeitpunkt (tB_{2_+SD}, tB_{2_M}, tB_{2_-SD}) eines jeden zeitlichen Konzentrationsverlaufs aus der Gruppe und dem zweiten Betätigungszeitpunkt (tB₂), insbesondere des zeitlichen Konzentrationsverlaufs, welche die geringste Differenz zwischen seinem berechneten Zielkonzentrationszeitpunkt (tB_{2_+SD}, tB_{2_M}, tB_{2_-SD}) und dem zweiten Betätigungszeitpunkt (tB₂) aufweist; und
- zum Berücksichtigen des ermittelten zeitlichen individuellen Konzentrationsverlaufs, insbesondere die ermittelte individuelle PK-Kurve (PK_{Ind}), beim Vorbestimmen der Höhe einer oder mehrerer der nachfolgenden Applikationsdosen (D₃, Dₙ, Dₙ₊₁, ...).

12. Nasenapplikator (100) nach einem der vorangegangenen Ansprüche, wobei die Steuervorrichtung (9) weiter programmiert ist
- zum Ermitteln der zu wenigstens einem betrachteten Betätigungszeitpunkt (tBₙ₋₁) oder zu wenigstens einem betrachteten Vorbestimmungszeitpunkt (tVₙ₋₁) im Körper oder im Blut des Benutzers vorliegenden Konzentration der Substanz (S), und zum Festsetzen dieser Konzentration als die Zielkonzentration (C_{ED});
und wobei die Steuervorrichtung (9) weiter programmiert ist, um
- am nächsten Vorbestimmungszeitpunkt (t_{Vn}) die Höhe der nächsten Applikationsdosis (Dₙ) derart festzusetzen, dass die Konzentration im Körper oder im Blut des Benutzers erst nach Ablauf einer vorbestimmten Zeitdauer, beginnend ab dem Abgabezeitpunkt (tAₙ) der nächsten Applikationsdosis (Dₙ), erneut auf die Zielkonzentration (C_{ED}) absinken wird.

13. Nasenapplikator (100) nach einem der vorangegangenen Ansprüche, wobei
- das Auswerten des mittels der Erfassungsvorrichtung (11) erfassten Betätigungsverhaltens des Benutzers das Erfassen von Betätigungen der Abrufvorrichtung (1) durch den Benutzer zu Betätigungszeitpunkten (tB_{1_v1}, tB_{2_v1}, tB_{1_v2}, tB_{2_v2}) innerhalb einer der
Sperrdauern (T_vaini, T_vain₂, ..., T_vainₙ₋₁, T_vainₙ, T_vainₙ₊₁, ...) umfasst;
- die Steuervorrichtung (9) weiter programmiert ist zum Ermitteln einer den Betätigungszeitpunkten (tB_{1_v1}, tB_{2_v1}, tB_{1_v2}, tB_{2_v2}) innerhalb einer der Sperrdauern (T_vain₁, T_vain₂, ..., T_vainₙ₋₁, T_vainₙ, T_vainₙ₊₁, ...) jeweils zugeordneten Konzentration (C(t)) im Körper, insbesondere im Blut;
- Festsetzen der Zielkonzentration (C_{ED}) oder ihres Mindestwerts auf einen Wert jeweils oberhalb der zugeordneten Konzentration (C(t)).

14. Nasenapplikator (100) nach Anspruch 13,
wobei die Erfassungsvorrichtung (11) weiter programmiert ist zum Erfassen von wenigstens einer Betätigung der Abrufvorrichtung (1) durch den Benutzer zu Betätigungszeitpunkten (t_{B1}, t_{B2}, ..., t_{Bn-1}, tBₙ, t_{Bn+1}, ...) nach der Sperrdauer (T_vain₁, T_vain₂, ..., T_vainₙ₋₁, T_vainₙ, T_vainₙ₊₁, ...) ;
wobei die Steuervorrichtung (9) weiter programmiert ist
- zum Ermitteln einer dem Betätigungszeitpunkt (tB₁, tB₂, ..., tBₙ₋₁, tBₙ, tBₙ₊₁, ...) nach der Sperrdauer (T_vaini, T_vain₂, ..., T_vainₙ₋₁, T_vainₙ, T_vainₙ₊₁, ...) jeweils zugeordneten Konzentration (C(t)) im Körper, insbesondere im Blut;
- zum Ermitteln eines zeitlichen Konzentrationsverlaufs der Gruppe unter Berücksichtigung sowohl wenigstens eines, vorzugsweise des letzten, Betätigungszeitpunktes (tB_{1_v1}, tB_{2_v1}, tB_{1_v2}, tB_{2_v2}) innerhalb der Sperrdauer (T_vaini, T_{_}vain₂, ..., T_{_}vainₙ₋₁, T_{_}vainₙ, T_{_}vainₙ₊₁, ...) als auch wenigstens eines, vorzugsweise des ersten, Betätigungszeitpunktes (tB₁, tB₂, ..., tBₙ₋₁, tBₙ, tBₙ₊₁, ...) nach der Sperrdauer (T_vaini, T_{_}vain₂, ..., T_vainₙ₋₁, T_vainₙ, T_vainₙ₊₁, ...); und
- zum Berücksichtigen des ermittelten zeitlichen individuellen Konzentrationsverlaufs, insbesondere die ermittelte individuelle PK-Kurve (PK_{Ind}), beim Vorbestimmen der Höhe einer oder mehrerer der nachfolgenden Applikationsdosen (D₃, Dₙ, Dₙ₊₁, ...).

15. Nasenapplikator (100) nach einem der vorangegangenen Ansprüche, wobei
- das Auswerten des mittels der Erfassungsvorrichtung (11) erfassten Betätigungsverhaltens des Benutzers das Auswerten von Betätigungen der Abrufvorrichtung (1) durch den Benutzer zu Betätigungszeitpunkten (tB_{1_v1}, tB_{2_v1}, tB_{1_v2}, tB_{2_v2}) innerhalb einer der Sperrdauern (T_vaini, T_{_}vain₂, ..., T_vainₙ₋₁, T_vainₙ, T_vainₙ₊₁, ...) umfasst;
- die Steuervorrichtung (9) weiter programmiert ist zum Ermitteln einer Zunahme der Zeitabstände zwischen aufeinanderfolgenden Betätigungszeitpunkten (tB_{1_v1}, tB_{2_v1}, tB_{1_v2}, tB_{2_v2}) innerhalb einer der Sperrdauern (T_vain₁, T_vain₂, ..., T_vainₙ₋₁, T_vainₙ, T_vainₙ₊₁, ...) oder das Ausbleiben weiterer Betätigungszeitpunkten (tB_{1_v1}, tB_{2_v1}, tB_{1_v2}, tB_{2_v2}) innerhalb der Sperrdauer (T_vaini, T_{_}vain₂, ..., T_{_}vainₙ₋₁, T_{_}vainₙ, T_vainₙ₊₁, ...), und zum Festlegen eines Zeitpunkts, ab welchem das Maß der Zunahme oder das Ausbleiben vorbestimmten Kriterien genügt;
- die Steuervorrichtung (9) weiter programmiert ist zum Ermitteln einer dem Zeitpunkt zugeordneten Konzentration (C(t)) im Körper, insbesondere im Blut;
- Festsetzen der Zielkonzentration (C_{ED}) oder ihres Mindestwerts auf einen Wert jeweils oberhalb der zugeordneten Konzentration (C(t)).

16. Nasenapplikator (100) nach Anspruch 15,
wobei die Steuervorrichtung (9) weiter programmiert ist zum Auswerten von wenigstens einer erfassten Betätigung der Abrufvorrichtung (1) durch den Benutzer zu Betätigungszeitpunkten (tB₁, tB₂, ..., tBₙ₋₁, tBₙ, tBₙ₊₁, ...) nach der Sperrdauer (T_vain₁, T_vain₂, ..., T_vainₙ₋₁, T_vainₙ, T_vainₙ₊₁, ...) ;
wobei die Steuervorrichtung (9) weiter programmiert ist
- zum Ermitteln einer dem Betätigungszeitpunkt (tB₁, tB₂, ..., tBₙ₋₁, tBₙ, tBₙ₊₁, ...) nach der Sperrdauer (T_vaini, T_vain₂, ..., T_vainₙ₋₁, T_vainₙ, T_vainₙ₊₁, ...) jeweils zugeordneten Konzentration (C(t)) im Körper, insbesondere im Blut;
- zum Ermitteln eines zeitlichen Konzentrationsverlaufs der Gruppe unter Berücksichtigung sowohl eines, vorzugsweise des letzten, Betätigungszeitpunktes (tB_{1_v1}, tB_{2_v1}, tB_{1_v2}, tB_{2_v2}) innerhalb der Sperrdauer (T_vain₁, T_vain₂, ..., T_vainₙ₋₁, T_vainₙ, T_vainₙ₊₁, ...) als auch eines, vorzugsweise des ersten, Betätigungszeitpunktes (tB₁, tB₂, ..., tBₙ₋₁, tBₙ, tBₙ₊₁, ...) nach der Sperrdauer (T_vain₁, T_vain₂, ..., T_vainₙ₋₁, T_vainₙ, T_vainₙ₊₁, ...); und
- zum Berücksichtigen des ermittelten zeitlichen individuellen Konzentrationsverlaufs, insbesondere die ermittelte individuelle PK-Kurve (PK_{Ind}), beim Vorbestimmen der Höhe einer oder mehrerer der nachfolgenden Applikationsdosen (D₃, Dₙ, Dₙ₊₁, ...).

17. System, aufweisend
- einen oder mehrere Nasenapplikatoren (100) nach einem der vorangegangenen Ansprüche;
- ein oder mehrere Peripheriegeräte (101);
wobei der eine oder die mehreren Nasenapplikatioren (100) mit einem oder mehreren der Peripheriegeräte (101) in Signal- oder Kommunikationsverbindung stehen oder hierzu vorbereitet oder programmiert sind.
